# EUROPEAN PATENT APPLICATION

(11) **EP 1 544 295 A1**
(43) Date of publication of application: **22.06.2005**
(21) Application number: 03792752.2
(22) Date of filing: 20.08.2003
(51) Int. Cl.: C12N 15/09, C12N 1/19, C12N 1/21, C12N 5/10, C12N 9/12, A61K 31/7088, A61K 35/76, A61K 38/45, A61K 39/395, A61K 45/00, A61K 48/00, A61P 3/04, A61P 3/06, A61P 3/10, A61P 9/00, A61P 9/10, A61P 9/12, A61P 19/06, A61P 25/28, A61P 43/00, C07K 16/40

(54) **SALT-INDUCIBLE KINASES 2 AND USE THEREOF**

(30) Priority: 21.08.2002 JP 2002240092; 31.01.2003 JP 2003023295
(71) Applicant: Proteinexpress Co., Ltd., Chosi-shi, Chiba 288-0041 (JP); Takemori, Hiroshi, Suita-shi, Osaka 565-0871 (JP); Okamoto, Mitsuhiro, Suita-shi, Osaka 565-0871 (JP)
(72) Inventor: Takemori, Hiroshi, Suita-shi, Osaka 565-0871 (JP); Okamoto, Mitsuhiro, Suita-shi, Osaka 565-0871 (JP)
(74) Representative: Curtis, Philip Anthony
(86) International application number: PCT/JP2003/010535
(87) International publication number: WO 2004/018669

(57) **Abstract**

The polypeptide of the present invention has the amino acid sequence represented by SEQ ID NO: 2, 4, 6, 8, 10, or 12. The polypeptide of the present invention is useful as an agent for preventing or improving a disease with which the metabolic disorder of fat cells or the like is associated.

## Description

### Technical Field

The present invention relates to a salt-inducible kinase 2 (SIK2) polypeptide derived from a mouse, a polynucleotide encoding the polypeptide, an antibody reacting with the polypeptide, a method for screening a compound capable of promoting or inhibiting the activity of the polypeptide, and a composition suppressing or increasing the action of the polypeptide, and the like.

### Background Art

It has been known that various types of hormones or neurotransmitters increase the intracellular level of cAMP. When such an intracellular level of cAMP is increased, it activates cAMP-dependent protein kinase (PKA). It has been reported that when the catalytic domain of PKA is dissociated from a regulatory domain for negatively regulating the PKA activity, PKA is activated (Mayr et al., Nature Reviews Molecular Cell Biology (Nat. Rev. Mol. Cell Biol) 2, 599-609 (2001)). The activated PKA causes the phosphorylation of a cAMP responsive element binding protein (hereinafter referred to as "CREB" in the present specification), which is a transcription factor binding to a CAMP responsive element (hereinafter referred to as "CRE" at times in the present application), and that the thus phosphorylated CREB binds to CRE, thereby regulating the transcriptional activity of a gene.

Differentiation and functions of fat cells are also regulated by intracellular cAMP (Reusch et al., Molecular Cellular Biology (Mol. Cell. Biol.) 20, 1008-1020 (2000)). It has been reported that the activation of CREB is essential for induction of the differentiationof fat cells, and that mainly CREB regulates such differentiation of fat cells. It has also been reported that when the differential mechanism of insulin-inducible fat cells is inhibited by inhibition of prenylation, the differentiation of the fat cells is supplemented by activation of CREB. On the other hand, CREB regulates the mechanism of blood glucose homeostasis. That is to say, a cAMP-PKA-CREB-CRE signal plays an extremely important role in the differentiation and functions of fat cells and the regulation of blood glucose. Thus, it is considered that adiposis, diabetes, and other diseases can be prevented or improved by controlling such a cAMP-PKA-CREB-CRE signal.

However, since the CREB-binding protein (CBP) that has previously been reported to date is expressed also in tissues other than fat cells, it is difficult to regulate the functions of fat cells using such a CREB-binding protein. Thus, it is considered that if a modulator of CREB that specifically expresses in fat cells were identified and the physiological activity of the modulator were regulated, the development of adiposis, diabetes, and other diseases could be prevented or improved. Accordingly, it has been desired that a modulator of CREB that is specific for fat cells be developed.

cDNA encoding salt-inducible kinase (SIK) has been identified in the adrenal cortex of a rat treated with high salinity diet stress [Wang et al., FEBS Letters 453, 135-139 (1999)]. SIK is an enzyme belonging to an AMP-activated protein kinase family, which is serine threonine kinase playing an important role for the regulation of fat metabolism under stressful stimuli. It has been reported that this enzyme (SIK) is induced by a cAMP-PKA signal, that it regulates the gene expression activity of CREB activated with PKA, and that the regulation has a correlation with the protein-phosphorylating activity of SIK [J. Doi et al., Journal of Biological Chemistry277, 15629-15637 (2002)]. However, SIK is expressed in a wide range of tissues [X. Lin et al., Molecular Endocrinology 15 (8), 1264-1276(2001); and J.D. Feldman et al., Journal of Neurochemistry 74, 2227-2238 (2000)], and thus, it cannot be said that SIK is a modulator of CREB that is specific for fat cells.

It has been reported that fat metabolism in fat cell tissues is under the control of two hormone signaling pathways. That is to say, insulin induces the absorption of glucose and the generation of lipids. On the other hand, cAMP generated by exogenous stimulation, such as adrenaline or glucagon, induces lipolysis. If the balance between these two signal systems were lost, fat tissues would be afflicted with hyperinsulinism, and the fat tissues thereby gradually become resistant to stimulation with insulin [Sul. H. S. et al., Prog Nucleic Acid Res Mol Biol 60, 317-345 (1998); Saltiel A. R., and Kahn C. R. Nature 414, 799-806 (2001)]. Such insulin resistance occurring in tissues associated with biological metabolism, such as those of fat tissues, liver, or skeletal muscle, eventually results in a systemic disorder of energy metabolism, such as adiposis or type II diabetes. An insulin receptor substrate 1 (IRS-1) protein is a molecule that is a key of insulin-signaling cascade. That is, a tyrosine residue in the IRS-1 protein is phosphorylated by the action of insulin receptor kinase that is insulin-dependently activated, and the IRS-1 protein containing the phosphorylated tyrosine then induces intracellular cascade. It has been reported that a serine residue in the IRS-1 protein is phosphorylated under certain non-physiological conditions. It is considered that serine phosphorylation of the IRS-1 protein regulates the efficiency of insulin-signal cascade, and that as a result, organisms become resistant to stimulation with insulin. Several protein kinase molecules associated with the serine phosphorylation of the IRS-1 protein have been identified and reported [Tanasijevic M. J. et al., J Biol Chem 268, 18157-18166 (1993)].

As stated above, in order to prevent or improve the development of adiposis, diabetes, or other diseases, the roles of modulators of CREB specific for fat cells and IRS-1 are important.

It is an object of the present invention to provide a peptide or the like, which has an activity as a modulator of the aforementioned CREB specific for fat cells, as well as having an activity as a modulator of IRS-1.

### Disclosure of the Invention

It has been discovered that the aforementioned SIK has an isoform. Thus, the existing SIK is called SIK1, and the newly found SIK is called SIK2. As a result of analyzing these SIKs, it has been found that SIK1 is mainly expressed in the adrenal gland or ovary, whereas SIK2 is mainly expressed in fat cells. Thus, studies regarding a possibility of SIK2 regulating differential maturation of fat cells have progressed. At the same time, the full-length cDNA sequence of mouse SIK2 has been identified.

Moreover, as a result of analyzing the physiological functions of SIK2, it has been found that SIK2 regulates the activation of CREB through a leucine zipper portion (bZIP) of CREB. Furthermore, the present inventors have found that SIK2 has three domains, which are a kinase domain (domain 1) having kinase activity, a stabilizing domain (domain 2) playing a role of stabilizing a protein, and a regulating domain (domain 3) regulating kinase activity. They have clarified that domain 2 was regulated by phosphorylation, thereby losing its transcription-suppressing activity due to phosphorylation. In other words, it was clarified that domain 2 does not only govern the stabilization of a protein.

In addition, domain 3 plays an important role particularly for suppression of transcription, and it regulates the CREB-activating functions of SIK2. In particular, when domain 3 is phosphorylated, the CREB-suppressing activities of SIK1 andSIK2 are decreased. Such an effect of SIK2 is significant in fat cells, but that of SIK1 is not so significant therein. Moreover, in SIK1, domain 3 has a function of transferring an SIK1 protein into a nucleus and also a function of discharging it from the nucleus. In SIK2, however, it has only a function of discharging an SIK2 protein from the nucleus. When the enzyme activities of SIK1 and SIK2 disappear in a state where domain 3 has been phosphorylated, SIK1 and SIK2 activate CREB. However, since the domain 3 of SIK1 also acts for the transfer of the SIK1 protein into the nucleus, SIK1 cannot activate CREB in a low cAMP stimulation state. This is due to the difference of the functions of domain 3 between SIK1 and SIK2, that is, due to intracellular localization. The present inventors have found that differing from nuclear SIK that cannot activate CREB under any conditions, cytoplasmic SIK can activate CREB under the aforementioned conditions, that a large amount of SIK2 is expressed in fat cells, that SIK2 and SIK1 phosphorylate serine (794) of human IRS-1, that the activity and amount of SIK2 are decreased in brown adipose cells, and that the activity and amount thereof are increased in white adipose cells of animals suffering from diabetes, thereby completing the present invention.

That is to say, according to the present invention, there is provided a polypeptide comprising an amino acid sequence having homology of at least 90% to any one of the polypeptides described in the following (A) to (L), or a salt thereof:
(A) a polypeptide comprising an amino acid sequence identical to or substantially identical to the amino acid sequence represented by SEQ ID NO: 2, or a polypeptide comprising an amino acid sequence encoded by cDNA capable of hybridizing to the nucleotide sequence represented by SEQ ID NO: 1;
(B) a polypeptide comprising an amino acid sequence identical to or substantially identical to the amino acid sequence represented by SEQ ID NO: 2 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 1 and having activity of regulating the transcription of a gene that is under the control of a cAMP responsive element;
(C) a polypeptide comprising an amino acid sequence identical to or substantially identical to the amino acid sequence represented by SEQ ID NO: 4, or a polypeptide comprising an amino acid sequence encoded by cDNA capable of hybridizing to the nucleotide sequence represented by SEQ ID NO: 3;
(D) a polypeptide comprising an amino acid sequence identical to or substantially identical to the amino acid sequence represented by SEQ ID NO: 4 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 3 and having activity of regulating the transcription of a gene that is under the control of a cAMP responsive element;
(E) a polypeptide comprising an amino acid sequence identical to or substantially identical to the amino acid sequence represented by SEQ ID NO: 6, or a polypeptide comprising an amino acid sequence encoded by cDNA capable of hybridizing to the nucleotide sequence represented by SEQ ID NO: 5;
(F) a polypeptide comprising an amino acid sequence identical to or substantially identical to the amino acid sequence represented by SEQ ID NO: 6 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 5 and having activity of regulating the transcription of a gene that is under the control of a cAMP responsive element;
(G) a polypeptide comprising an amino acid sequence identical to or substantially identical to the amino acid sequence represented by SEQ ID NO: 8, or a polypeptide comprising an amino acid sequence encoded by cDNA capable of hybridizing to the nucleotide sequence represented by SEQ ID NO: 7;
(H) a polypeptide comprising an amino acid sequence identical to or substantially identical to the amino acid sequence represented by SEQ ID NO: 8 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 7 and having activity of regulating the transcription of a gene that is under the control of a cAMP responsive element;
(I) a polypeptide comprising an amino acid sequence identical to or substantially identical to the amino acid sequence represented by SEQ ID NO: 10, or a polypeptide comprising an amino acid sequence encoded by cDNA capable of hybridizing to the nucleotide sequence represented by SEQ ID NO: 9;
(J) a polypeptide comprising an amino acid sequence identical to or substantially identical to the amino acid sequence represented by SEQ ID NO: 10 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 9 and having activity of regulating the transcription of a gene that is under the control of a cAMP responsive element;
(K) a polypeptide comprising an amino acid sequence identical to or substantially identical to the amino acid sequence represented by SEQ ID NO: 12, or a polypeptide comprising an amino acid sequence encoded by cDNA capable of hybridizing to the nucleotide sequence represented by SEQ ID NO: 11; and
(L) a polypeptide comprising an amino acid sequence identical to or substantially identical to the amino acid sequence represented by SEQ ID NO: 12 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 11 and having activity of regulating the transcription of a gene that is under the control of a cAMP responsive element.

In addition, according to the present invention, there is provided a polynucleotide comprising a nucleotide sequence having homology of at least 95% to any one of the polynucleotides described in the following (a) to (m) :
(a) a polynucleotide comprising the nucleotide sequence represented by SEQ ID NO: 1, or a polynucleotide that is cDNA capable of hybridizing to the polynucleotide comprising the nucleotide sequence represented by SEQ ID NO: 1;
(b) a polynucleotide comprising the nucleotide sequence represented by SEQ ID NO: 3, or a polynucleotide that is cDNA capable of hybridizing to the polynucleotide comprising the nucleotide sequence represented by SEQ ID NO: 3;
(c) a polynucleotide comprising the nucleotide sequence represented by SEQ ID NO: 5, or a polynucleotide that is cDNA capable of hybridizing to the polynucleotide comprising the nucleotide sequence represented by SEQ ID NO: 5;
(d) a polynucleotide comprising the nucleotide sequence represented by SEQ ID NO: 7, or a polynucleotide that is cDNA capable of hybridizing to the polynucleotide comprising the nucleotide sequence represented by SEQ ID NO: 7;
(e) a polynucleotide comprising the nucleotide sequence represented by SEQ ID NO: 9, or a polynucleotide that is cDNA capable of hybridizing to the polynucleotide comprising the nucleotide sequence represented by SEQ ID NO: 9;
(f) a polynucleotide comprising the nucleotide sequence represented by SEQ ID NO: 11, or a polynucleotide that is cDNA capable of hybridizing to the polynucleotide comprising the nucleotide sequence represented by SEQ ID NO: 11;
(g) a polynucleotide comprising a nucleotide sequence encoding a polypeptide comprising an amino acid sequence identical to or substantially identical to the amino acid sequence represented by SEQ ID NO: 2, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 2;
(h) a polynucleotide comprising a nucleotide sequence encoding a polypeptide comprising an amino acid sequence identical to or substantially identical to the amino acid sequence represented by SEQ ID NO: 4, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 4;
(i) a polynucleotide comprising a nucleotide sequence encoding a polypeptide comprising an amino acid sequence identical to or substantially identical to the amino acid sequence represented by SEQ ID NO: 6, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 6;
(j) a polynucleotide comprising a nucleotide sequence encoding a polypeptide comprising an amino acid sequence identical to or substantially identical to the amino acid sequence represented by SEQ ID NO: 8, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 8;
(k) a polynucleotide comprising a nucleotide sequence encoding a polypeptide comprising an amino acid sequence identical to or substantially identical to the amino acid sequence represented by SEQ ID NO: 10, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 10;
(l) a polynucleotide comprising a nucleotide sequence encoding a polypeptide comprising an amino acid sequence identical to or substantially identical to the amino acid sequence represented by SEQ ID NO: 12, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 12; and
(m) a polynucleotide capable of hybridizing to any one of the polynucleotides described in (a) to (l) above under stringent conditions.

Moreover, according to the present invention, there is provided a recombinant vector and an expression vector comprising the above described polynucleotide.

Furthermore, according to the present invention, there is provided host cells harboring the above described expression vector.

Still further, according to the present invention, there is provided a method for producing the above described polypeptide or a salt thereof, which comprises culturing the above described host cells under conditions that are suitable for the expression of the polypeptide, and recovering the polypeptide from the culture product obtained.

Still further, according to the present invention, there is provided a nucleic acid probe, which is useful for detecting the above described nucleic acid or a polynucleotide encoding the above described polypeptide.

Still further, according to the present invention, there is provided an antibody having an affinity for the above described polypeptide, and a hybridoma capable of generating an antibody having an affinity for the above described polypeptide.

Still further, according to the present invention, there is provided a pharmaceutical composition, which comprises the above described polypeptide or the recombinant vector of the present invention.

Still further, according to the present invention, there is provided a pharmaceutical composition, which comprises the above described antibody, a fragment thereof, or an antisense nucleotide complementarily.

Still further, according to the present invention, there is provided a composition for use in gene diagnosis, which comprises.

Still further, according to the present invention, there is provided a method for preventing or improving a disease or physiological condition, which comprises administration of the above described pharmaceutical composition.

Still further, according to the present invention, there is provided a method for screening a compound capable of promoting or inhibiting the activity of the above described polypeptide, comprising the steps of: allowing an analyte comprising the above described polypeptide to come into contact with a test compound; and detecting an activity of promoting or inhibiting the activity of the above described polypeptide.

Still further, according to the present invention, there is provided a method for screening a compound capable of promoting or inhibiting the activity of the polypeptide of the present invention, the method comprising steps of: allowing an expression vector comprising the above described polypeptide and a reporter gene that is under the control of a cAMP responsive element to come into contact with a test compound; and detecting an activity of promoting or inhibiting the activity of the polypeptide of the present invention.

Still further, according to the present invention, there is provided a pharmaceutical composition, which comprises the compound capable of promoting or inhibiting the activity of the above described polypeptide, which is identified by the above described screening method.

Still further, according to the present invention, there is provided a method for preventing or improving a disease or physiological condition which is developed by an increase in the expression of salt-inducible kinase 2, characterized in that the method comprises administration of the above described pharmaceutical composition.

Still further, according to the present invention, there is provided a method for preventing or improving a disease or physiological condition which is developed by a decrease in the expression of salt-inducible kinase 2, characterized in that the method comprises administration of the above described pharmaceutical composition.

Still further, according to the present invention, there is provided a method for screening a compound specifically binding to the above described polypeptide, comprising steps of: allowing the above described polypeptide to come into contact with a test compound; and detecting the binding of the test compound with the polypeptide, thereby identifying a compound specifically binding to the polypeptide.

Still further, according to the present invention, there is provided a method for screening a compound having an ability to regulate the activity of the above described polypeptide, the method comprising steps of: allowing the above described polypeptide to come into contact with a test compound under conditions where the polypeptide exhibits its activity; evaluating the activity of the polypeptide in the presence of the test compound; comparing the thus evaluated activity with the activity of the polypeptide in the absence of the test compound; and identifying the ability of the test compound to regulate the activity of the polypeptide based on the comparison results.

Still further, according to the present invention, there is provided a method for screening a compound having effects on the expression of the above described polynucleotide, comprising steps of allowing a target polynucleotide analyte containing the above described polynucleotide to come into contact with a test compound under conditions that are suitable for the expression of the above described target polynucleotide, detecting a change in the expression of the above described target nucleotide, and comparing the expression of the above described target polynucleotide in the absence of the above described test compound and in the presence of various amounts of the above described test compounds.

Still further, according to the present invention, there is provided a method for screening a compound capable of promoting or inhibiting the activity of a protein having an auto-phosphorylation ability, comprising: a phosphorylating step of phosphorylating a protein having auto-phosphorylation ability in the presence of a test compound; an antibody-binding step of allowing an antibody recognizing an auto-phosphorylated portion of the protein that is in a state where it has been phosphorylated or has not been phosphorylated, to react with the protein; and a measuring step of measuring the reaction of the protein with the antibody.

Still further, according to the present invention, there is provided a method for screening a compound capable of promoting or inhibiting the activity of a protein having an auto-phosphorylation ability, comprising: a phosphorylating step of phosphorylating a protein having auto-phosphorylation ability in the presence of a test compound; an antibody-binding step of allowing an antibody recognizing an auto-phosphorylated portion of the protein that is in a state where it has been phosphorylated or has not been phosphorylated, to react with the protein; and a measuring step of measuring the reaction of the protein with the antibody.

Still further, according to the present invention, there is provided a method for screening a compound capable of promoting or inhibiting the activity of a protein having an auto-phosphorylation ability, comprising: a step of culturing cells having an ability to generate a protein having an auto-phosphorylation ability in the presence of a test compound under conditions that are suitable for the expression of the protein; an antibody-binding step of allowing the cells to come into contact with an antibody recognizing an auto-phosphorylated portion of the protein that is in a state where it has been phosphorylated or has not been phosphorylated, so as to allow the protein to react with the antibody; and a measuring step of detecting the reaction of the protein with the antibody.

Still further, according to the present invention, there is provided a method for screening a compound capable of promoting or inhibiting the activity of salt-inducible kinase, comprising: a phosphorylating step of allowing salt-inducible kinase to come into contact with a substrate that is to be phosphorylated with the salt-inducible kinase in the presence of a test compound, so as to phosphorylate the substrate; an antibody-binding step of allowing an antibody recognizing a substrate that is in a state where it has been phosphorylated or has not been phosphorylated, to react with the substrate; and a measuring step of detecting the reaction of the substrate with the antibody.

Still further, according to the present invention, there is provided a kit for screening a compound capable of promoting or inhibiting the activity of the above described polypeptide, comprising the above described polypeptide.

Still further, according to the present invention, there is provided a kit for screening a compound capable of promoting or inhibiting the activity of a protein having an auto-phosphorylation ability, comprising a protein having an auto-phosphorylation ability and an antibody recognizing an auto-phosphorylated portion of the protein that is in a state where it has been phosphorylated or has not been phosphorylated.

### Brief Description of the Drawings

Figure 1 shows the amino acid sequences of rat SIK1 and mouse SIK2 proteins;
Figure 2 shows the results obtained by examining the presence of SIK1 mRNA and SIK2 mRNA in various tissues;
Figure 3 shows the results obtained by comparing the protein-phosphorylating activity of rat SIK1 with that of mouse SIK2;
Figure 4 shows the results obtained by comparing the CRE-suppressing activity of rat SIK1 with that of mouse SIK2;
Figure 5 shows the results showing the protein-phosphorylating activity of mouse SIK2 that is inhibited by staurosporine;
Figure 6 shows the results obtained by detecting an antibody;
Figure 7 shows the results showing the reactivity between an antibody and a peptide;
Figure 8 is a view showing the results obtained by analyzing an SIK inhibitor;
Figure 9 shows the results obtained by comparison of CRE-suppressing activity;
Figure 10 shows the results regarding the intracellular distribution of rat SIK1 and mouse SIK2 in 3T3-L1 cells;
Figure 11 shows the results obtained by assaying the mRNA level of various types of fat cell differentiation markers, SIK1 and SIK2, which is induced during a fat cell differentiation process;
Figure 12 shows the results obtained by comparing the protein-phosphorylating activity of SIK2 to GST-human IRS1 with that to GST-human IRS1 (S794A);
Figure 13 shows the results obtained by examining the amounts of SIK2 mRNA and SIK2 protein in various types of cells and the activity of SIK;
Figure 14 shows the results obtained by examining an auto-phosphorylated portion of SIK;
Figure 15 shows the results showing the reaction of an antibody recognizing double phosphorylation with phosphorylated SIK; and
Figure 16 shows the results obtained by reacting various antibodies with SIK in the presence of an SIK inhibitor.

### Best Mode for Carrying Out the Invention

The present invention will be described in detail below.

First, the polypeptide of the present invention will be explained.

The polypeptide of the present invention is a polypeptide comprising an amino acid sequence having homology of at least 90% to any one of the polypeptides described in the following (A) to (L), or a salt thereof:
(A) a polypeptide comprising an amino acid sequence identical to or substantially identical to the amino acid sequence represented by SEQ ID NO: 2, or a polypeptide comprising an amino acid sequence encoded by cDNA capable of hybridizing to the nucleotide sequence represented by SEQ ID NO: 1;
(B) a polypeptide comprising an amino acid sequence identical to or substantially identical to the amino acid sequence represented by SEQ ID NO: 2 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 1 and having activity of regulating the transcription of a gene that is under the control of a cAMP responsive element;
(C) a polypeptide comprising an amino acid sequence identical to or substantially identical to the amino acid sequence represented by SEQ ID NO: 4, or a polypeptide comprising an amino acid sequence encoded by cDNA capable of hybridizing to the nucleotide sequence represented by SEQ ID NO: 3;
(D) a polypeptide comprising an amino acid sequence identical to or substantially identical to the amino acid sequence represented by SEQ ID NO: 4 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 3 and having activity of regulating the transcription of a gene that is under the control of a cAMP responsive element;
(E) a polypeptide comprising an amino acid sequence identical to or substantially identical to the amino acid sequence represented by SEQ ID NO: 6, or a polypeptide comprising an amino acid sequence encoded by cDNA capable of hybridizing to the nucleotide sequence represented by SEQ ID NO: 5;
(F) a polypeptide comprising an amino acid sequence identical to or substantially identical to the amino acid sequence represented by SEQ ID NO: 6 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 5 and having activity of regulating the transcription of a gene that is under the control of a cAMP responsive element;
(G) a polypeptide comprising an amino acid sequence identical to or substantially identical to the amino acid sequence represented by SEQ ID NO: 8, or a polypeptide comprising an amino acid sequence encoded by cDNA capable of hybridizing to the nucleotide sequence represented by SEQ ID NO: 7;
(H) a polypeptide comprising an amino acid sequence identical to or substantially identical to the amino acid sequence represented by SEQ ID NO: 8 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 7 and having activity of regulating the transcription of a gene that is under the control of a cAMP responsive element;
(I) a polypeptide comprising an amino acid sequence identical to or substantially identical to the amino acid sequence represented by SEQ ID NO: 10, or a polypeptide comprising an amino acid sequence encoded by cDNA capable of hybridizing to the nucleotide sequence represented by SEQ ID NO: 9;
(J) a polypeptide comprising an amino acid sequence identical to or substantially identical to the amino acid sequence represented by SEQ ID NO: 10 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 9 and having activity of regulating the transcription of a gene that is under the control of a cAMP responsive element;
(K) a polypeptide comprising an amino acid sequence identical to or substantially identical to the amino acid sequence represented by SEQ ID NO: 12, or a polypeptide comprising an amino acid sequence encoded by cDNA capable of hybridizing to the nucleotide sequence represented by SEQ ID NO: 11; and
(L) a polypeptide comprising an amino acid sequence identical to or substantially identical to the amino acid sequence represented by SEQ ID NO: 12 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 11 and having activity of regulating the transcription of a gene that is under the control of a cAMP responsive element.

The term "substantially identical" as used herein means that the activity of a polypeptide is substantially identical to that of another polypeptide. When several amino acids are deleted, substituted, or added, if a polypeptide having such a deletion, substitution, or addition has the same activity as that of another polypeptide that does not comprise such a deletion, substitution, or addition, both polypeptides are substantially identical to each other. In general, when a polypeptide has an amino acid sequence having homology of 90%, and preferably of 95% to the entire amino acid sequence of another polypeptide, it is then understood that both polypeptides are substantially identical to each other. In general, a polypeptide comprising an amino acid sequence containing a deletion, substitution, or addition of a part of amino acids (preferably 1 to 20, more preferably 1 to 10, and most preferably several amino acids) is substantially identical to another polypeptide having the entire amino acid sequence.

The polypeptide of the present invention will be explained below.

The polypeptide of the present invention is a polypeptide comprising an amino acid sequence having homology of at least 90% to any one of the polypeptides described in the following (A) to (L) above, or a salt thereof.

The polypeptide comprising the amino acid sequence represented by SEQ ID NO: 2 means a peptide sequence consisting of 931 amino acids from methionine at the N-terminus of salt-inducible kinase 2 (SIK2) derived from a mouse to threonine at the C-terminus thereof. A polypeptide comprising an amino acid sequence substantially identical to the above polypeptide is considered to be identical to a polypeptide comprising an amino acid sequence having homology of approximately 90%, and preferably 95% or more to the entire amino acid sequence represented by SEQ ID NO: 2. Accordingly, a polypeptide having a deletion, substitution, or addition of a part of amino acids (preferably 1 to 20, more preferably 1 to 10, and most preferably several amino acids) with respect to the amino acid sequence represented by SEQ ID NO: 2 is also considered to be substantially identical. As a DNA encoding such a polypeptide, cDNA capable of hybridizing to the nucleotide sequence represented by SEQ ID NO: 1 is used.

A polypeptide comprising an amino acid sequence identical to or substantially identical to the polypeptide comprising the amino acid sequence represented by SEQ ID NO: 2 plays a role in the protein-phosphorylating activity of SIK2. At the same time, when such a polypeptide is allowed to express in common cultured cells including fat cells, it has an activity of regulating the transcription of a gene that is under a cAMP responsive element (CRE). That is, a polypeptide comprising an amino acid sequence identical to or substantially identical to the polypeptide comprising the amino acid sequence represented by SEQ ID NO: 2 plays a role in the protein-phosphorylating activity of SIK2, as well as having activity of suppressing the transcription of a gene that is under a cAMP responsive element (CRE) when such a polypeptide is allowed to express in common cultured cells including fat cells.

The polypeptide comprising the amino acid sequence represented by SEQ ID NO: 4 means a peptide sequence that is a regulating domain portion (domain 3 portion) consisting of 47 amino acids from proline at position 577 of salt-inducible kinase 2 (SIK2) derived from a mouse to glutamine at position 623 thereof, which corresponds to the polypeptide represented by SEQ ID NO: 2. A polypeptide comprising an amino acid sequence substantially identical to the above polypeptide is considered to be identical to a polypeptide comprising an amino acid sequence having homology of approximately 90%, and preferably 95% or more to the entire amino acid sequence represented by SEQ ID NO: 4. Accordingly, a polypeptide having a deletion, substitution, or addition of a part of amino acids (preferably 1 to 5 amino acids) with respect to the amino acid sequence represented by SEQ ID NO: 4 is also considered to be substantially identical. As DNA encoding such a polypeptide, cDNA capable of hybridizing to the nucleotide sequence represented by SEQ ID NO: 3 is used.

A polypeptide comprising an amino acid sequence identical to or substantially identical to the polypeptide comprising the amino acid sequence represented by SEQ ID NO: 4 regulates the protein-phosphorylating activity of SIK2. At the same time, when such a polypeptide is allowed to express in common cultured cells including fat cells, it undergoes phosphorylation, thereby having activity of regulating the transcription of a gene that is under a cAMP responsive element (CRE). That is, a polypeptide comprising an amino acid sequence identical to or substantially identical to the polypeptide comprising the amino acid sequence represented by SEQ ID NO: 4 regulates the protein-phosphorylating activity of SIK2. Moreover, when such a polypeptide is allowed to express in common cultured cells including fat cells, it undergoes phosphorylation, and as a result, it has functions as a modulator for the transcriptional activity of a gene that is under a cAMP responsive element (CRE).

The polypeptide comprising the amino acid sequence represented by SEQ ID NO: 6 means a peptide sequence that is a domain portion having kinase activity (domain 1 portion) consisting of 252 amino acids from tyrosine at position 20 of salt-inducible kinase 2 (SIK2) derived from a mouse to methionine at position 271 thereof, which corresponds to the polypeptide represented by SEQ ID NO: 2. A polypeptide comprising an amino acid sequence substantially identical to the above polypeptide is considered to be identical to a polypeptide comprising an amino acid sequence having homology of approximately 90%, and preferably 95% or more to the entire amino acid sequence represented by SEQ ID NO: 6. Accordingly, a polypeptide having a deletion, substitution, or addition of a part of amino acids (preferably 1 to 20, more preferably 1 to 10, and most preferably several amino acids) with respect to the amino acid sequence represented by SEQ ID NO: 6 is also considered to be substantially identical. As a DNA encoding such a polypeptide, cDNA capable of hybridizing to the nucleotide sequence represented by SEQ ID NO: 5 is used.

A polypeptide comprising an amino acid sequence identical to or substantially identical to the polypeptide comprising the amino acid sequence represented by SEQ ID NO: 6 plays a role in the protein-phosphorylating activity of SIK2. At the same time, when such a polypeptide is allowed to express in common cultured cells including fat cells, it has activity of regulating the transcription of a gene that is under a cAMP responsive element (CRE). That is, a polypeptide comprising an amino acid sequence identical to or substantially identical to the polypeptide comprising the amino acid sequence represented by SEQ ID NO: 6 plays a role in the protein-phosphorylating activity of SIK2, as well as having activity of suppressing the transcription of a gene that is under the control of a cAMP responsive element (CRE) when such a polypeptide is allowed to express in common cultured cells including fat cells. Moreover, when the polypeptide comprising the amino acid sequence represented by SEQ ID NO: 6 loses its phosphorylation activity, it increases the transcription of a gene that is under the control of CRE.

The polypeptide comprising the amino acid sequence represented by SEQ ID NO: 8 means a peptide sequence that is a stabilizing domain portion (domain 2 portion) consisting of 53 amino acids from isoleucine at position 293 of salt-inducible kinase 2 (SIK2) derived from a mouse to proline at position 345 thereof, which corresponds to the polypeptide represented by SEQ ID NO: 2. A polypeptide comprising an amino acid sequence substantially identical to the above polypeptide is considered to be identical to a polypeptide comprising an amino acid sequence having homology of approximately 90%, and preferably 95% or more to the entire amino acid sequence represented by SEQ ID NO: 8. Accordingly, a polypeptide having a deletion, substitution, or addition of a part of amino acids (preferably 1 to 5 amino acids) with respect to the amino acid sequence represented by SEQ ID NO: 8 is also considered to be substantially identical. As a DNA encoding such a polypeptide, cDNA capable of hybridizing to the nucleotide sequence represented by SEQ ID NO: 7 is used. A polypeptide comprising an amino acid sequence identical to or substantially identical to the polypeptide comprising the amino acid sequence represented by SEQ ID NO: 8 plays a role in stabilization of a protein. At the same time, when such a polypeptide is allowed to express in common cultured cells including fat cells, it undergoes phosphorylation, thereby having activity of regulating the transcription of a gene that is under a cAMP responsive element (CRE). That is, a polypeptide comprising an amino acid sequence identical to or substantially identical to the polypeptide comprising the amino acid sequence represented by SEQ ID NO: 8 plays a role in stabilization of a protein. Moreover, when such a polypeptide is allowed to express in common cultured cells including fat cells, it undergoes phosphorylation, and as a result, it has functions as a modulator for the transcriptional activity of a gene that is under a cAMP responsive element (CRE).

The polypeptide comprising the amino acid sequence represented by SEQ ID NO: 10 means a peptide sequence that is a regulating domain portion (domain 3a portion) consisting of 18 amino acids from proline at position 577 of salt-inducible kinase 2 (SIK2) derived from a mouse to glycine at position 594 thereof, which corresponds to the polypeptide represented by SEQ ID NO: 2. A polypeptide comprising an amino acid sequence substantially identical to the above polypeptide is considered to be identical to a polypeptide comprising an amino acid sequence having homology of approximately 90%, and preferably 95% or more to the entire amino acid sequence represented by SEQ ID NO: 10. Accordingly, a polypeptide having a deletion, substitution, or addition of a part of amino acids (preferably 1 to 2 amino acids) with respect to the amino acid sequence represented by SEQ ID NO: 10 is also considered to be substantially identical. As a DNA encoding such a polypeptide, cDNA capable of hybridizing to the nucleotide sequence represented by SEQ ID NO: 9 is used. A polypeptide comprising an amino acid sequence identical to or substantially identical to the polypeptide comprising the amino acid sequence represented by SEQ ID NO: 10 is involved in elimination of an SIK2 protein from the nucleus. At the same time, when such a polypeptide is allowed to express in common cultured cells including fat cells, it undergoes phosphorylation, thereby having activity of regulating the transcription of a gene that is under a cAMP responsive element (CRE). That is, a polypeptide comprising an amino acid sequence identical to or substantially identical to the polypeptide comprising the amino acid sequence represented by SEQ ID NO: 10 is involved in elimination of an SIK2 protein from the nucleus. Moreover, when such a polypeptide is allowed to express in common cultured cells including fat cells, it undergoes phosphorylation, and as a result, it has functions as a modulator for the transcriptional activity of a gene that is under a cAMP responsive element (CRE).

The polypeptide comprising the amino acid sequence represented by SEQ ID NO: 12 means a peptide sequence that is a regulating domain portion (domain 3b portion) consisting of 29 amino acids from isoleucine at position 595 of salt-inducible kinase 2 (SIK2) derived from a mouse to glutamine at position 623 thereof, which corresponds to the polypeptide represented by SEQ ID NO: 2. A polypeptide comprising an amino acid sequence substantially identical to the above polypeptide is considered to be identical to a polypeptide comprising an amino acid sequence having homology of approximately 90%, and preferably 95% or more to the entire amino acid sequence represented by SEQ ID NO: 12. Accordingly, a polypeptide having a deletion, substitution, or addition of a part of amino acids (preferably 1 to 2 amino acids) with respect to the amino acid sequence represented by SEQ ID NO: 12 is also considered to be substantially identical. As a DNA encoding such a polypeptide, cDNA capable of hybridizing to the nucleotide sequence represented by SEQ ID NO: 11 is used. A polypeptide comprising an amino acid sequence identical to or substantially identical to the polypeptide comprising the amino acid sequence represented by SEQ ID NO: 12 is involved in cytoplasmic localization of an SIK2 protein.

Polypeptides represented by SEQ ID NOS: 2, 4, 6, 8, 10, and 12 are specifically expressed in fat cells of mice and other warm-blooded animals (e.g. a human, a rat, a chicken, a rabbit, a swine, a sheep, a goat, a bovine, a horse, a monkey, etc.). Accordingly, a polypeptide comprising an amino acid sequence identical to or substantially identical to the amino acid sequence represented by SEQ ID NO: 2, 4, 6, 8, 10, or 12, and especially represented by SEQ ID NO: 4, may be a polypeptide derived from an SIK2 protein collected from mouse tissues or cells, an SIK homologue collected from tissues or cells of warm-blooded animals other than a mouse, a polypeptide produced by genetic engineering based on the nucleotide sequence encoding the above polypeptide, or a polypeptide chemically synthesized based on the above amino acid sequence.

A polypeptide comprising an amino acid sequence identical to or substantially identical to the amino acid sequence represented by SEQ ID NO: 2, 4, 6, 8, 10, or 12 has activity of regulating the transcription of a gene that is under the control of CRE. Specifically, it is a polypeptide having homology of approximately 90% or more, and preferably approximately 95% or more to the amino acid sequence represented by SEQ ID NO: 2, 4, 6, 8, 10, or 12. Such a polypeptide comprising an amino acid sequence substantially identical to the amino acid sequence represented by SEQ ID NO: 2, 4, 6, 8, 10, or 12 includes: a polypeptide comprising an amino acid sequence comprising a deletion, insertion, or addition of 1 or 2 or more amino acids with respect to the amino acid sequence represented by SEQ IDNO: 2, 4, 6, 8, 10, or 12; a polypeptide comprising an amino acid sequence comprising 1 or 2 amino acids that are substituted with other amino acids; a polypeptide comprising an amino acid sequence that is modified with phosphoric acid, sugar chain, fatty acid, or the like; and a polypeptide comprising an amino acid sequence that is made by the combination thereof. In addition, such a polypeptide comprising an amino acid sequence substantially identical to the amino acid sequence represented by SEQ ID NO: 2, 4, 6, 8, 10, or 12 also includes a fusion protein used during the expression of a protein, such as glutathione S-transferase (GST), maltose-binding protein (MBP), and SIK2 to which a histidine hexamer or the like binds.

In the polypeptide of the present invention, the left terminus is an N-terminus (amino group terminus) and the right terminus is a C-terminus (carboxyl group terminus). The C-terminus may be any one of a carboxyl group (-COOH), carboxylate (-COO-), amide (-CONH₂), and ester (-COOR). Examples of R in the above ester may include: alkyl groups containing 1 to 6 carbon atoms such as methyl, ethyl, n-propyl, isopropyl, or n-butyl; cycloalkyl groups containing 3 to 8 carbon atoms such as cyclopentyl or cyclohexyl; aryl groups; and aralkyl groups. Moreover, the polypeptide of the present invention also includes: polypeptides the amino acid group of the amino acid residue at the N-terminus of which is protected by a protecting group; polypeptides a substituent on the side chain of amino acid in a molecule of which is protected by a suitable protecting group; complex proteins such as a glycoprotein to which a sugar chain binds; and others.

Examples of a salt of the polypeptide of the present invention may include salts formed with physiologically acceptable inorganic acids and organic acids, and salts formed with bases such as alkali metal salts. Physiologically acceptable acid addition salts are preferable. Examples of such an inorganic acid may include hydrochloric acid, phosphoric acid, hydrobromic acid, and sulfuric acid. Examples of such an organic acid may include acetic acid, formic acid, propionic acid, fumaric acid, maleic acid, succinic acid, tartaric acid, citric acid, malic acid, oxalic acid, benzonic acid, methanesulfonic acid, and benzenesulfonic acid.

The polypeptide of the present invention or a salt thereof can be produced by: homogenizing the cells or tissues from a warm-blooded animal as mentioned above; extracting them with acid or the like; isolating and purifying an SIK2 protein from the obtained extract by known protein purification methods including the combined use of various types of chromatography such as reverse phase chromatography or ion exchange chromatography; and preparing, as necessary, a polypeptide containing a portion corresponding to the amino acid sequence represented by SEQ ID NO: 2, 4, 6, 8, 10, or 12, using peptidase or the like. Otherwise, a DNA sequence encoding the above polypeptide is prepared by the reverse transcription polymerase chain reaction method (RT-PCR method) or the like, and it is then inserted into a suitable expression vector. Thereafter, the expression vector is introduced into host cells. The obtained transformant is cultured, so as to produce a polypeptide of interest. That is to say, the polypeptide of the present invention may also be a recombinant protein. The culture of the above host cells may also be carried out in the presence of a substance having an action to induce fat differentiation. By carrying out the culture of the above host cells in the presence of a substance having an action to induce fat differentiation, mRNA encoding the polypeptide of the present invention is induced, thereby promoting the generation of the polypeptide of the present invention. Thus, the use of a substance having an action to induce fat differentiation is preferable.

Also, precursor fat cells are cultured in the presence of a substance having an action to induce fat differentiation, and then, the polypeptide of the present invention or a salt thereof can be produced from the thus obtained culture product. In this case also, mRNA encoding the polypeptide of the present invention is induced by the substance having an action to induce fat differentiation, thereby promoting the generation of the polypeptide of the present invention. Examples of such a substance having an action to induce fat differentiation may include dexamethasone, insulin, cAMP, and a thiazolidine derivative.

Moreover, the polypeptide of the present invention can also be produced by chemical synthesis using a commercially available protein synthetic resin.

A polypeptide corresponding to domain 3 represented by SEQ ID NO: 4 or a salt thereof can be produced by a previously known peptide synthesis method. It can also be produced by cleaving the polypeptide represented by SEQ ID NO: 2 with a suitable peptidase.

A polypeptide corresponding to domain 1 represented by SEQ ID NO: 6 or a salt thereof can be produced by a previously known peptide synthesis method. It can also be produced by cleaving the polypeptide represented by SEQ ID NO: 2 with a suitable peptidase.

A polypeptide corresponding to domain 2 represented by SEQ ID NO: 8 or a salt thereof can be produced by a previously known peptide synthesis method. It can also be produced by cleaving the polypeptide represented by SEQ ID NO: 2 with a suitable peptidase.

A polypeptide corresponding to domain 3a represented by SEQ ID NO: 10 or a salt thereof can be produced by a previously known peptide synthesis method. It can also be produced by cleaving the polypeptide represented by SEQ ID NO: 2 or 4 with a suitable peptidase.

A polypeptide corresponding to domain 3b represented by SEQ ID NO: 12 or a salt thereof can be produced by a previously known peptide synthesis method. It can also be produced by cleaving the polypeptide represented by SEQ ID NO: 2 or 4 with a suitable peptidase.

When the obtained polypeptide is a loose body, it can be converted into a suitable salt by known methods. When the peptide is obtained in the form of a salt, it can be converted into a loose body or other salts by known methods.

Any polynucleotide can be used as a polynucleotide encoding the polypeptide of the present invention, as long as it has a nucleotide sequence encoding the aforementioned polypeptide of the present invention. It may also be genomic DNA, mRNA derived from the aforementioned cells or tissues, cDNA prepared from the mRNA using reverse transcriptase, synthetic DNA, double-stranded RNA, or an RNA-DNA hybrid.

Next, the polynucleotide of the present invention will be described.

The polynucleotide of the present invention has a polynucleotide comprising a nucleotide sequence having homology of at least 95% to any one of the polynucleotides described in the following (a) to (m):
(a) a polynucleotide comprising the nucleotide sequence represented by SEQ ID NO: 1, or a polynucleotide that is cDNA capable of hybridizing to the polynucleotide comprising the nucleotide sequence represented by SEQ ID NO: 1;
(b) a polynucleotide comprising the nucleotide sequence represented by SEQ ID NO: 3, or a polynucleotide that is cDNA capable of hybridizing to the polynucleotide comprising the nucleotide sequence represented by SEQ ID NO: 3;
(c) a polynucleotide comprising the nucleotide sequence represented by SEQ ID NO: 5, or a polynucleotide that is cDNA capable of hybridizing to the polynucleotide comprising the nucleotide sequence represented by SEQ ID NO: 5;
(d) a polynucleotide comprising the nucleotide sequence represented by SEQ ID NO: 7, or a polynucleotide that is cDNA capable of hybridizing to the polynucleotide comprising the nucleotide sequence represented by SEQ ID NO: 7;
(e) a polynucleotide comprising the nucleotide sequence represented by SEQ ID NO: 9, or a polynucleotide that is cDNA capable of hybridizing to the polynucleotide comprising the nucleotide sequence represented by SEQ ID NO: 9;
(f) a polynucleotide comprising the nucleotide sequence represented by SEQ ID NO: 11, or a polynucleotide that is cDNA capable of hybridizing to the polynucleotide comprising the nucleotide sequence represented by SEQ ID NO: 11;
(g) a polynucleotide comprising a nucleotide sequence encoding a polypeptide comprising an amino acid sequence identical to or substantially identical to the amino acid sequence represented by SEQ ID NO: 2, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 2;
(h) a polynucleotide comprising a nucleotide sequence encoding a polypeptide comprising an amino acid sequence identical to or substantially identical to the amino acid sequence represented by SEQ ID NO: 4, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 4;
(i) a polynucleotide comprising a nucleotide sequence encoding a polypeptide comprising an amino acid sequence identical to or substantially identical to the amino acid sequence represented by SEQ ID NO: 6, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 6;
(j) a polynucleotide comprising a nucleotide sequence encoding a polypeptide comprising an amino acid sequence identical to or substantially identical to the amino acid sequence represented by SEQ ID NO: 8, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 8;
(k) a polynucleotide comprising a nucleotide sequence encoding a polypeptide comprising an amino acid sequence identical to or substantially identical to the amino acid sequence represented by SEQ ID NO: 10, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 10;
(l) a polynucleotide comprising a nucleotide sequence encoding a polypeptide comprising an amino acid sequence identical to or substantially identical to the amino acid sequence represented by SEQ ID NO: 12, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 12; and
(m) a polynucleotide capable of hybridizing to any one of the polynucleotides described in (a) to (l) above under stringent conditions.

The polynucleotide of the present invention encodes the aforementioned polypeptide of the present invention. Specifically, the polynucleotide of the present invention includes a polynucleotide that is cDNA capable of hybridizing to the polynucleotide represented by SEQ ID NO: 1, 3, 5, 7, 9, or 11.

In addition, the polynucleotide of the present invention includes a polynucleotide that is cDNA capable of hybridizing to a polynucleotide comprising a nucleotide sequence encoding a polypeptide comprising an amino acid sequence identical to or substantially identical to the amino acid sequence represented by SEQ ID NO: 2, 4, 6, 8, 10, or 12.

Moreover, the polynucleotide of the present invention includes a polynucleotide capable of hybridizing to any one of the above described polynucleotides under stringent conditions.

A polypeptide comprising an amino acid sequence identical to or substantially identical to the aforementioned amino acid sequence represented by SEQ ID NO: 2, 4, 6, 8, 10, or 12 has activity of regulating the transcription of a gene that is under the control of a cAMP responsive element (CRE), when it is allowed to express in common cultured cells including fat cells.

In the present specification, the term "stringent conditions" is used to mean conditions where a polynucleotide comprising a nucleotide sequence having homology of approximately 95% or more, and preferably 97% or more to the nucleotide sequence represented by SEQ ID NO: 1, 3, 5, 7, 9, or 11 can hybridize with any one of the above described polynucleotides. Such conditions preferably consist of a sodium concentration between approximately 19 and 20 mM and a temperature between approximately 60°C and 65°C, and more preferably consist of a sodium concentration of approximately 19 mM and a temperature of approximately 65°C. Hybridization can be carried out in accordance with known methods.

As means for cloning a polynucleotide encoding the polypeptide of the present invention, the polynucleotide can be amplified by the PCR method using synthetic DNA primers having partial nucleotide sequences of an SIK2 gene derived from a human or other warm-blooded animals. Otherwise, it is allowed to hybridize with a polynucleotide incorporated into a suitable vector, which has been then labeled with a nucleotide fragment encoding a part or the entire region of the polypeptide of the present invention or with a synthetic nucleotide, followed by selection.

The nucleotide sequence of DNA is converted, so as to produce a polypeptide into which a mutation is partially introduced. The nucleotide sequence of DNA can be converted using a commercially available kit such as Mutan™-super ExpressKm (Takara Shuzo Co., Ltd.) or Mutan™-K (Takara Shuzo Co., Ltd.).

The cloned polynucleotide of the present invention can directly be used depending on purposes. Also, it can be digested with restriction enzymes, or a linker can be added to the cloned polynucleotide, as desired, before use. The polynucleotide may have ATG acting as a translation initiation codon on the 5'-terminal side thereof, and may have TAA, TGA, or TAG acting as a translation termination codon on the 3'-terminal side thereof. These codons can also be added using a suitable synthetic adaptor.

The polynucleotide of the present invention can be used as a probe for detecting the abnormality (gene abnormality) of DNA or mRNA encoding SIK2 of warm-blooded animals such as a human, rat, mouse, Guinea pig, rabbit, chicken, sheep, swine, bovine, horse, cat, dog, monkey, or chimpanzee (the polynucleotide of the present invention, a polynucleotide encoding the polynucleotide of the present invention).

Next, the recombinant vector and expression vector of the present invention will be described.

An expression vector for the polypeptide of the present invention can be produced by cutting a polynucleotide fragment of interest from a polynucleotide encoding the polypeptide of the present invention and then ligating the polynucleotide fragment downstream of a promoter in a.suitable expression vector. Examples of a vector may include a plasmid derived from Escherichia coli (e.g. pBR322, pBR325, pUC18, pUC118, etc.), a plasmid derived from Bacillus subtilis (e.g. pUB110, pTP5, or pC194), a plasmid derived from yeast (e.g. pSH19 or pSH15), bacteriophage such as λ phage, animal viruses such as retrovinus, vaccinia virus, or baculovirus, pA1-11, pXT1, pRc/CMV, pRc/RSV, and pcDNAI/Neo. Any promoter can be used in the present invention, as long as it is compatible with a host used for gene expression. For example, when a host is Escherichia coli, a trp promoter, lac promoter, recA promoter, λPL promoter, lpp promoter, T7 promoter, T3 promoter, araBAD promoter, or the like is preferable. When a host is a bacterium belonging to genus bacillus, an SPO1 promoter, penP promoter, XYL promoter, HWP promoter, CWP promoter, or the like is preferable. When a host is Bacillus subtilis, an SPO1 promoter, SPO2 promoter, penP promoter, or the like is preferable. When a host is yeast, a PHO5 promoter, PKG promoter, GAP promoter, ADH promoter, or the like is preferable. When an animal cell is used as a host, an SRα promoter, SV40 promoter, LTR promoter, CMV promoter, HSV-TK promoter, or the like is preferably used. When an insect cell is used as a host, a polyhedrin promoter, OplE2 promoter, or the like is used.

Other than the above described promoter, products known in the present technical field, such as an enhancer, a splicing signal, a poly A addition signal, a selective marker, and an SV40 replication origin (hereinafter abbreviated as SV40 or dgi at times), can be added to the expression vector, as desired. Moreover, a protein encoded by the DNA of the present invention can be expressed in the form of a fusion protein with another protein (for example, glutathione S-transferase and protein A), as necessary. Such a fusion protein can be cleaved with site-specific protease, so as to separate into different proteins.

Examples of the aforementioned selective marker may include a dihydrofolate reductase (hereinafter abbreviated as dhfr at times) gene [methotrexate (MTX)-resistant], an ampicillin resistance gene (hereinafter abbreviated as Amp^{r} at times), and a neomycin resistance gene (hereinafter abbreviated as Neo^{r}, G418-resistant). In particular, when dhfr gene-deficient Chinese hamster cells are used together with a dhfr gene as a selective marker, a gene of interest can be selected from a medium that does not contain thymidine.

Examples of a host cell may be genus escherichia, genus bacillus, yeast, insect cells, insects, and animal cells. Specific examples of genus escherichia used herein may include Escherichia coli K12/DH1 (Proc. Natl. Acad. Sci. USA, Vol. 60, 160 (1968)), JM103 (Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)), DH5α, and JM109. Examples of genus bacillus used herein may include Bacillus subtilis MI114 (Gene, Vol. 24, 255 (1983)), 207-21 [Journal of Biochemistry, Vol. 95, 87 (1984)], and *Bacillus brevis*. Examples of yeast used here in may include Saccaromyces cerevisiae AH22, AH22R-, NA87-11A, DKD-5D, and 20B-12, Schizosaccaromyces pombe NCYC1913 and NCYC2036, *Pichia pastoris* M71, and Hansenula polymorpha. When virus is AcNPV, examples of insect cells used herein may include an established cell line derived from cabbage armyworm (Spodoptera frugiperda cells; Sf cells), MG1 cells derived from the midgut of Trichoplusia ni, High Five™ cells derived from the egg of Trichoplusiani, cells derived from *Mamestra brassicae*, and cells derived from Estigmena acrea. When virus is BmNPV, an established cell line derived from silkworm (Bombyx mori N cells, BmN cells) and the like can be used. Examples of such Sf cells used herein may include Sf9 cells (ATCC CRL1711) and Sf21 cells (described in Vaughn J. L. et al., In Vivo, 13, 213-217 (1977)). An example of an insect used herein may be silkworm [Maeda et al., Nature, Vol. 315, 592 (1985)]. Examples of mammalian cells used herein may include monkey COS-7, Vero, Chinese hamster cells CHO (hereinafter abbreviated as CHO cells), dhfr gene-deficient Chinese hamster cells CHO (hereinafter abbreviated as CHO(dhfr⁻)), mouse L cells, mouse AtT-20, mouse myeloma cells, rat GH3, and human FL cells.

Moreover, a polynucleotide encoding a signal sequence suitable for host cells may be added to the 5'-terminal side of the polynucleotide encoding the polypeptide of the present invention, as necessary. When genus escherichia is used as a host cell, a PhoA/signal sequence, OmpA/signal sequence, or the like is used. When genus bacillus is used as a host cell, an α-amylase/signal sequence, subtilisin/signal sequence, or the like is used. When yeast is used as a host cell, an MFα/signal sequence, SUC2/signal sequence, or the like is used. When animal cells are used as host cells, signal sequences such as an insulin/signal sequence, α-interferon/signal sequence, or antibody molecule/signal sequence, are used. The thus constructed expression vector containing the polynucleotide encoding the polypeptide of the present invention is used to produce a transformant.

The aforementioned host cells can be transformed in accordance with methods known in the present technical field. The transformation of genus escherichia can be carried out by the methods described, for example, in Proc. Natl. Acad. Sci. USA, Vol. 69, 2110 (1972); and Gene, Vol. 17, 107 (1982) . The transformation of genus bacillus can be carried out by the method described, for example, in Molecular & General Genetics, Vol. 168, 111 (1979). The transformation of yeast can be carried out by the methods described, for example, in Methods in Enzymology, Vol. 194, 182-187 (1991), and Proc. Natl. Acad. Sci. USA, Vol. 75, 1929 (1978). The transformation of insect cells or an insect can be carried out by the method described, for example, in Bio/Technology, 6, 47-55 (1988). The transformation of mammalian cells can be carried out by the methods described, for example, in *Saibo Kogaku Bessatsu* 8 (Cell Technology Supplementary Volume 8), *Shin-Saibo Kogaku Jikken Protocol* (New Cell Technology Experimental Protocol), 263-267 (1995) Shujunsha Co., Ltd.; and Virology, Vol. 52, 456 (1973). Thus, a transformant which has been transformed with an expression vector containing the polynucleotide encoding the polypeptide of the present invention can be obtained.

The aforementioned transformant can be cultured by methods known in the present technical field. When a transformant a host of which is genus escherichia or genus bacillus is cultured, a liquid medium is preferably used as a medium used in culture. The liquid medium may contain a carbon source, a nitrogen source, an inorganic product, and other products, which are necessary for the growth of the transformant. Examples of such a carbon source may include glucose, dextrin, soluble starch, and sucrose. Examples of such a nitrogen source may include inorganic or organic substances such as ammonium salts, nitrate salts, corn steep liquor, peptone, casein, meat extract, soybean cake, or potato extract. Examples of inorganic substances may include calcium chloride, sodium dihydrogen phosphate, and magnesium chloride. In addition, yeast extract, vitamins, a growth-promoting factor, or the like may also be added to the aforementioned liquid medium. The medium preferably has pH between approximately 5 and 8. A preferred example of a medium used in the culture of genus escherichia may be an M9 medium containing glucose and casamino acid [Miller, Journal of Experiments in Molecular Genetics, 431-433, Cold Spring Harbor Laboratory, New York, 1972]. In order to make a promoter work efficiently, agents such as 3β-indolyl acrylic acid may be added to the medium, as necessary. When a host cell is genus escherichia, the culture is generally carried out at a temperature between approximately 15°C and 43°C for about 3 to 24 hours. Thereafter, aeration or stirring may be then carried out, as necessary. When a host cell is genus Bacillus, the culture is generally carried out at a temperature between approximately 30°C and 40°C for about 6 to 24 hours. Thereafter, aeration or stirring may be then carried out, as necessary. When a transformant whose host cell is yeast is cultured, examples of a medium used herein may include a Burkholder minimum medium [Bostian K. L. et al., Proc. Natl. Acad. Sci. USA, Vol. 77, 4505 (1980)] and an SD medium containing 0.5% casamino acid [Bitter G. A. et al., Proc. Natl. Acad. Sci. USA, Vol. 81, 5330 (1984)]. The pH of such a medium is preferably adjusted to approximately 5 to 8. The culture is generally carried out at a temperature between approximately 20°C and 35°C for about 24 to 72 hours. Thereafter, aeration or stirring may be then carried out, as necessary.

When a transformant whose host cell is an insect cell or insect is cultured, a medium produced by adding, as appropriate, additives such as 10% inactivated bovine serum to a Grace's Insect Medium (Grace, T.C.C., Nature, 195, 788 (1962)), is preferably used. The pH of such a medium is preferably adjusted to approximately 6.2 to 6.4. The culture is generally carried out at a temperature of approximately 27°C for about 3 to 5 days. Thereafter, aeration or stirring may be then carried out, as necessary. When a transformant whose host cell is a mammalian cell is cultured, examples of a medium used herein may include MEM medium containing approximately 5% to 20% fetal bovine serum [Science, Vol. 122, 501 (1952)], DMEM medium [Virology, Vol. 8, 396 (1959)], RPMI 1640 medium [The Journal of the American Medical Association, Vol. 199, 519 (1967)], and 199 medium [Proceeding of the Society for the Biological Medicine, Vol. 73, 1 (1950)]. The pH of such a medium is preferably adjusted to approximately 6 to 8. The culture is generally carried out at a temperature between approximately 30°C and 40°C for about 15 to 60 hours. Thereafter, aeration or stirring may be then carried out, as necessary.

As stated above, the polypeptide of the present invention can be generated in the cell, in the cell membrane, or out of the cell of a transformant. The polypeptide of the present invention can be separated and purified from the above culture product by the following method, for example. In order to extract the polypeptide of the present invention from the cultured cell mass or cells, a method comprising: collecting a cell mass or cells by methods known in the present technical field after the culture; suspending the cell mass or cells in a suitable buffer solution; disintegrating the cell mass or cells by ultrasonic, lysozyme treatment, and/or freezing thawing treatment; and obtaining a crude extract of protein by centrifugation or filtration, is applied as appropriate. A buffer solution may contain protein-denaturing agents such as urea or guanidine hydrochloride, surfactants such as Triton X-100™, and other agents. When the polypeptide of the present invention is secreted into the culture solution, a cell mass or cells are separated from the supernatant by the known method after completion of the culture, and the surfactant is then collected. The polypeptide of the present invention contained in the thus obtained culture supernatant or extract is purified as appropriate by the combined use of known separation and purification methods. Examples of such known separation and purification methods may include: separation and purification methods using solubility, such as salting out or solvent precipitation; separation and purification methods mainly using the difference in molecular weights, such as dialysis, ultrafiltration, gel filtration, or SDS-polyacrylamide gel electrophoresis; separation and purification methods using the difference in charge, such as ion exchange chromatography; separation and purification methods using specific affinity, such as affinity chromatography; separation and purification methods based on the difference in hydrophobicity, such as reverse phase high performance liquid chromatography; and separation and purification methods using the difference in isoelectric points, such as isoelectric focusing.

When the polypeptide of the present invention is obtained in the form of a loose body, it can be converted into a salt by known methods or methods equivalent thereto. In contrast, when the peptide is obtained in the form of a salt, it can be converted into a loose body or other salts by known methods or method equivalent thereto. In addition, a suitable protein modification enzyme is allowed to act on the polypeptide generated from a transformant before or after purification, so as to add modification as appropriate, or to eliminate a part of the polypeptide. Examples of such a protein modification enzyme may include trypsin, chymotrypsin, arginyl endopeptidase, protein kinase, and glycosidase. The presence of the thus obtained polypeptide of the present invention can be determined by enzyme immunoassay using a specific antibody, Western blotting, and the like. The antibody reacting with the polypeptide of the present invention or a salt thereof may be either a polyclonal antibody or a monoclonal antibody, as long as it is capable of recognizing the polypeptide of the present invention or a salt thereof. The antibody reacting with the polypeptide of the present invention or a salt thereof (hereinafter abbreviated as the polypeptide of the present invention, at times) can be produced by known methods for producing an antibody or antiserum using the polypeptide of the present invention as an antigen. Either the aforementioned polypeptide of the present invention or a salt thereof may be used as an antigen.

Next, the antibody of the present invention, a fragment thereof, and a hybridoma generating the above antibody will be explained.

The antibody of the present invention or a fragment thereof has an affinity for the aforementionedpolypeptide of the present invention. Either a polyclonal antibody or a monoclonal antibody can be used, as long as it has an affinity for the aforementioned polypeptide of the present invention. The antibody can be produced by the previously known method for producing an antibody or antiserum, using the polypeptide of the present invention or a salt thereof as an antigen. Moreover, a hybridoma generating the aforementioned antibody can be produced by the previously known methods. The methods for producing a monoclonal antibody and a polyclonal antibody will be described below.

### [Production of monoclonal antibody]

### (a) Production of monoclonal antibody-generating cells

The polypeptide of the present invention is administered singly or together with a carrier or diluent to a site of a warm-blooded animal, which is capable of generating an antibody due to the administration of the polypeptide. In order to enhance an ability of the above site to generate an antibody, it is also possible to administer Freund's complete adjuvant or Freund's incomplete adjuvant. The polypeptide of the present invention is generally administered once every 2 to 6 weeks, approximately 2 to 10 times in total. Examples of a warm-blooded animal used herein may include a monkey, a rabbit, a dog, a Guinea pig, a mouse, a rat, a sheep, a goat, and a chicken. Among the above described animals, a mouse and a rat are preferably used. In order to produce monoclonal antibody-generating cells, from warm-blooded animals, for example, mice immunized with antigen, individuals, in which an antibody titer has been observed, are selected, and the spleen or lymph node is collected therefrom on 2 to 5 days after the final immunization. Thereafter, cells generating antibodies contained in the spleen or lymph node are fused with the myeloma cells of homogeneous or heterogeneous animals, so as to prepare hybridomas generating monoclonal antibodies. The antibody titer in an antiserum can be measured, for example, by allowing a labeled polypeptide to react with the antiserum and measuring the activity of the labeling agent binding to the antibody. The fusion operations can be carried out by known methods, such as the method of Kohler and Milstein [Nature, 256, 495 (1975)]. Examples of a fusion promoter used herein may include polyethylene glycol (PEG) and Sendai virus. PEG is preferably used.

Examples of myeloma cells used herein may include the myeloma cells of warm-blooded animals, such as NS-1, P3U1, SP2/0, or AP-1. Among the above described myeloma cells, P3U1 is preferably used. The preferred ratio between the number of antibody-generating cells (spleen cells) used and the number of myeloma cells used is between approximately 1 : 1 and 20 : 1. PEG (preferably PEG 1000 to PEG 6000) is added thereto at a concentration between approximately 10% and 80%. The obtained mixture is incubated at a temperature between 20°C and 40°C and preferably between 30°C and 37°C, for 1 to 10 minutes, so as to efficiently carry out cell fusion. Various methods can be applied to screen a hybridoma generating a monoclonal antibody. Examples of such a method may include: a method comprising adding a hybridoma culture supernatant to a solid phase (e.g. a microplate) on which a protein antigen has been adsorbed directly or together with a carrier, and then adding an anti-immunoglobulin antibody or protein A labeled with a radioactive substance or enzyme (when cells used in cell fusion are mouse cells, an anti-mouse immunoglobulin antibody is used), so as to detect a monoclonal antibody binding to the solid phase; and a method comprising adding a hybridoma culture supernatant to a solid phase on which an anti-immunoglobulin antibody or protein A has been adsorbed, and then adding a protein labeled with a radioactive substance or enzyme, so as to detect a monoclonal antibody binding to the solid phase. Selection of a monoclonal antibody can be carried out by known methods or methods equivalent thereto. In general, such selection can be carried out using a medium used for animal cells, to which HAT (hypoxanthine, aminopterin, thymidine) is added. Any medium can be used as a medium for selection and bleeding, as long as a hybridoma can grow therein. For example, RPMI 1640 medium containing 1% to 20%, and preferably 10% to 20% fetal bovine serum, GIT medium containing 1% to 10% fetal bovine serum (Wako Pure Chemical Industries, Ltd.), or a serum-free medium used for the culture of hybridoma (SFM-101, Nissui Pharmaceutical Co., Ltd.) can be used. The culture temperature is generally between 20°C and 40°C and preferably at approximately 37°C. The culture time is generally for 5 days to 3 weeks, and preferably for 1 to 2 weeks. The culture can generally be carried out in the presence of 5% carbon dioxide. The antibody titer in a hybridoma culture supernatant can be measured in the same manner as the aforementioned measurement of an antibody titer in an antiserum.

### (b) Purification of monoclonal antibody

Separation and purification of a monoclonal antibody can be carried out by known methods, such as separation and purification methods for immunoglobulin [e.g. salting out, alcohol precipitation, isoelectric point precipitation, electrophoresis, the adsorption and desorption method using ion exchangers (e.g. DEAE), ultracentrifugation, gel filtration, a specific purification method comprising collecting only an antibody using an antigen-binding solid phase or activity absorbents such as protein A or protein G and then dissociating the binding thereof, so as to obtain an antibody].

### [Production of polyclonal antibody]

The polyclonal antibody of the present invention can be produced by known methods or methods equivalent thereto. For example, a polypeptide acting as an immunizing antigen or a complex consisting of the polypeptide and a carrier protein is produced. Thereafter, a warm-blooded animal is immunized in the same manner as in the aforementioned method for producing a monoclonal antibody. Thereafter, a product containing an antibody reacting with the polypeptide of the present invention is collected from the thus immunized animal, followed by separation and purification of the antibody, thereby producing the polyclonal antibody of the present invention. With regard to the complex consisting of an immunizing antigen and a carrier protein used for the immunization of a warm-blooded animal, the type of a carrier protein and the mixing ratio between a carrier and a hapten are not limited. Thus, any type of carrier protein may be crosslinked with a hapten at any ratio, as long as an antibody is efficiently produced with respect to the hapten with which a carrier is crosslinked for immunization. For example, bovine serum albumin, bovine thyroglobulin, hemocyanin, or the like is coupled to a hapten at a weight ratio between approximately 0.1 : 1 and 20 : 1, and preferably between approximately 1 : 1 and 5 : 1. For the coupling a hapten to a carrier, various condensing agents can be used. For example, glutaraldehyde, carbodiimide, or active ester reagents containing a maleimide active ester, a thiol group, or a dithioviridyl group are used. The condensation product is administered singly or together with a carrier or diluent to a site of a warm-blooded animal, which is capable of generating an antibody. In order to enhance an ability of the above site to generate an antibody, it is also possible to administer Freund's complete or incomplete adjuvant. The condensation product is generally administered once every 2 to 6 weeks, approximately 3 to 10 times in total. The polyclonal antibody can be collected from the blood, ascites, and the like of the warm-blooded animal immunized by the above described method, and preferably from the blood thereof. The polyclonal antibody titer in an antiserum can be measured in the same manner as in the aforementioned measurement of an antibody titer in the antiserum. Separation and purification of a polyclonal antibody can be carried out by the same immunoglobulin separation and purification method as in the aforementioned separation and purification of a monoclonal antibody.

An antibody having an affinity for the polypeptide of the present invention can be used for the qualitative analysis, assay, histological stain, and the like of the polypeptide of the present invention contained in a test solution (for example, an SIK2 protein contained in a biological sample) by immunoassay. In addition, the antibody of the present invention can be used in the analysis of CRE-suppressing activity that is judged based on the expression level of SIK2 or intracellular localization thereof. In particular, an antibody capable of distinguishing SIK1 from SIK2 can be used to individually analyze the expression level and activity of SIK1 and SIK2. Moreover, it is also possible to specifically neutralize or regulate the activity of each of SIK1 and SIK2.

Furthermore, an antibody specifically recognizing post-translation modification of SIK1 and/or SIK2, such as phosphorylation thereof, can be applied to treatments using antibodies. For these purposes, an antibody molecule itself may be used, or a F(ab')₂, Fab', or Fab fraction of the antibody molecule may be used. The determination method of the polypeptide of the present invention using the antibody of the present invention is not particularly limited. The amount of an antibody, an antigen, or an antibody-antigen complex corresponding to the amount of an antigen contained in a solution to be measured (that is, the polypeptide of the present invention) is detected by chemical or physical means. As such detection means, for example, nephelometry, the competition method, the immunometric method, and the sandwich method are preferably used. Examples of a labeling agent used for the measurement method using the labeling substance may include a radioisotope, an enzyme, a fluorescent substance, and a luminescent substance. Examples of a radioisotope used herein may include [¹²⁵I], [¹³¹I], [³H], and [¹⁴C]. As the aforementioned enzyme, those that are stable and have large specific activity are preferably used. Examples of such an enzyme may include β-galactosidase, β-glucosidase, alkaline phosphatase, peroxidase, and malate dehydrogenase. Examples of a fluorescent substance may include fluorescamine and flurescein isothiocyanate. Examples of a luminescent substance may include luminol, a luminol derivative, luciferin, and lucigenin. Moreover, a biotin-avidin system may be used in the binding of an antibody or antigen to a labeling agent. For insolubilization of an antigen or antibody, physical adsorption may be used, or a method involving chemical binding that is generally used for the insolubilization or immobilization of a protein or enzyme may also be applied. Examples of a carrier used herein may include insoluble polysaccharides such as agarose, dextran, or cellulose, synthetic resins such as polystyrene, polyacrylamide, or silicon, glass, and magnetic particles.

The polypeptide of the present invention can be assayed with good sensitivity by using the antibody of the present invention. Further, when the concentration of SIK2 in a biological sample is assayed with the antibody of the present invention, if an increase in the concentration of SIK2 were detected, it could be diagnosed that it is affected with disease with which a suppressed differentiation of fat cells or the disorder of the metabolic function of fat cells is associated (for example, diabetes, adiposis, hyperlipidemia, hypertension, arteriosclerosis, hyperuricemia, cardiovascular disease, etc.) or that it has a high risk of being affected with such disease in future. In contrast, if the concentration of SIK2 were decreased, excessive activation of CRE would be continued, and thus, it could be used to predict an abnormal growth of cells or a degree of risk of being affected with cancer associated therewith.

Furthermore, the antibody of the present invention can be used to produce an antibody column for separating and purifying SIK2 or the recombinant polypeptide of the present invention existing in fat cells or tissues, or to detect SIK2 or a recombinant polypeptide contained in each fraction during purification. Further, the antibody of the present invention canbe used to analyze the behavior of SIK2 or the polypeptide of the present invention in test cells.

Next, the pharmaceutical composition of the present invention will be described.

The pharmaceutical composition of the present invention comprises the polypeptide of the present invention or the recombinant vector of the present invention.

The pharmaceutical composition also comprises the antibody of the present invention, a fragment thereof, or an antisense nucleotide complementarily binding to the polynucleotide of the present invention.

The pharmaceutical composition comprising the polypeptide of the present invention or the recombinant vector of the present invention (the pharmaceutical composition in a first embodiment of the present invention) will be described.

SIK2 regulates the transcription of a gene that is under the control of CRE in fat cells, thereby regulating the differentiation from precursor fat cells to fat cells or the function of glucose or lipid metabolism in fat cells. Accordingly, the polypeptide of the present invention can be used as an agent for preventing or improving various diseases, with which an increase in the differentiation of fat cells or an increase in metabolic functions is associated. For example, in the case of a patient in the body of which SIK2 is decreased or defected, and thus, in which suppression of the differentiation of fat cells and the regulation of metabolic functions are not sufficiently carried out, the role of SIK2 in the patient can be complemented by the following means: (1) administration to the patient of an expression vector comprising the polynucleotide of the present invention that is under the control of a promoter capable of functioning in target cells, and allowing the polynucleotide of the present invention to express in the living body; (2) introduction of the polynucleotide of the present invention into cells that have been removed in the same manner as described above, allowing the polypeptide of the present invention to express therein, and then transplanting the cells into the patient; or (3) administration of the polypeptide of the present invention to the patient. When the polynucleotide of the present invention is used as the aforementioned preventing or improving agent, it is used singly, or it is inserted into a suitable vector such as a retrovirus vector, adenovirus vector, or adenovirus-associated virus vector, and it is then administered to patients by conventional means.

The polynucleotide of the present invention is directly administered, or it is converted into a pharmaceutical together with a physiologically acceptable carrier such as a supplement used to promote intake. The obtained pharmaceutical is then administered using a particle gun or a catheter such as a hydrogel catheter. When the polypeptide of the present invention is used as the aforementioned preventing or improving agent, it is preferable that the polypeptide be purified at a degree of preferably 90%, more preferably 95% or more, further more preferably 98% or more, and most preferably 99% or more, before use. The polypeptide of the present invention can be orally administered in the form of a tablet coated with sugar as necessary, a capsule, an elixir, or a microcapsule. It can also be aerosolized and administered in the form of an inhalant. Otherwise, it can be parenterally used in the form of an injection containing a sterile solution consisting of the present polypeptide and water or a pharmacologically acceptable solution other than water, or containing a suspension thereof.

For example, the polypeptide of the present invention is mixed with a physiologically acceptable carrier, flavor, excipient, vehicle, antiseptic, stabilizer, binder, etc. in the unit, dosage, and form required for the formulation of pharmaceuticals that is generally acceptable, so as to produce a pharmaceutical. The amount of active ingredients in such a pharmaceutical is set within a range instructed, such that an appropriate dose can be obtained. Examples of an additive that can be added to a tablet or capsule may include binders such as gelatin, corn starch, tragant, or gum Arabic, excipients such as crystalline cellulose, swelling agents such as corn starch, gelatin, or alginic acid, lubricants such as magnesium stearate, sweetening agents such as sucrose, lactose, or saccharine, and flavors such as peppermint, Gaultheria adenothrix oil, or cherry. When the form of a pharmaceutical is a capsule, a liquid carrier such as fat and oil can also be added to the aforementioned materials. An injection can be prepared by dissolving, suspending, or emulsifying the polypeptide of the present invention in a sterilized aqueous or oily solution that is generally used for injections. Examples of an aqueous solution used for injections may be a saline and an isotonic solution containing glucose or other assistant agents. Such an aqueous solution may also be used with the combination of suitable solubilizing agents such as alcohol (e.g. ethanol), polyalcohol (e.g. propylene glycol, polyethylene glycol), nonionic surfactants [e.g. Polysorbate 80, HCO-50 (polyoxyethylene (50 mol) adduct of hydrogenated castor oil)], and the like. Examples of an oily solution may include sesame oil and soybean oil. Such an oily solution may also be used with the combination of solubilizing agents such as benzyl benzoate or benzyl alcohol. In addition, buffer solutions (e.g. phosphate buffer solution, sodium acetate buffer solution, etc.), soothing agents (e.g. benzalkonium chloride, procaine hydrochloride, etc.), stabilizers (e.g. human serum albumin, polyethylene glycol, etc.), preservatives (e.g. benzyl alcohol, phenol, etc.), antioxidants, and other agents may be mixed therewith. The prepared injection is generally filled in a suitable ampule.

A vector into which the polynucleotide of the present invention is inserted is also converted into a pharmaceutical in the same manner as described above, and in general, it is parenterally used. Since the thus obtained pharmaceutical is safe and has low toxicity, it can be administered, for example, to warm-blooded animals (e.g. a human, a rat, a mouse, a Guinea pig, a rabbit, a chicken, a sheep, a swine, a bovine, a horse, a cat, a dog, a monkey, a chimpanzee, etc.). The dosage of the polypeptide of the present invention depends on a target disease, an administration target, an administration route, and the like. When the polypeptide of the present invention is orally administered as anti-obesity agent for example, in general, it is administered to an adult (with a body weight of 60 kg) at a dosage preferably between approximately 0.1 mg and 100 mg, more preferably between approximately 1.0 mg and 50 mg, and most preferably between approximately 1.0 mg and 20 mg per day. When the polypeptide of the present invention is parenterally administered, the dosage of the above polypeptide for an administration depends on an administration target, a target disease, and the like. When the polypeptide of the present invention is administered as anti-obesity agent in the form of an injection to an adult (with a body weight of 60 kg) for example, it is convenient to administer the above polypeptide at a dosage preferably between approximately 0.01 mg and 30 mg, more preferably between approximately 0.1 mg and 20 mg, and most preferably between approximately 0.1 mg and 10 mg per day. In the case of administering the above polypeptide to other animals also, the polypeptide can be administered at an amount that is relative to a body weight of 60 kg.

The aforementioned pharmaceutical composition may contain a substance having an action to induce fat differentiation. Examples of such a substance having an action to induce fat differentiation may include dexamethasone, insulin, cAMP, and a thiazolidine derivative. It is considered that such a substance having an action to induce fat differentiation promotes the differentiation from precursor fat cells to fat cells, thereby promoting the generation of SIK2.

Next, the pharmaceutical composition comprising an antisense nucleotide complementarily binding to the polynucleotide of the present invention (the pharmaceutical composition in a second embodiment of the present invention) will be described.

An antisense nucleotide that complementarily binds to the polynucleotide of the present invention and thereby suppresses the expression of the polynucleotide can suppress the generation of the polynucleotide of the present invention in a living body. Thus, the antisense nucleotide suppresses the expression of SIK2, and it can be used as an agent for preventing or treating disease, with which the excessive expression of SIK2 is associated (for example, diabetes, adiposis, hyperlipidemia, hypertension, arteriosclerosis, hyperuricemia, cardiovascular disease, etc.). When the antisense nucleotide is used as such a preventive or therapeutic agent, it can be used in the same manner as those for various preventive or therapeutic agents containing the aforementioned polynucleotide of the present invention. For example, when the antisense nucleotide is used, it is used singly, or it is inserted into a suitable vector such as a retro virus vector, adenovirus vector, adenovirus-associated vector, and it is then administered by conventional means. The antisense nucleotide is directly administered, or it is converted into a pharmaceutical together with a physiologically acceptable carrier such as a supplement, so as to promote intake. The obtained pharmaceutical is then administered using a particle gun or a catheter such as a hydrogel catheter. Otherwise, it is aerosolized, and it can be administered into the trachea in the form of an inhalant. The dosage of the antisense nucleotide depends on a target disease, an administration target, an administration route, and the like. When the antisense nucleotide of the present invention is locally administered into the trachea in the form of an inhalant for example, the antisense nucleotide is generally administered to an adult (with a body weight of 60 kg) at a dosage between approximately 0.1 mg and 100 mg per day.

Any antisense nucleotide may be used as an antisense nucleotide complementarily binding to the polynucleotide of the present invention, as long as it has a nucleotide sequence that is complementary to or substantially complementary to the nucleotide sequence of the polynucleotide of the present invention and also has an action to suppress the expression of the above polynucleotide. Among others, antisense DNA is preferable. An example of a nucleotide sequence substantially complementary to the polynucleotide of the present invention may be a nucleotide sequence having homology of preferably approximately 70% or more, more preferably approximately 80% or more, further more preferably approximately 90% or more, and most preferably approximately 95% or more to the entire nucleotide sequence that is complementary to the polynucleotide of the present invention (that is, a complementary strand of the polynucleotide of the present invention).

The antisense nucleotide can also be used as a diagnostic oligonucleotide probe for examining the presence of the polynucleotide of the present invention in tissues or cells, or the state of expression thereof.

Next, the pharmaceutical composition comprising the antibody of the present invention or a fragment thereof (the pharmaceutical composition in a third embodiment of the present invention) will be described.

The antibody of the present invention having an action to neutralize the activity of SIK2 can be used as a pharmaceutical (a preventive or therapeutic agent) for disease, with which the excessive expression of SIK2 is associated (for example, diabetes, adiposis, hyperlipidemia, hypertension, arteriosclerosis, hyperuricemia, a cardiovascular disease, etc.). The preventive or therapeutic agent for the aforementioned diseases that comprises the antibody of the present invention is directly used as a liquid pharmaceutical. Or, it is converted into a pharmaceutical composition having a suitable dosage form, and it is then orally or parenterally administered to warm-blooded animals (e.g. human, rat, rabbit, sheep, swine, bovine, cat, dog, monkey, etc.). The dosage depends on an administration target, a target disease, symptoms, an administration route, and the like. When the antibody of the present invention is used to treat or prevent the diabetes of an adult, it is convenient to administer by injection at a dosage preferably between approximately 0.01 and 20 mg/kg body weight, more preferably between approximately 0.1 and 10 mg/kg body weight, and most preferably between approximately 0.1 and 5 mg/kg body weight, preferably 1 to 5 times per day, and more preferably 1 to 3 times per day. In the case of other parenteral and oral administrations also, the antibody of the present invention can be administered at a dosage equivalent thereto.

When the symptom is serious, an increased dosage may be administered depending thereon. The antibody of the present invention can be administered directly or in the form of an appropriate pharmaceutical composition. The pharmaceutical composition used in the aforementioned administrations contains the antibody of the present invention and a pharmacologically acceptable carrier, diluent, or excipient. Such a composition can be provided in a dosage form that is suitable for oral or parenteral administration. That is to say, when it is a composition that is to be administered via an oral route for example, the composition may have solid or liquid dosage forms. Specific examples of such a solid or liquid dosage form may include tablets (including a tablet coated with sugar and a film coating tablet), pills, granules, powders, capsules (including a soft capsule), syrups, emulsions, and suspensions. Such a composition is produced by known methods, and it contains a carrier, diluent, or excipient that are commonly used in the pharmaceutical field. Examples of a carrier and an excipient used for tablets may include lactose, starch, sucrose, and magnesium stearate. Examples of a composition for use inparenteral administration may include an injection and a suppository. Examples of an injection may be an intravenous injection, a subcutaneous injection, an intradermal injection, an intramuscular injection, and a drip-feed solution. Such an injection is prepared by known methods, for example, by dissolving, suspending, or emulsifying the aforementioned antibody or a salt thereof in a sterilized aqueous or oily solution commonly used for injections. Examples of an aqueous solution used for injections may be a saline and an isotonic solution containing glucose or other assistant agents. Such an aqueous solution may also be used with the combination of suitable solubilizing agents such as alcohol (e.g. ethanol), polyalcohol (e.g. propylene glycol, polyethylene glycol), nonionic surfactants [e.g. Polysorbate 80, HCO-50 (polyoxyethylene (50 mol) adduct of hydrogenated castor oil)], and the like. Examples of an oily solution may include sesame oil and soybean oil. Such an oily solution may also be used with the combination of solubilizing agents such as benzyl benzoate or benzyl alcohol. The prepared injection is generally filled in a suitable ampule. The suppository used for rectal administration is prepared by mixing the aforementioned antibody or a salt thereof into a common substrate used for suppositories. It is convenient that the aforementioned pharmaceutical composition for use in oral or parenteral administration be prepared in a dosage form that is suitable for the dosage of active ingredients. Examples of such a dosage form may include tablets, pills, capsules, injections (ampules), and suppositories. The aforementioned antibody is generally contained in each dosage form at a dosage between 5 and 500 mg. In particularly, in the case of an injection, the antibody is preferably contained at a dosage between 5 and 100 mg. In other dosage forms, the antibody is preferably contained between 10 and 250 mg. In addition, each of the aforementioned compositions may contain other active ingredients, unless unfavorable interaction is generated as a result of the mixing of other active ingredients with the aforementioned antibody.

Next, the composition for use in gene diagnosis of the present invention will be described.

The composition for use in gene diagnosis of the present invention comprises the polynucleotide of the present invention.

When the polynucleotide of the present invention is used as a probe for example, it can detect the abnormality (gene abnormality) of DNA or mRNA encoding SIK2 in warm-blooded animals (e.g. human, rat, mouse, Guinea pig, rabbit, chicken, sheep, swine, bovine, horse, cat, dog, monkey, chimpanzee, etc.). Thus, it is useful as an agent for gene diagnosis, which diagnoses the injury, mutation, or decreased expression of the DNA or mRNA, the increase or excessive expression thereof, and others. The aforementioned gene diagnosis using the polynucleotide of the present invention can be carried out by the known methods such as Northern hybridization or the PCR-SSCP method (Genomics, Vol. 5, pp. 874-879 (1989), Proceedings of the National Academy of Sciences of the United States of America, Vol. 86, pp. 2766-2770 (1989)). For example, when the excessive expression of the above described DNA or mRNA is detected by Northern hybridization, or when a mutation of the DNA was detected by the PCR-SSCP method, it can be diagnosed that it is affected with disease with which a suppressed differentiation of fat cells or the disorder of the metabolic function of fat cells is associated (for example, diabetes, adiposis, hyperlipidemia, hypertension, arteriosclerosis, hyperuricemia, cardiovascular disease, etc.) or that it has a high risk of being affected with such disease in future.

Next, the method of the present invention for preventing or improving a disease or physiological conditions will be described.

The method of the present invention for preventing or improving a disease or physiological conditions prevents or improves disease or physiological conditions, which are developed with a decrease in the expression of salt-inducible kinase 2, or disease or physiological conditions, which are developed with an increase in the expression of salt-inducible kinase 2.

The method for preventing or improving a disease or physiological conditions developed with a decrease in the expression of salt-inducible kinase 2 comprises administration of the pharmaceutical composition in the first embodiment of the present invention.

In addition, the method for preventing or improving a disease or physiological conditions developed with an increase in the expression of salt-inducible kinase 2 comprises administration of the pharmaceutical composition in the second or third embodiment of the present invention.

SIK2 regulates the transcription of a gene that is under the control of CRE in fat cells, thereby regulating the differentiation from precursor fat cells to fat cells or the function of glucose or lipid metabolism in fat cells. Accordingly, the polypeptide or polynucleotide of the present invention is used to prevent or improve various diseases, with which an increase in the differentiation of fat cells or an increase in metabolic functions is associated. For example, in the case of a patient in the body of which SIK2 is decreased or defected, and thus, in which suppression of the differentiation of fat cells and the regulation of metabolic functions are not sufficiently carried out, the pharmaceutical composition in the first embodiment of the present invention is administered to the patient, so as to prevent or improve disease or physiological conditions, such as the disorder of glucose metabolism, the disorder of lipid metabolism, or cranial nerve injury.

In addition, the pharmaceutical composition according to the second or third embodiment of the present invention is administered to the patient, so as to prevent or improve disease or physiological conditions developed with an increase in the expression of SIK2.

Examples of such disease or physiological conditions may include the disorder of glucose metabolism, the disorder of lipid metabolism, and cranial nerve injury.

Next, the screening method of the present invention will be described.

The screening method in a first embodiment of the present invention is characterized in that it comprises the steps of: allowing an analyte comprising the polypeptide of the present invention to come into contact with a test compound; and detecting an activity of promoting or inhibiting the activity of the polypeptide of the present invention. According to the screening method of the present invention, a compound capable of promoting or inhibiting the activity of the polypeptide of the present invention can be screened.

The screening method in the first embodiment of the present invention may comprise steps of allowing the polypeptide of the present invention to come into contact with a test compound, and detecting the binding of the above described test compound with the above described polypeptide, thereby identifying a compound specifically binding to the above described polypeptide.

In addition, the screening method in the first embodiment of the present invention may comprise steps of allowing the polypeptide of the present invention to come into contact with a test compound under conditions where the above described polypeptide exhibits its activity, evaluating the activity of the above described polypeptide in the presence of the above described test compound, comparing the thus evaluated activity with the activity of the above described polypeptide in the absence of the above described test compound, and identifying the ability of the above described test compound to regulate the activity of the above described polypeptide based on the comparison results.

The screening method in a second embodiment of the present invention is characterized in that it comprises steps of: allowing an expression vector comprising the polynucleotide of the present invention and a reporter gene that is under the control of a cAMP responsive element to come into contact with a test compound; and detecting an activity of promoting or inhibiting the activity of the polypeptide of the present invention.

In addition, the screening method in the second embodiment of the present invention may comprise steps of allowing a target polynucleotide analyte containing the polynucleotide of the present invention to come into contact with a test compound under conditions that are suitable for the expression of the above described target polynucleotide, detecting a change in the expression of the above described target nucleotide, and comparing the expression of the above described target polynucleotide in the absence of the above described test compound and in the presence of various amounts of the above described test compounds.

SIK2, for example, negatively regulates CRE activity in fat cells, so as to regulate the differentiation of fat cells and the functions thereof. Since the CRE activity-regulating activity of a kinase domain correlates with the protein-phosphorylating activity, a compound inhibiting the protein-phosphorylating activity of the polypeptide of the present invention also inhibits the action to regulate CRE activity (CRE-inhibiting effects) at the same time, and promotes the differentiation from precursor fat cells to fat cells and/or the metabolic functions in fat cells. Thus, such a compound is useful as an agent for preventing or improving a disease such as abnormal glucose tolerance, diabetes, adiposis, or hyperlipidemia.

On the other hand, a compound promoting the protein-phosphorylating activity of the polypeptide of the present invention is likely to inhibit cell survival activity via CRE activation, so as to cause cell death. Thus, it is expected that such a compound will be applied to treatment of cancers. In addition, the primary structure of the kinase domain of SIK1 is extremely similar to that of SIK2, it is predicted that a compound inhibiting or promoting the activity of the polypeptide of the present invention also has effects on the functions of SIK1. In such a case, the aforementioned compound efficiently changes tissues or organs on which it acts, depending on the difference in the expression level of SIK1 and SIK2 in such tissues or organs. For example, SIK1 is abnormally induced in the hippocampus in the brain when convulsion is induced. In such a state, in the presence of SIK1, a compound inhibiting or promoting the activity of the polypeptide of the present invention specifically acts to regulate CRE activity associated with cell survival. Thus, it is expected that the compound is useful for preventing brain damage and the like. Focusing on the hippocampus, the compound has the same above CRE activity-regulating effects. Thus, it is expected that it will have effects of improving Alzheimer's disease and the like.

Accordingly, an agent for inhibiting or promoting the activity of the SIK2 kinase domain to regulate CRE activity can be screened by detecting the effects of a test compound on the protein-phosphorylating activity of the polypeptide of the present invention. Otherwise, such an agent for inhibiting or promoting the activity of the SIK2 kinase domain to regulate CRE activity can also be screened, using, as an indicator, the expression of a reporter gene that is under the control of CRE in cells, into which such a reporter gene has been introduced.

The CRE-regulating activity when each domain of SIK1 and SIK2 is phosphorylated is summarized in the following Table 1.

**Table 1**

| | Domain 1 | Domain 2 | Domain 3 | SIK1 | SIK2 |
|---|---|---|---|---|---|
| Phosphorylation | Yes | Yes | Yes | Slightly increased | Slightly increased |
| Phosphorylation | Yes | Yes | No | Slightly suppressed | Slightly suppressed |
| Phosphorylation | Yes | No | No | Significant ly suppressed | Significant ly suppressed |
| Phosphorylation | No | No | No | Not changed | Increased |
| Phosphorylation | No | Yes | Yes | Increased | Increased |
| Phosphorylation | No | No | Yes | Increased | Increased |
| Phosphorylation | No | Yes | No | Not changed | Increased |

The screening method of the present invention uses the polypeptide of the present invention. In addition, the screening method of the present invention may use cells having an ability to generate the polypeptide of the present invention (preferably, a transformant transformed with a polynucleotide encoding the polypeptide of the present invention (for example, cells such as yeast cells, insect cells, or mammalian cells)). Such a transformant is preferably transformed with a polynucleotide containing the polynucleotide encoding the polypeptide of the present invention and a reporter gene that is under the control of CRE.

The screening methods in third and fourth embodiments of the present invention are screening methods using the auto-phosphorylating ability of a protein as an indicator. The screening method in the third embodiment of the present invention is characterized in that it comprises the step of phosphorylating a protein in the presence of a test compound under conditions suitable for auto-phosphorylation; an antibody-binding step of allowing an antibody recognizing an auto-phosphorylated portion of the above described protein that is in a state where it has been phosphorylated or has not been phosphorylated, to react with the above described protein; and a measuring step of detecting the reaction of the above described protein with the above described antibody.

The screening method in the fourth embodiment of the present invention is characterized in that it comprises the step of culturing cells having an ability to generate a protein under conditions suitable for the expression of the protein; a step of phosphorylating the protein generated from the above described cells in the presence of a test compound under conditions for auto-phosphorylation; an antibody-binding step of allowing an antibody recognizing an auto-phosphorylated portion of the above described protein that is in a state where it has been phosphorylated or has not been phosphorylated, to react with the above described protein; and a measuring step of detecting the reaction of the above described protein with the above described antibody.

The screening method in a fifth embodiment of the present invention is characterized in that it comprises the phosphorylating step of allowing salt-inducible kinase to come into contact with a substrate that is to be phosphorylated with the salt-inducible kinase in the presence of a test compound, so as to phosphorylate the above described substrate; an antibody-binding step of allowing an antibody recognizing a substrate that is in a state where it has been phosphorylated or has not been phosphorylated, to react with the above described substrate; and a measuring step of detecting the reaction of the above described substrate with the above described antibody.

The screening method of the present invention will be specifically described below.

The screening method in the first embodiment of the present invention, which uses phosphorylating activity as an indicator, will be described.

The polypeptide of the present invention is allowed to react with a test compound and [³²P]-ATP [and, as necessary, also with a substrate protein or peptide that is phosphorylated with SIK2 (for example, GST-Syntide 2 (Lin et al., Molecular Endocrinology (2001) 15, 1264-1276), Syntide 2, histone deacetylase, HDAC5, histone acetylase, p300, etc.)] in the presence of a divalent cation. The reaction solution is subjected to gel electrophoresis. Thereafter, the ³²P intake by the protein on the gel is detected with an X-ray film, so as to measure the phosphorylating activity of the protein (that is, the auto-phosphorylating activity of the protein and/or the activity thereof of phosphorylating other proteins). When the ³²P intake disappears, or when the intake level becomes smaller in the presence of a test compound than in the absence thereof, it can be concluded that the used test compound has an effect of inhibiting the phosphorylating activity of the protein, that is, an effect of inhibiting the activity of regulating CRE activity. In contrast, when the ³²P intake level becomes greater than in the presence of a test compound than in the absence thereof, it can be concluded that the used test compound has an effect of promoting the phosphorylating activity of the protein, that is, an effect of promoting the activity of regulating CRE activity. Moreover, other than an SDS-PAGE X-ray film, materials capable of trapping a peptide or protein (for example, a DE81 cellulose film, a PVDF film, etc.) can also be used, and ³²P binding to such materials may be measured. Furthermore, a change in the total charge obtained by binding of a phosphate group to a peptide or protein, a change in the molecular weight, and a change in the antigen-antibody reactivity have already been commonly used for the measurement of the phosphorylating activity of protein kinase. Thus, other than a method using a radioisotope, these can also be used for the detection of the phosphorylating activity of an SIK2 kinase domain.

Next, the screening method in the second embodiment of the present invention, which uses an expression vector comprising the polynucleotide of the present invention and a CRE-reporter gene, will be described.

The aforementioned expression vector containing the polynucleotide encoding the polypeptide of the present invention and expression vector containing a reporter gene (for example, luciferase, chloramphenicol acetyl transferase, β-galactosidase, alkaline phosphatase, peroxidase, etc.) that is under the control of CRE are introduced into host cells suitable for these vectors. Such expression vectors can be produced by methods known in the present technical field.

Preferred examples of the aforementioned host cells may include precursor fat cells such as 3T3-L1; COS-7 cells, COS-1 cells, CHO cells, and Y1 cells, but examples are not limited thereto. Moreover, instead of CRE, promoters for cAMP-dependently activating the transcription of a gene [for example, CYP11A (Lin et al., as described above), c/EBP β (Reusch et al., Molecular Cellular Biology (2000) 20, 1008-1020), etc.] may also be used. A method of introducing a gene into host cells is not particularly limited. Examples of such a method used herein may include lipofection, the calcium phosphate method, the electric pulse method, and microinjection. Cells are cultured for a certain period of time, and the cultured cells are then stimulated for several hours in an environment containing a reagent for activating CRE in the presence of a test compound (for example, a solution containing 1 µM dexamethasone, 1 mM dibutyryl-cAMP, and 1 µg/ml insulin, etc.) and the test compound. Thereafter, the cells are recovered, and the expression level of a reporter gene is then measured by known methods. If the expression level of the reporter gene increases, it can be concluded that the used test compound has an effect of inhibiting the activity of SIK2 to regulate CRE activity. In contrast, if the expression level of the reporter gene decreases, it can be concluded that the used test compound has an effect of promoting the activity of SIK2 to regulate CRE activity.

In order to analyze the effects of cAMP, it is possible that a forskolin treatment be carried out or that a PKA expression vector be introduced together with the aforementioned expression vector.

Examples of a test compound used in the aforementioned screening method of the present invention may include a peptide, a protein, a nonpeptide compound, a synthetic compound, a fermented product, a cell extract, a plant extract, and an animal tissue extract. These compounds may be either novel compounds or known compounds.

Next, the screening method in the third embodiment of the present invention will be described.

The screening method in the third embodiment of the present invention is characterized in that it comprises the step of phosphorylating a protein in the presence of a test compound under conditions suitable for auto-phosphorylation; an antibody-binding step of allowing an antibody recognizing an auto-phosphorylated portion of the above described protein that is in a state where it has been phosphorylated or has not been phosphorylated, to react with the above described protein; and a measuring step of detecting the reaction of the above described protein with the above described antibody.

An example of the aforementioned protein having an auto-phosphorylation ability may be salt-inducible kinase. Moreover, in the screening method in the third embodiment of the present invention, the polypeptide of the present invention can be used. The protein having an auto-phosphorylation ability that is used in the screening method in the third embodiment of the present invention is hereinafter simply referred to as a "protein or the like" at times.

In the screening method in the third embodiment of the present invention, a protein or the like is phosphorylated in the presence of a test compound. Examples of a test compound used in the screening method of the present invention may include a peptide, a protein, a nonpeptide compound, a synthetic compound, a fermented product, a cell extract, a plant extract, and an animal tissue extract. These compounds may be either novel compounds or known compounds.

Phosphorylation of the aforementioned protein or the like can be carried out in a buffer capable of inducing the phosphorylation of the aforementioned protein. Examples of such a buffer may include 50 mM Tris-HCl (pH 7.4), 1 mM dTT (dithiothreitol), 5 mM MnCl₂, and 5 mM ATP. The protein or the like is preferably phosphorylated by leaving at a temperature between 4°C and 40°C for about 30 seconds to 90 minutes. Phosphorylation of the protein or the like may be carried out by dissolving the above protein or the like in the above buffer. However, it is also possible to phosphorylate the above protein or the like that is immobilized on a carrier. For example, a protein is immobilized on a solid phase (for example, insoluble polysaccharides such as agarose, dextran, or cellulose; synthetic resins such as polystylene, polyacrylamide, or silicon; glass, etc.), directly or via an antibody or the like.

Further, cells generating salt-inducible kinase acting as a protein having an auto-phosphorylation ability, or the host cells of the present invention containing a recombinant vector expressing salt-inducible kinase, are cultured under conditions suitable for expression of a polypeptide (a protein having an auto-phosphorylation ability). Thus, a polypeptide obtained from the culture product may also be used. In this case, since salt-inducible kinase is induced by a compound such as dexamethasone, such a compound may be added into a medium for cell culture.

Subsequently, an antibody that recognizes an auto-phosphorylated portion of a protein in a state where it has been phosphorylated and that does not react with a protein in a state where it has not been auto-phosphorylated, or an antibody that recognizes the above protein in a state where it has not been phosphorylated and that does not react with the above protein in a state where it has been auto-phosphorylated, is allowed to react with the above protein. The reaction is carried out by leaving or stirring at a temperature between 4°C and 40°C for about 1 to 24 hours. The above antibody may be either a polyclonal antibody or a monoclonal antibody. The above antibody can be produced by producing a synthetic peptide having a sequence corresponding to the auto-phosphorylated portion and then using, as an antigen, the synthetic peptide or the synthetic peptide that has been phosphorylated, according to the previously known method for producing antibodies. The production method of such an antibody is the same as described in the production method of the antibody of the present invention.

Subsequently, the reaction between the phosphorylated protein or the like and an antibody recognizing an auto-phosphorylated portion of the protein that is in a state where it has been phosphorylated or has not been phosphorylated is measured. An example of a method for measuring the binding of a protein with an antibody may be a method using an antigen-antibody reaction that has been known to persons skilled in the art. For example, it is a method comprising labeling an antibody (hereinafter referred to as a secondary antibody at times) reacting with an antibody binding to the protein and recognizing an auto-phosphorylated portion thereof (hereinafter referred to as a primary antibody at time) and using the secondary antibody. Such labeling can be carried out by methods known to persons skilled in the art, using enzyme, a fluorescent substance, metal, pigment, etc.

For example, the reaction between a protein and an antibody can be measured by the method described below. A primary antibody is allowed to bind to a protein or the like that has been immobilized, and the resultant product is washed. Thereafter, a secondary antibody labeled with a labeling agent is allowed to react therewith, and the resultant product is then washed. Thereafter, the secondary antibody that has been immobilized via the primary antibody is detected. The labeled secondary antibody is detected, for example, by labeling with an enzyme such as α-D-galactosidase, peroxidase, alkaline phosphatase, or glucose oxidase, and developing or comparing color, using the activity of the used enzyme. However, the secondary antibody is not necessarily used, but it is also possible to directly measure the reaction of the primary antibody by labeling it. As stated above, the binding of a protein with an antibody is measured, and then the results are compared with those in a case where no test compounds exist.

In a measurement system wherein an antibody recognizing an auto-phosphorylated portion of a protein in a state where it has been phosphorylated is used, it is found that an auto-phosphorylating activity is promoted when the binding of an antibody is increased in the presence of a test compound, and that such an auto-phosphorylating activity is inhibited when such a binding is decreased. In a measurement system wherein an antibody recognizing an auto-phosphorylated portion of a protein in a state where it has not been phosphorylated is used, it is found that an auto-phosphorylating activity is inhibited when the binding of an antibody is increased in the presence of a test compound, and that such an auto-phosphorylating activity is promoted when such a binding is decreased.

Next, the screening method in the fourth embodiment of the present invention will be described.

The screening method in the fourth embodiment of the present invention is characterized in that it comprises the step of culturing cells having an ability to generate a protein having an auto-phosphorylation ability under conditions that are suitable for the expression of the above described protein; a step of phosphorylating the protein generated from the above described cells in the presence of a test compound under conditions that are suitable for auto-phosphorylation; an antibody-binding step of allowing an antibody recognizing an auto-phosphorylated portion of the above described protein that is in a state where it has been phosphorylated or has not been phosphorylated, to react with the above described protein; and a measuring step of detecting the reaction of the above described protein with the above described antibody.

The protein having an auto-phosphorylation ability, the antibody recognizing an auto-phosphorylated portion of the protein in a state where it has been phosphorylated or has not been phosphorylated, and the method for measuring a complex consisting of the protein and the antibody, are all the same as those in the screening method in the third embodiment of the present invention.

Examples of cells having an ability to generate a protein having an auto-phosphorylation ability may include precursor fat cells and the host cells of the present invention. Such cells are cultured until they grow and become confluent. Thereafter, the expression of a protein is induced under conditions suitable for the expression of a protein or the like. After completion of the induction, a test compound is added thereto, and the mixture is further cultured. Thereafter, the cells are immobilized with an immobilizing solution containing paraformaldehyde or the like, and the immobilized cells are preferably allowed to react with the above antibody.

When precursor fat cells are used, it is preferable to culture the cells in the presence of a substance having an action to induce fat differentiation, when the expression of a protein is induced. Examples of such a substance having an action to induce fat differentiation may include dexamethasone, insulin, cAMP, and a thiazolidine derivative. Such a substance having an action to induce fat differentiation promotes the differentiation from precursor fat cells to fat cells. Accordingly, when the aforementioned cells are cultured, such a substance is preferably added to a medium.

As described above, the reaction between the protein or the like and the antibody is measured, and the obtained results are then compared with those in a case where no test compounds exist.

In a measurement system wherein an antibody recognizing an auto-phosphorylated portion of a protein in a state where it has been phosphorylated is used, it is found thatan auto-phosphorylating activity is promoted when the binding of an antibody is increased in the presence of a test compound, and that such an auto-phosphorylating activity is inhibited when such a binding is decreased. In a measurement system wherein an antibody recognizing an auto-phosphorylated portion of a protein in a state where it has not been phosphorylated is used, it is found that an auto-phosphorylating activity is inhibited when the binding of an antibody is increased in the presence of a test compound, and that such an auto-phosphorylating activity is promoted when such a binding is decreased.

Next, the screening method in the fifth embodiment of the present invention will be described.

The screening method in the fifth embodiment of the present invention is characterized in that it comprises the phosphorylating step of allowing salt-inducible kinase to come into contact with a substrate that is to be phosphorylated with the salt-inducible kinase in the presence of a test compound, so as to phosphorylate the above described substrate; an antibody-binding step of allowing an antibody recognizing a substrate that is in a state where it has been phosphorylated or has not been phosphorylated, to react with the above described substrate; and a measuring step of detecting the reaction of the above described substrate with the above described antibody.

Examples of a substrate used in the above screening method may include p300, HDAC5, GST-Syntide 2 (Lin et al., as described above), Syntide 2, histone deacetylase, histone acetylase, and IRS-1. An antibody recognizing such a substrate that is in a state where it has been phosphorylated or has not been phosphorylated can be produced by common methods.

In addition, in the screening method in the fifth embodiment, salt-inducible kinase may be used as a substrate. In this case, screening is carried out using the auto-phosphorylating activity of the salt-inducible kinase as an indicator. Moreover, the antibody used in this case is the same as that used in the aforementioned screening method in the third embodiment.

The reaction between the substrate and the antibody is measured as described above, and the results are compared with those in a case where no test compounds exist.

In a measurement system wherein an antibody recognizing a substrate that has been phosphorylated is used, it is found that an auto-phosphorylating activity is promoted when the binding of an antibody is increased in the presence of a test compound, and that such an auto-phosphorylating activity is inhibited when such a binding is decreased. In a measurement system wherein an antibody recognizing a substrate that has not been phosphorylated is used, it is found that an auto-phosphorylating activity is inhibited when the binding of an antibody is increased in the presence of a test compound, and that such an auto-phosphorylating activity is promoted when such a binding is decreased.

The above described screening method of the present invention is used to search for a compound preventing or treating diabetes, adiposis, hyperlipidemia, hypertension, arteriosclerosis, hyperuricemia, and a cardiovascular disease. That is to say, the compound capable of promoting or inhibiting the activity of the polypeptide of the present invention, which is identified by the screening method of the present invention, is used as a pharmaceutical composition.

In other words, the compound promoting the activity of the polypeptide of the present invention is used as in the case of the aforementioned pharmaceutical composition in the first embodiment of the present invention.

On the other hand, the compound inhibiting the activity of the polypeptide of the present invention is used as in the case of the aforementioned pharmaceutical compositions in the second and third embodiments of the present invention. That is to say, such compounds are used as pharmaceutical compositions preventing or treating diabetes, adiposis, hyperlipidemia, hypertension, arteriosclerosis, hyperuricemia, and a cardiovascular disease.

Next, the screening kit of the present invention will be described.

The screening kit in a first embodiment of the present invention is characterized in that it comprises the polypeptide of the present invention. It is used to screen a compound capable of promoting or inhibiting the activity of the polypeptide of the present invention.

The screening kit according to the first embodiment of the present invention preferably contains the polypeptide of the present invention or a phosphate group donor that phosphorylates other substrates. In addition, it may also contain a substrate polypeptide that is phosphorylated by the polypeptide of the present invention.

The screening kit in the first embodiment of the present invention uses a phosphorylating activity as an indicator.

A kit used for the screening method of the present invention using a phosphorylating activity as an indicator contains, as a constituent, a polypeptide comprising an amino acid sequence having the aforementioned kinase activity of the present invention or a polypeptide substantially equivalent thereto. Preferably, the screening kit further contains a phosphate group donor (for example, [³²P]-labeled or -non-labeled ATP, etc.) with which the polypeptide of the present invention phosphorylates itself or other substrates. Moreover, the screening kit preferably contains, as a constituent, a substrate protein or peptide capable of being phosphorylated by the polypeptide of the present invention, as necessary. Examples of such a substrate protein or peptide may include GST-Syntide 2 (Lin et al., as described above), Syntide 2, histone deacetylase, HDAC5, histone acetylase, and p300. Furthermore, the screening kit may also contain other reagents or devices that are necessary for detection of a phosphorylating activity.

The screening kit in a second embodiment of the present invention is characterized in that it comprises host cells transformed with the expression vector of the present invention. It is used to screen for a compound capable of promoting or inhibiting the activity of the polypeptide of the present invention.

The screening kit in the second embodiment of the present invention preferably contains a medium used for the above host cells and a reagent for activating a cAMP responsive element.

The screening kit in the second embodiment of the present invention uses the expression of a reporter gene that is under the control of CRE as an indicator.

The screening kit in the second embodiment of the present invention comprises a polypeptide comprising an amino acid sequence having the kinase activity of the present invention or host cells [preferably, a transformant (for example, cells such as yeast cells, insect cells, or mammalian cells) into which a polynucleotide encoding the polypeptide of the present invention and CRE-reporter are co-introduced] transformed with an expression vector having an ability to generate a polypeptide substantially equivalent thereto and containing a reporter gene (hereinafter abbreviated as CRE-reporter at times) that is under the control of CRE or an alternative cAMP-dependent cis element [for example, CYP11A (Lin et al., as described above), c/EBPβ (Reusch et al., as described above), etc.]. Examples of cells endogenously generating the polypeptide of the present invention may include fat cells derived from a human or other warm-blooded animals. Examples of an expression vector carrying a polynucleotide encoding the polypeptide of the present invention and a CRE-reporter used herein are the same as described above. The method for introducing the vector is also the same method as described above. In addition, the above screening kit may further contain, as constituents, a medium for the above cells, a reagent for activating CRE (for example, a solution containing 1 µM dexamethasone, 1 mM dibutyryl-cAMP, and 1 µg/ml insulin, etc.), and a reagent forendogenouslyincreasing cAMP (for example, forskolin, etc.).

The screening kit in a third embodiment of the present invention is characterized in that it comprises a protein having an auto-phosphorylation ability and an antibody recognizing an auto-phosphorylated portion of the above described protein that is in a state where it has been phosphorylated or has not been phosphorylated.

An example of the aforementioned protein having an auto-phosphorylation ability may be salt-inducible kinase.

As an antibody recognizing an auto-phosphorylated portion of the above protein that is in a state where it has been phosphorylated or has not been phosphorylated, those used in the aforementioned screening method of the present invention are used.

The screening kit in a fourth embodiment of the present invention is characterized in that it comprises cells having an ability to generate a protein having an auto-phosphorylation ability and an antibody recognizing an auto-phosphorylated portion of the above described protein that is in a state where it has been phosphorylated or has not been phosphorylated.

An example of the above described protein having an auto-phosphorylation ability may be salt-inducible kinase.

As an antibody recognizing an auto-phosphorylated portion of the above protein that is in a state where it has been phosphorylated or has not been phosphorylated, those used in the aforementioned screening method of the present invention are used.

A compound obtained using the screening method or screening kit of the present invention, or a salt thereof, is selected from the aforementioned test compounds. For example, it is a compound selected from the group consisting of a peptide, a protein, a nonpeptide compound, a synthetic compound, a fermented product, a cell extract, a plant extract, an animal tissue extract, and blood plasma. For example, it is a compound inhibiting or promoting the activities of the kinase domain of the present invention to phosphorylate proteins and to regulate CRE activity. More specific examples of an inhibitor for the polypeptide of the present invention may include dimethyl sulfoxide and staurosporine, which are know inhibitors for protein kinases.

A compound inhibiting the activity of the polypeptide of the present invention to regulate CRE activity is useful as an agent preventing or improving a disease, with which suppression of the differentiation of fat cells or the disorder of the function of glucose or lipid metabolism is associated (e.g. diabetes, adiposis, hyperlipidemia, hypertension, arteriosclerosis, hyperuricemia, cardiovascular disease, etc.). A compound promoting the activity of the polypeptide of the present invention to regulate CRE activity, for example, inhibits CRE activation, so as to promote cell death. Thus, it is expected that such a compound will be applied to treatment of cancers. In addition, when such an agent has effects on the activity of SIK1, it can be used to improve brain damage.

When a compound obtained using the screening method or screening kit of the present invention or a salt thereof is used as the aforementioned preventive or therapeutic agent, such an agent can be produced by conventional means. For example, as with the aforementioned pharmaceutical comprising the polypeptide of the present invention, the above compound or a salt thereof is converted into a tablet, a capsule, an elixir, a microcapsule, a sterilized solution, a suspension, etc., and it is then administered orally or parenterally. Since the thus obtained pharmaceutical is safe and has low toxicity, it can be administered to, for example, warm-blooded animals (e.g. a human, a mouse, a rat, a rabbit, a sheep, a swine, a bovine, a horse, a chicken, a cat, a dog, a monkey, a chimpanzee, etc.). The dosage of the above compound or a salt thereof depends on the action thereof, a target disease, an administration target, an administration route, etc. For example, when a compound inhibiting the activity of the polypeptide of the present invention to regulate CRE activity is orally administered for the purpose of treating diabetes, in general, it is administered to an adult (with a body weight of 60 kg) at a dosage between approximately 0.1 mg and 100 mg, preferably between approximately 1.0 mg and 50 mg, and more preferably between approximately 1.0 mg and 20 mg per day. When the compound is parenterally administered, the dosage thereof depends on an administration target, a target disease, etc. For example, when the compound inhibiting the activity of the polypeptide of the present invention is administered in the form of an injection to an adult (with a body weight of 60 kg) for the purpose of treating diabetes, it is convenient to administered the compound at a dosage generally between approximately 0.01 mg and 30 mg, preferably between approximately 0.1 mg and 20 mg, and more preferably between approximately 0.1 mg and 10 mg. In the case of administering the above compound to other animals also, it can be administered at an amount that is relative to a body weight of 60 kg.

Next, a method for screening a compound capable of promoting or inhibiting the induction of the polypeptide of the present invention will be described. The method for screening a compound capable of promoting or inhibiting the induction of the polypeptide of the present invention is characterized in that it comprises a step of detecting the activity of promoting or inhibiting the induction of the polypeptide of the present invention, using, as an indicator, mRNA encoding the polypeptide of the present invention, the auto-phosphorylating activity of the polypeptide of the present invention and/or the activity thereof of phosphorylating other proteins, or the activity of regulating the transcription of a gene that is under the control of a cAMP responsive element. Detection of mRNA can be carried out by known methods such as Northern blotting. The phosphorylating activity and the transcription-regulating activity can be detected by the above described methods.

When nucleotides, amino acids, and the like are indicated with abbreviated symbols in the present specification and drawings, such abbreviated symbols are used based on the abbreviated symbols created in accordance with IUPAC-IUB Commission on Biochemical Nomenclature or common abbreviated symbols in the present field. Examples of abbreviated symbols used in the present specification are shown below. When an amino acid may have an optical isomer, it indicates an L form, unless otherwise specified.

DNA: deoxyribo nucleic acid; cDNA: complementary deoxyribo nucleic acid; A: adenine; T: thymine; G: guanine; C: cytosine; RNA: ribonucleic acid; mRNA: messenger ribonucleic acid; dATP: deoxyadenosine triphosphate; dTTP: deoxythymidine triphosphate; dGTP: deoxyguanine triphosphate; dCTP: deoxycytidine triphosphate; ATP: adenosine triphosphate; EDTA: ethylenediamine tetraacetic acid; SDS: sodium dodecyl sulfate; Gly: glycine; Ala: alanine; Val: valine; Leu: leucine; Ile: isoleucine; Ser: serine; Thr: threonine; Cys: cysteine; Met: methionine; Glu: glutamic acid; Asp: asparatic acid; Lys: lysine; Arg: arginine; His: histidine; Phe: phenylalanine; Tyr: tyrosine; Trp: tryptophan; Pro: proline; Asn: asparagine; Gln: glutamine; pGlu: pyroglutamic acid

Moreover, substituents, protecting groups, and reagents frequently used in the present specification are indicated with the following symbols:
Me: methyl group; Et: ethyl group; Bu: butyl group; Ph: phenyl group; TC: thiazolidine-4(R)-carboxamide group; Tos: p-toluenesulfonyl; CHO: formyl; Bzl: benzyl; Cl₂-Bzl: 2,6-dichlorobenzyl; Bom: benzyloxymethyl; Z: benzyloxycarbonyl; Cl-Z: 2-chlorobenzyloxycarbonyl; Br-Z: 2-bromobenzyloxycarbonyl; Boc: t-butoxycarbonyl; DNP: dinitrophenyl; Trt: trityl; Bum: t-butoxymethyl; Fmoc: N-9-fluorenylmethoxycarbonyl: HOBt: 1-hydroxybenztriazole; HOOBt: 3,4-dihydro-3-hydroxy-4-oxo-1,2,3-benzotriazine; HONB: 1-hydroxy-5-norbornen-2,3-dicarboximide; DCC: N,N'-dicyclohexylcarbodiimide

### Examples

The present invention will be described in detail in the following examples. Needless to say, the examples are not intended to limit the scope of the present invention.

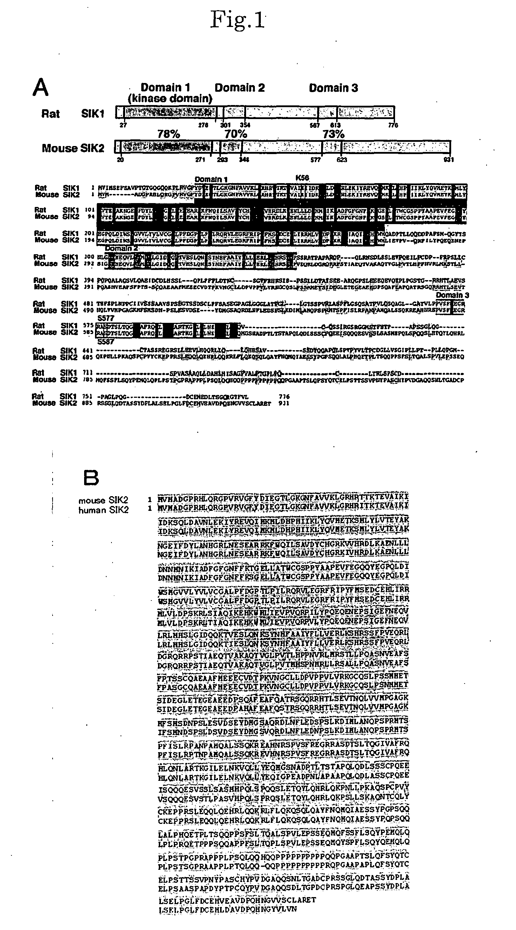

The amino acid sequences of rat SIK1 (Genbank Accession No. AB020480) and mouse SIK2 (Genbank Accession No. AB067780) proteins are shown in Figure 1A. The common region is enclosed with a square in Figure 1A. From the comparison between the primary structure of the rat SIK1 protein and that of the mouse SIK2 protein, it is clear that SIK family kinase has 3 or more domains (hereinafter the three domains are referred to as domains 1, 2, and 3). Domain 1 has a concordance rate of 78%, domain 2 has a concordance rate of 70%, and domain 3 has a concordance rate of 73%. Thus, it is considered that proteins having domains 1 to 3 belong to an SIK family. Moreover, from the results in Examples 3 and 4 described later, it can be said that the physiological activities of these proteins are also homologous.

Blast search suggests that a human has a protein, which is homologous with the mouse SIK2 protein but its functions are still unknown (GenBank Accession No. XM 041314).

Figure 1B shows the comparison between the mouse SIK2 protein and a putative protein derived from a human homologous gene (hereinafter referred to as human SIK2). Mouse SIK2 has extremely high homology with human SIK2, and such homology is higher than the homology between rat SIK1 and mouse SIK2. That is to say, it is predicted that the findings regarding the comparison between rat SIK1 and mouse SIK2 or specificity thereof are naturally applicable to human SIK2.

Moreover, from the results in Example 4, it was found that SIK2 has two protein-phosphorylating activities (an auto-phosphorylating activity and an activity of phosphorylating GST-Syntide 2) and that these activities are correlated with each other. SIK1 and SIK2 has an identical enzyme activity, and both of them lose the enzyme activity due to the substitution of homologous amino acids (SIK1 (K56M) and SIK2 (K49M)). That is to say, it is concluded that the homology between SIK1 and SIK2 is reflected on their physiological functions.

Furthermore, SIK1-specific and SIK2-specific antibodies that recognize the phosphorylated state of domain 3 were produced in Examples 7 and 8 described later. When serine existing in domain 3 of SIK1 and SIK2 (which is at position 577 in SIK1 and at position 587 in SIK2) is phosphorylated with PKA, CRE-suppressing activity is reduced, regardless of the presence or absence of the activity of kinase domain. By using these antibodies appropriately, they can be used not only as reagents for indirectly evaluating the CRE-suppressing activity of SIK, but also it can be expected that they are applied to clinical reagents used for testing diseases caused by SIK1 and SIK2.

The present invention will be further specifically described in the following examples, but the present invention is not limited to these examples. It is to be noted that gene manipulation using Escherichia coli was carried out in accordance with the method described in Molecular Cloning.

### Example 1

With reference to the cDNA sequences of an EST clone (ID: 2842716, GenBank acc: AV146436) whose 5'-terminus is homologous with rat SIK1 and an EST clone (ID: IMAGE1230878, GenBank acc: AA880086) whose 3'-terminus is homologous with rat SIK1, a 5'-terminus primer of mouse SIK2 represented by SEQ ID NO: 13 and a 3'-terminus primer of mouse SIK2 represented by SEQ ID NO: 14 were produced. It is to be noted that the portion from the 5'-terminus to the nucleotide at position 8 in SEQ ID NO: 13 is a BamHI linker. Since G at position 46 in SEQ ID NO: 14 is originally T, it is cleaved with BamHI. However, since it is preferable that the BamHI cleavage site exist in the 5'-side of cDNA in the following cloning, T at this position is substituted with G. Subsequently, from RNA derived from mouse white adipose cells (Slc: ddy (Nippon SLC)), the full-length cDNA in the protein translation region of SIK2 was amplified by the RT-PCR method.

The amplified cDNA was introduced into a pTarget vector (manufactured by Promega) using an enzyme attached therewith. Then, 5 types of the obtained independent clones were sequenced (Figure 1A). Since the sequences of 5 types of the clones were completely matched, it was concluded that the determined nucleotide sequences did not contain mutation due to PCR. When the amino acid sequence predicted from the nucleotide sequence of mouse SIK2 was confirmed through the Blast search of NCBI, it was not matched with the amino acid sequences of known proteins. That is to say, it was confirmed that mouse SIK2 was a novel protein. Figure 1A shows the sequences of mouse SIK2 and rat SIK1. Mouse SIK2 is a protein with a molecular weight of approximately 120 kDa, which has 931 amino acid residues. Mouse SIK2 has homology of 33.5% with rat SIK1. Mouse SIK2 has a sequence similar to that of rat SIK1, and it has three highly conserved domains. Domain 1 is serine/threonine protein kinase domain, which is located around the N-terminus of SIK2. Domain 1 of mouse SIK2 has homology of 78% with that of rat SIK1. Domain 2 is located in the center of the protein. Domain 2 of mouse SIK2 has homology of 70% with that of rat SIK1. Domain 3 is located at the position of one third of the C-terminus thereof. It has a protein kinase A-dependent phosphorylated portion.

### Example 2

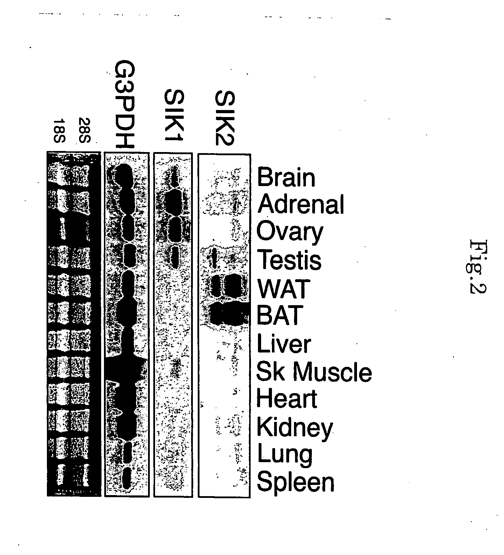

Using cDNA of mouse SIK1 (mSIK1) and that of mouse SIK2 (mSIK2) as probes (labeled with ³²P using a kit manufactured by Amersham-Bioscience), the presence of mRNA in various types of mouse tissues was analyzed by the Northern method. The results are shown in Figure 2. Figure 2 shows the results obtained by examining the presence of mRNA of SIK1 and SIK2 in various tissues. Figure 2 shows the results obtained using various tissues such as brain tissues, adrenal tissues, ovary tissues, testis tissues, WAT (white adipose tissues), BAT (brown adipose tissues), liver tissues, Sk muscle (skeletal muscle tissues), heart tissues, kidney tissues, lung tissues, and spleen tissues. G3PDH represents glyceraldehyde-3-phosphodehydrogenase. As shown in Figure 2, it was found that SIK2 was weakly expressed in the testis, but that it was specifically expressed in fat cells. Using an X-ray film, it took only a night to detect mRNA of SIK2, but it took 1 week to detect mRNA of SIK1. Thus, it was predicted that the expression level of SIK2 in fat cells that are SIK2 high expression tissues would be 10 times or more of the expression level thereof in the adrenal that are SIK1 high expression tissues.

### Example 3

Subsequently, mSIK2 (K49M) that is deficient in protein-phosphorylating activity was produced by site-directed mutagenesis, using oligo DNA represented by SEQ ID NO: 15 and a pTarget-mSIK2 vector as a template, applying GenEditor Mutagenesis kit (manufactured by Promega). The produced mutant SIK2 cDNA encodes mutant SIK2, in which lysine at position 49 was substituted with methionine with respect to the full-length SIK2.

mSIK2 was allowed to express in cultured cells and then purified. Thereafter, for the purpose of detecting the physiological activity of mSIK2, a cDNA fragment encoding wild-type SIK2 and mutant SIK2 (K49M) was cleaved from the pTarget vector by digestion with *Bam*HI and *NotI* (wherein *Not*I exists in the cloning site of pTarget and is not derived from mSIK2 cDNA). Thereafter, the cDNA fragment was introduced into the *Bam*HI-*Not*I site of glutathione S-transferase (GST) tag fusion expression vector pEBG (manufactured by Cell Signaling), so as to produce pEBG-SIK2.

### Example 4

Subsequently, the enzyme activity of mSIK2 was detected. For the expression of GST-mSIK2 or GST-mSIK2 (K49M), COS-7 cells were used. For comparison purpose, GST-rat SIK1 and GST-rat SIK1 (K59M) that was mutant SIK1 in which lysine at position 56 was substituted with methionine with respect to the full-length SIK1 (Lin et al., Molecular Endocrinology (2001) 15, 1264-1276) were produced.

PEBG-SIK2 (3 µg) obtained in Example 3 was introduced into COS-7 cells using Lipofectoamin 2000 reagent (10 µl; manufactured by Invitrogen). On the second day after the introduction, the COS-7 cells were washed with a phosphate buffer solution (PBS) twice, and they were then dissolved in 700 µl of a cell-dissolving solution (Lin et al., as described above). Thereafter, a cell extract was prepared by the method of Lin et al. (as described above). Subsequently, a GST-mSIK2 or GST-mSIK2 (K49M) protein contained in the cell extract was purified using MicroSpin GST-Purification Module (manufactured by Amersham-Biosciences).

The amount and quality of the purified GST-mSIK2 or GST-mSIK2 (K49M) protein were examined by the Western blot method using anti-GST antibody (manufactured by Amersham-Biosciences) and anti-goat-HRP fusion secondary antibody (manufactured by Rockland).

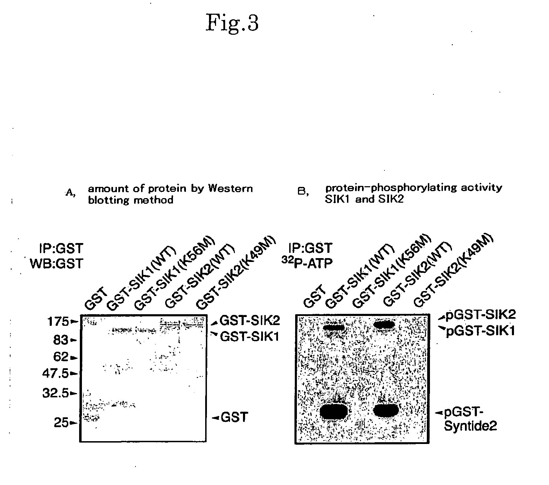

The obtained GST-mSIK2 was mixed with 1 µl of GST-Syntide 2 (Lin et al., as describe above) and [³²P]-APT (37 kBq; manufactured by ICN) in the presence of a 50 mM Tris-HCl buffer solution (pH 8.0) and 10 mM MnCl₂ (20 µl in total). The mixture was allowed to react at 30°C for 1 hour. The reaction was then terminated by adding 10 µl of an SDS-sample buffer and heating at 100°C for 5 minutes. 10 µl of the reacted sample was separated using 15% SDS-PAGE, and the SDS-PAGE gel was then dried. The intake of ³²P was detected by exposing it to light for 30 minutes, using an X-ray film (MS-film; manufactured by Kodac). The results are shown in Figure 3. Figure 3 shows the results obtained by comparing the protein-phosphorylating activity of rat SIK1 with that of mouse SIK2. As is apparent from Figure 3, it was found that wild-type GST-mSIK2 has an auto-phosphorylating activity and an activity of phosphorylating GST-Syntide 2. On the other hand, single GST and mutant GST-mSIK2 (K49M) did not have such activities. For comparison purpose, GST-rat SIK1 and GST-rat SIK1 (K56M) were subjected to the same experiment. Consequently, the same results were obtained.

### Example 5

For the purpose of detecting the CRE transcription-suppressing activity of SIK2, using Lipofectoamin 200 reagent (2 µl; manufactured by Invitrogen), 200 ng each of pTarget-SIK2 and pTarget-SIK2 (K49M), together with an expression vector (pTAL-CRE; 200 ng; manufactured by Clontech) in which a luciferase gene is located under the control of CRE, were introduced into precursor fat cells 3T3-L1. Moreover, mSIK2 (S587A) having a destroyed PKA phosphorylated portion, which was predicted to exist in mouse SIK2 domain 3 from the findings regarding rat SIK1, was introduced therein, using oligo DNA represented by SEQ ID NO: 16.

In the thus produced mSIK2 (S587A), serine at position 587 was substituted with alanine with respect to the full-length SIK2. For correction of introduction efficiency, a vector (pRL-SC40; 30 ng; manufactured by Promega) that allows luciferase derived from Renilla to express under an SV40 promoter was co-introduced therein. 16 hours later, the cells were stimulated with forskolin (50 µM) for 6 hours, so as to activate CRE. Thereafter, the cells were recovered, and the expression level of a reporter gene was analyzed using Dual Luciferase Assay kit (manufactured by Promega).

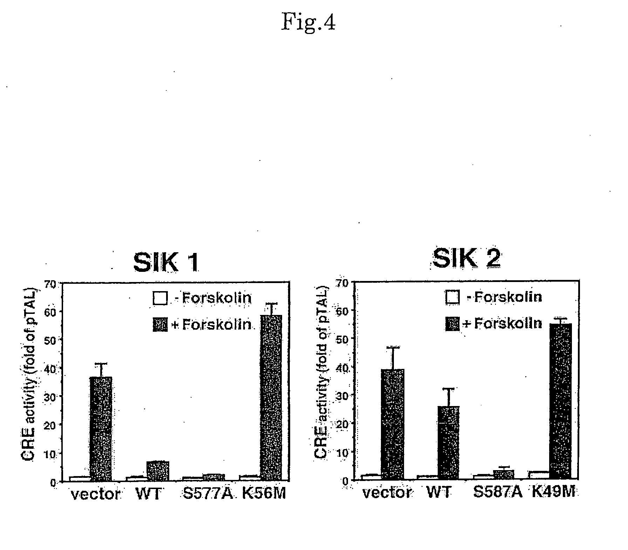

As an indicator of the CRE-specific activity, the luciferase activity of luciferase vector (pTAL)-introduced cells, to which pTAL-CRE or CRE did not bind, was first corrected with the activity of Renilla-derived luciferase. The corrected pTAL-CRE-derived activity was divided by the activity derived from pTAL, so as to obtain a specific activity derived from CRE. The results are shown in Figure 4. Figure 4 shows the results obtained by comparing the CRE-suppressing activity of rat SIK1 with that of mouse SIK2. As is apparent from Figure 4, it was found that the forced expression of SIK2 inhibits the activation of CRE. Such a CRE-suppressing activity was not observed in a case where only the vector was introduced, or in mSIK2 (K49M) that defects phosphorylating activity. From such results, it was found that the phosphorylating activity of SIK2 and the CRE-suppressing activity thereof are correlated with each other. In addition, the forced expression of SIK2 (K49M) that defects phosphorylating activity activated CRE more significantly than in the case where only the vector was introduced (control). Thus, it was suggested that differing from a normal kinase domain, the mouse SIK2 kinase domain (domain 1) comprising a mutation regarding deficiency of phosphorylating activity has an action to promote the transcription of a gene that is under the control of CRE.

For comparison purpose, the same above experiment was carried out on pIRES-rat SIK1, pIRES-rat SIK1 (K56M), and pIRES-SIK1 (S577A) wherein serine at position 577 existing in rat SIK1 domain 3 is substituted with alanine. Consequently, the same results as those in the case of mouse SIK2 were obtained. The results are shown in Figure 4.

### Example 6

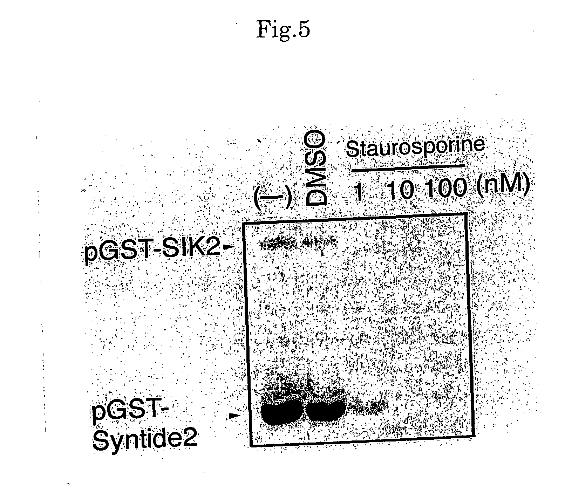

A GST-mSIK2 protein that was allowed to express in COS-7 cells and then purified was mixed with DMSO solutions containing 5% DMSO and 5% each of 1, 10, 100 nM staurosporine (Sigma) in a reaction system in the test tube used in Example 3. Thereafter, phosphorylating activity was detected by the SDS-PAGE method, so as to evaluate the inhibition of enzyme activity of mouse SIK2 by staurosporine. The results are shown in Figure 5. Figure 5 shows the results showing the protein-phosphorylating activity of mouse SIK2 that is inhibited by staurosporine. The auto-phosphorylating activity and GST-Syntide 2-phosphorylating activity of GST-mSIK2 were approximately 30% inhibited even by the single use of DMSO. Further, the phosphorylating activity of SIK2 was concentration-dependently inhibited by staurosporine. It was inhibited by 1 nM staurosporine until the residual activity became approximately 20%. By 10 nM and 100 nM staurosporines, it was almost 100% inhibited.

### Example 7

A cDNA region encoding amino acids at positions 342 to 776 in rat SIK1 or amino acids at positions 346 to 779 in mouse SIK2 was introduced into a vector (pET28a; manufactured by Novagen) that allows it to express under the control of a T7 promoter, and it was then allowed to express using a BL21 (ED3) strain. A polypeptide of interest was then purified, and it was used as an antigen. 0.4 mg of the antigen was administered 3 times to a female rabbit (strain: Japanese white rabbit) with a body weight of 1.9 kg. Titer Max Gold was used as an adjuvant at an amount of 0.5 ml/administration.

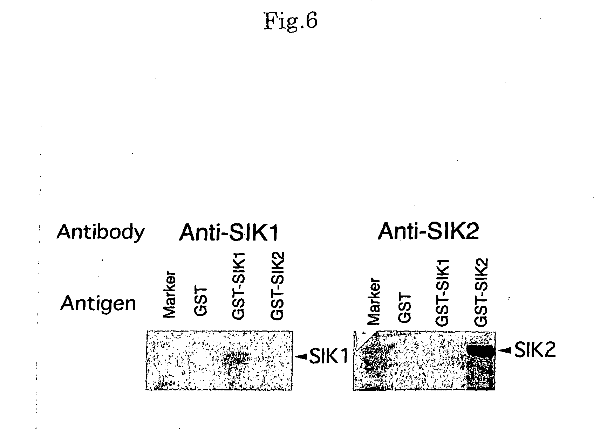

An antibody was detected by Western blotting. The results are shown in Figure 6. As shown in Figure 6, antibodies having specific reactivity to each of rat SIK1 and mouse SIK2 were produced.

### Example 8

A peptide, wherein serine existing in rat SIK1 domain 3 (serine at position 577) was phosphorylated (CQEGRRApSDTSLT; wherein cysteine was introduced into the N-terminus of amino acids from positions 571 to 582, and wherein pS represents phosphoserine), was purchased from Sigma Genosys Co., Ltd. A KLH-peptide complex was produced by binding the above peptide to keyhole limpet hemocyanin (KLH). Using this KLH-peptide complex as an immunogen, an antibody was produced. 0.4 mg of the immunogen was administered 3 times to a female rabbit (strain: Japanese white rabbit) with a body weight of 1.9 kg. Titer Max Gold was used as an adjuvant at an amount of 0.5 ml/administration.

Subsequently, the biotin-binding peptide obtained by binding the thus obtained peptide, wherein serine at position 577 was phosphorylated, to biotin was then allowed to bind to a streptoavidin column, so as to produce an affinity column. Using this affinity column, a specific antibody was purified.

10 ml of antiserum was 2 times diluted with PBS, and the diluted antiserum was then passed through the affinity column. Thereafter, a protein that had not bound was washed out with 20 ml of PBS. The bound specific antibody was eluted with 5 ml of Tris-glycine (pH 2.0), and the elution of the antibody was detected by SDS-PAGE. Thereafter, it was neutralized with Tris-HCl (pH 9.5) in an amount of one-fifth, and it was then conserved.

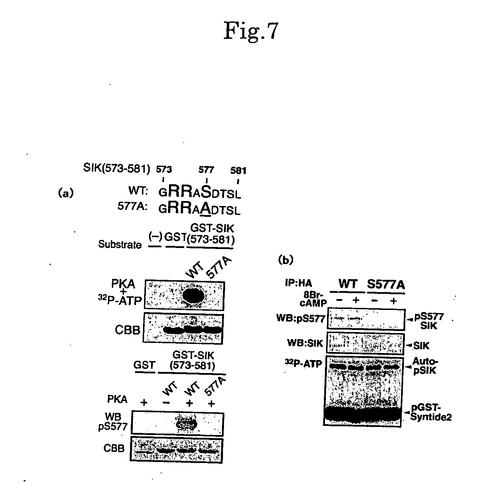

Figure 7(a) shows the results indicating the reaction between a wild-type peptide or a peptide wherein serine at position 577 is substituted with alanine, and an antibody. The term "CBB" represents Coomassie brilliant blue staining. CBB is used as a control substance showing that there is no reactivity when it does not exist and that there is reactivity when it exits. As shown in Figure 7(a), it was found that GST of a carrier protein did not react, that a wild-type peptide that was phosphorylated by PKA reacted, and that a peptide that became unphosphorylated by substituting serine at position 577 with alanine did not react. The lower experiment in Figure 7(a) shows that when a wild-type peptide is not phosphorylated by PKA, it does not react.

HA-tagged SIK1 and S577A mutant SIK1 were allowed to highly express in COS-7 cells, and they were then treated with 8Br-cAMP for 30 minutes. Thereafter, SIK1 was immunoprecipitated from the cells, using an HA-tagged antibody. Subsequently, Western blotting was carried out using an anti-SIK1 antibody and anti-phosphorylation Ser577A antibody, so as to detect SIK1 and phosphorylated Ser577.

As is clear from Figure 7(b), it was confirmed that the present antibody is capable of detecting the phosphorylation state of domain 3.

### Example 9

It was studied whether or not a peptide p300 is phosphorylated by SIK when serine at position 89 in p300 is substituted with alanine (S89A). Moreover, it was also studied whether or not the peptide p300 [84-93 (S89A)] can inhibit the phosphorylating activity of SIK. When the activity of SIK2 was measured by the method of Lin et al., a DMSO solution containing 5% DMSO (dimethyl sulfoxide) and staurosporine was added. SIK2 was allowed to express in the form of a GST binding form in COS-7 cells, and it was then purified using GST-Mirospin kit from Amersham Pharmacia.

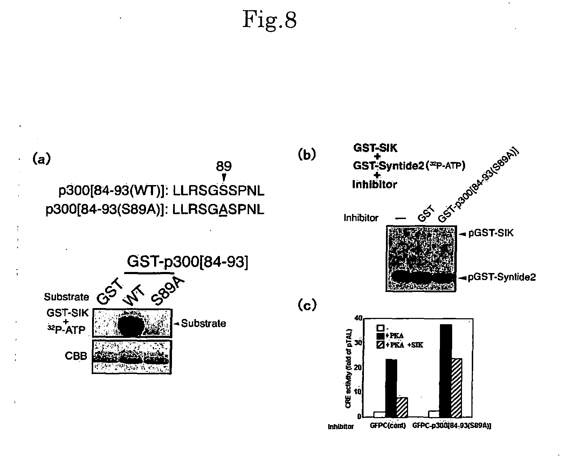

The results are shown in Figure 8. Figure 8 is a view showing the results obtained by studying an SIK inhibitor.

As shown in Figure 8 (a), it is found that a peptide wherein serine at position 89 in p300 is substituted with alanine becomes unphosphorylated.

As shown in Figure 8(b), it is found that the unphosphorylated peptide p300 wherein serine at position 89 is substituted with alanine inhibits the enzyme activity of SIK. The left lane and the central lane show the results regarding a synthetic substrate (Syntide 2) phosphorylated by SIK.

Figure 8(c) shows the experiment results obtained by examining the effects of a peptide having inhibitory activity on CRE activity. GFPC is a C-terminus binding-type green fluorescent protein. GFPC is used as a peptide carrier, wherein a peptide of interest binds to the C-terminus of GFP. The left shows a control, and the right shows the CRE activity obtained when p300 [84-93 (S89A)] binds thereto. The black one represents CRE activity obtained in a case where only PKA exists but SIK does not exist. The grey one is CRE activity obtained when both PKA and SIK exist. Thus, it was shown that p300 [84-93 (S89A)] inhibits the CRE-suppressing activity of SIK in cells, that is, the phosphorylating activity thereof.

### Example 10

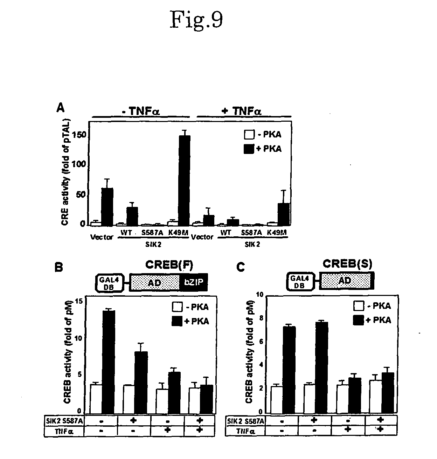

To the CRE assay system in the precursor fat cells in Example 5, 50 ng/ml Tumor Necrosis Factor-α (TNFα) was added. Thereafter, the same experiment as in Example 5 was carried out. It is to be noted that it has been reported that TNFα inhibits CREB. The results are shown in Figure 9. As is clear from Figure 9A, even when SIK2 was not highly expressed, TNFα sufficiently suppressed the activation of CRE due to PKA.

In addition, CREB was introduced into the N-terminal side of the Gal4 protein of budding yeast (Gal4 DNA binding domain; Gal4DB). Using a plasmid wherein a reporter gene (fire fly luciferase) was introduced downstream of a promoter having a sequence consisting of 5 consecutive Gal4-binding sequences, the ability of DB to transcribe CREB without the medium of CRE was analyzed. In the case of a Gal-CREB (full-length) system, since it has a bZIP domain, the transcription was inhibited by SIK2 and TNFα (refer to Figure 9B).

Inaddition, in the case of a Gal4-CREB (-bZIP) system, it was found that since there were no working points (bZIP) of SIK2, the transcription was not suppressed by SIK2, but that the transcription was suppressed by TNFα (refer to Figure 9C).

From the above results, it was confirmed that SIK2 suppresses the transcriptional activity using the bZIP domain as a working point.

Accordingly, it became clear that an SIK2 can be applied to screen an SIK2 inhibitor. That is to say, each of CREB (full-length) and CREB (-bZIP) is allowed to bind to a different DNAbinding factor. Thereafter, a reporter gene having a different sequence corresponding to the different DNA binding factor was introduced downstream thereof. For example, 5 x Gal4 binding sequence-fire fly luciferase is produced for Gal4-CREB (full-length), and 5 x LexA binding sequence-Lenilla luciferase is produced for LexA-CREB (-bZIP). These products and SIK2 are co-introduced into cells. A compound to be studied is then added thereto. Based on the ratio between the activity of fire fly luciferase and that of Lenilla, it can be studied whether or not the compound inhibits SIK2.

### Example 11

mSIK2 (S587A) having a destroyed PKA phosphorylated portion that is predicted to exist in mouse SIK2 domain 3 was produced by site-directed mutagenesis, using oligo DNA represented by SEQ ID NO: 16 and a Target-mSIK2 vector as a template, applying GenEditor Mutagenesis kit (manufactured by Prome). The produced SIK2 cDNA encoded mutant SIK2, in which serine at position 587 was substituted with alanine with respect to the full-length SIK.

mSIK2 was allowed to express in cultured cells. Thereafter, for the purpose of examining the intracellular distribution of mSIK2, cDNA fragments encoding wild-type SIK2 or mutant SIK2 (S578A) were cleaved with *Bam*HI and *NotI*. The obtained *Bam*HI-*NotI* fragments were introduced into the *Bam*HI-*Not*I site of pGFPC, so as to produce green fluorescent protein (GFP) tag fusion expression vectors, pGFP-SIK2 and pGFP-SIK2 (S587A), respectively.

The thus obtained pGFP-SIK2 and pGFP-SIK2 (S587A) (0.5 µg) were introduced into 3T3-L1 cells, using Lipofectoamin 2000 reagent (10 µl; manufactured by Invitrogen). The 3T3-L1 cells were cultured in Dulbecco's Modified Eagle's medium (DMEM; Sigma Chemical, St. Louis, MO) containing 10% fetal bovine serum and antibiotics at 37°C in an atmosphere consisting of 5% CO₂ and 95% air. After the culture was carried out for 16 hours, the cells were treated with differentiation cocktail mix (0.5 mM 3-methyl-1-isobutylxanthin, 1 µg/ml insulin, and 1 µM dexamethasone) for 12 hours. Thereafter, the cells were immobilized, followed by observation by light emission of GFP (green fluorescent protein).

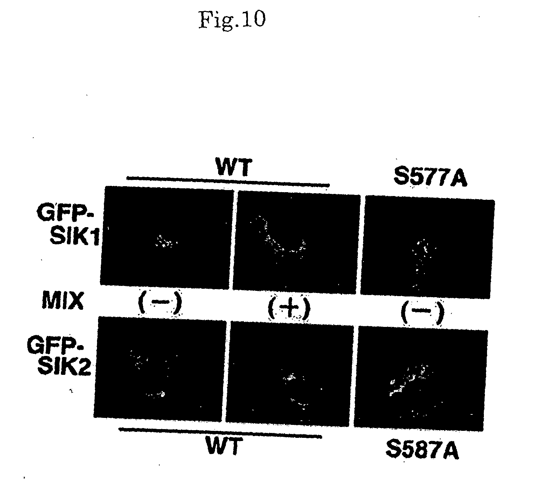

As a control, the same experiment was carried out with the exception that the treatment with differentiation cocktail mix was not carried out. In addition, using GFP-rat SIK1 and GFP-rat SIK1 (S577A), the same above experiment was carried out. The results are shown in Figure 10. Figure 10 shows the results regarding the intracellular distribution of rat SIK1 and mouse SIK2 in 3T3-L1 cells. In the figure, WT represents GFP-rat SIK and GFP-mouse SIK2. S577A and S587A represent GFP-rat SIK (S577A) and GFP-mouse SIK2 (S587A), respectively.

As shown in Figure 10, almost of GFP-SIK1 expressed in the 3T3-L1 cells existed in the nucleus. In the case of treating the cells with differentiation cocktail mix, GFP-SIK1 was transferred to the cytoplasm. On the other hand, with regard to GFP-mouse SIK2, a weak green fluorescent signal was detected in the nucleus, but almost of GFP-mouse SIK2 existed in the cytoplasm, both in the cells at the resting stage and in the cells treated with differentiation cocktail mix.

### Example 12

Since it was found that an SIK2 gene was expressed at a high level in fat cell tissues, the level of SIK2 mRNA in the differentiation process from precursor cells to fat cells was then measured. Specifically, 3T3-L1 fibroblasts were cultured in a DMEM medium for 4 days, so that the cells became confluent. After the cells became confluent, they were cultured in a DMEM medium containing differentiation cocktail mix (0.5 mM methylisobutylxanthin, 1 µg/ml insulin, and 1 µM dexamethasone) and a high concentration of glucose (2.5 g/L) for 2 days, so as to initiate the differentiation of the cells. While exchanging the medium with a fresh medium containing a high concentration of glucose every 2 days, the culture was continued for 7 days. Several cells were removed every a certain period of time, before the cells became confluent (Growth), after the cells became confluent (Confluent), and after the cells were treated. With regard to the thus removed cells, the mRNA level in SIK2 and SIK1, and other fat cell differentiation markers (Pref-1, c/EBPβ, c/EBPδ, SREBP-1, c/EBPα, PPARγ, and aP2) were assayed. Such assay was carried out by the Northern blotting analysis using necessary cDNA probes.

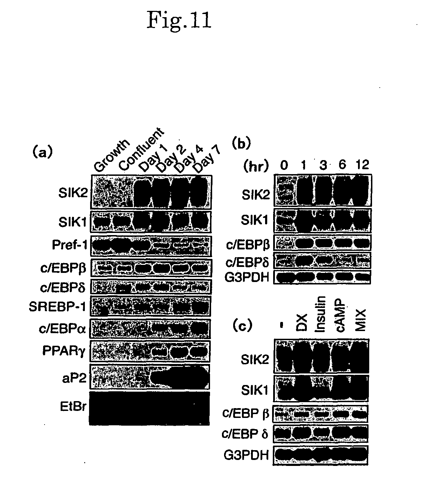

The results are shown in Figure 11. Figure 11 shows the results obtained by assaying the mRNA level of various types of fat cell differentiation markers, which is induced in the differentiation process of fat cells. As shown in Figure 11 (a), after completion of the initial culture for 24 hours, mRNA of SIK2 was significantly expressed. The mRNA level was maintained high until almost all precursor fat cells were differentiated into mature fat cells after 7 days (judged by oil Rel-O staining). On the other hand, the expression level of mRNA of SIK1 was much lower than that of SIK2. (In Figure 11(a), the exposure time of the film was different in SIK1 and SIK2.) However, the expression level increased for the initial 24 hours, and the concentration was maintained until the 7^{th} day. The mRNA level of Pref-1 as a marker of precursor fat cells began to decrease on the 2^{nd} day. The mRNA levels of c/EBPβ and c/EBPδ that were known transcriptional factors appearing at the initial stage of fat differentiation increased for the initial 24 hours, as in the case of SIK2. SREBP-1, c/EBPα, PPARγ, and aP2 are known as late responsive genes in the generation of fats. The mRNA level of these genes began to increase on the 2^{nd} or 4^{th} day.

With regard to c/EBPβ and c/EBPδ that are known to appear at the initial stage of fat differentiation, dibutyl cAMP (1 mM) was used as a constituent for differentiation cocktail mix, instead of methylisobutylxanthin for the differentiation cocktail mix. The expression level of mRNA was examined until 1 to 12 hours after stimulation for differentiation. The results are shown in Figure 11(b). As shown in Figure 11 (b), the mRNA levels of both c/EBPβ and c/EBPδ increased within 1 hour after the stimulation. Two hours later, however, the mRNA level of c/EBPδ gradually decreased.

It was examined which one of the 3 types of hormones, insulin, cAMP, and dexamethasone (DX) contained in a mixture thereof, stimulates the transcription of an SIK2 gene. The SIK2 gene was incubated with each of these hormones, and with the mixture consisting of the 3 types of hormones, for 2 hours. Thereafter, the mRNA level of SIK2 was measured. With regard to the concentration of hormones used, insulin was 1 µg/ml, cAMP was 1 mM, and dexamethasone was 1 µM. The measurement was carried out by the Northern blot analysis using cDNA probes. The results are shown in Figure 11(c). As shown in Figure 11 (c), insulin and cAMP promoted the transcription of the SIK2 gene when they were used singly. When dexamethasone was used singly, it activated the SIK2 gene at the level equivalent to that obtained by the mixture consisting of the 3 types of hormones.

### Example 13

The aforementioned results strongly suggest that SIK2 is associated with signal transduction pathway in fat cell tissues. In order to confirm such suggestion, it is necessary to specify an intracellular target molecule of SIK2 activity. A portion of the amino acid sequence of a human insulin receptor substrate (IRS) 1 was matched with the phosphorylation motif of SIK. Thus, it was examined whether or not this peptide could function as a substrate of SIK2.

In order to prepare a mammalian expression vector used for human IRS-1, pEBG-hIRS-1, *Spe* I, and *Not* I sites were produced by site-directed mutagenesis that was performed on the 5'- and 3'-termini of human IRS-1 cDNA . The obtained full-length human IRS-1 cDNA was isolated by digestion with *Spe*I and *Not*I, and the isolated fragment was then ligated into the *Spe*I*-Not*I site of a pEBG vector, so as to obtain a vector. A vector used for mutant human IRS-1 (S794A) was prepared by site-directed mutagenesis using oligo DNA represented by SEQ ID NO: 17.

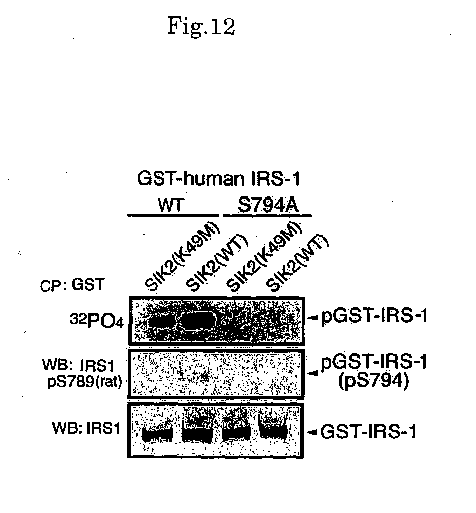

The thus obtained vector was introduced into COS-7 cells using Lipofectoamin 2000 reagent (10 µl; manufactured by Invitrogen). In addition, the vector expressing GST-SIK2 obtained in Example 3 was introduced therein, and the cells were then pre-incubated with H₃³²PO₄. Thereafter, GST-human IRS-1 or GST-human IRS-1 (S794A) was allowed to express together with GST-SIK2 or GST-SIK2 (K49M). The cells were cultured together with ³²PO₄ (0.05 mCi: 1.85 MBq) for 12 hours. Thereafter, the cells were dissolved in 700 µl of a cell dissolving solution (Lin et al., as described above). GST-human IRS-1 was purified using a glutathione column (MicroSpin GST Purification Module; manufactured by Amersham Biosciences), followed by SDS-PAGE (10%). The level of phosphorylation was visualized by autoradiography. The results are shown in the upper column in Figure 12. Figure 12 shows the results obtained by comparing the phosphorylation of GST-human IRS-1 and that of GST-human IRS-1 (S794A). As shown in Figure 12, GST-human IRS-1 was rapidly phosphorylated by SIK2, but it was also slightly phosphorylated in the cells wherein SIK2 (K49M) was expressed. This is because IRS-1 was phosphorylated by protein kinase other than SIK2 in the COS-7 cells. On the other hand, GST-human IRS-1 (S794A) was hardly phosphorylated by SIK2 and SIK2 (K49M). These results suggest that serine at position 794 in human IRS-1 was phosphorylated by SIK2 in the cells.

### Example 14

From C57BLKs/J db/db mice (hereinafter referred to as db/db fat diabetic mice) andC57BL/6Crmice (hereinafter referred to as wild-type mice), white adipose cells, brown adipose cells, the liver, and the skeletal muscle were collected. Thereafter, mRNA was extracted from each of these organs. 5 µg of RNA from each of the white adipose cell tissues and the brown adipose cell tissues, and 30 µg of RNA from the liver and the skeletal muscle were subjected to the northern blot analysis, so as to detect SIK2 mRNA.

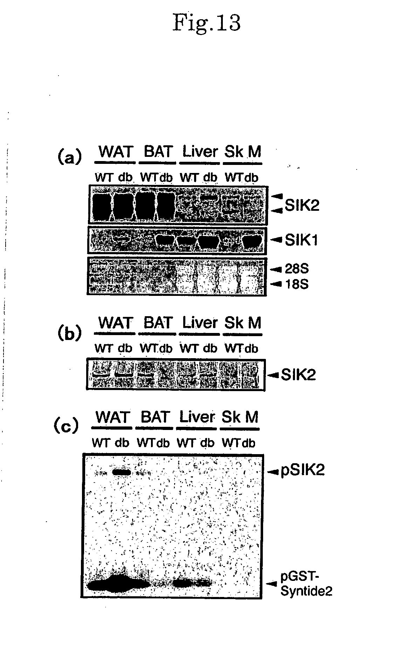

The results are shown in Figure 13 (a). In the figure, WT represents wild-type mice and db represents db/db/ fat diabetic mice. In addition, WAT represents white adipose cell tissues, BAT represents brown adipose cell tissues, Liver represents the liver, and SkM represents the skeletal muscle. As shown in Figure 13(a), in both cases of the wild-type mice and the db/db/ fat diabetic mice, SIK2 mRNA was detected at the same level in the white adipose cell tissues and in the brown adipose cell tissues. In contrast, in both cases of the wild-type mice and the db/db/ fat diabetic mice, the concentration of SIK2 mRNAwas extremely low in the liver and the skeletal muscle. The level of mRNA was the same in the wild-type mice and the db/db fat diabetic mice. As a control, the level of SIK1 mRNA was detected. The level of SIK1 mRNA in the brown adipose cell tissues, the liver, and the skeletal muscle was greater in the db/db fat diabetic mice than in the wild-type mice.

### Example 15

A protein was purified by the immunoprecipitation method from a homogenate containing 3 mg each of the white adipose cell tissues and the brown adipose cell tissues, and 18 mg each of the liver and the skeletal muscle obtained in Example 14. SIK2 was then detected by the Western blot analysis. The Western blot analysis was carried out using an anti-SIK2 antibody after SDS-PAGE. The results are shown in Figure 13(b). As shown in Figure 13(b), it is found that the amount of the SIK2 protein in the white adipose cell tissue of the db/db fat diabetic mice was larger than that of the wild-type mice. On the other hand, in the case of the brown adipose cell tissues, the amount of the protein in the db/db fat diabetic mice was smaller than that in the wild-type mice. In the liver and the skeletal muscle, the amount of SIK2 was small, and thus, almost no difference was observed between the db/db fat diabetic mice and the wild-type mice.

### Example 16

Subsequently, with regard to SIK2 purified by immunoprecipitation from the tissues used in Examples 14 and 15, the kinase activity *in vitro* was assayed using GST-Syntide 2 as a substrate. The results are shown in Figure 13 (c). As shown in Figure 13 (c), it is found that the SIK2 activity in the white adipose cell tissue of the db/db fat diabetic mice was higher than that of the wild-type mice. On the other hand, in the case of the brown adipose cell tissues, although the SIK2 activity was low both in the wild-type mice and in the db/db fat diabetic mice, the activity of the wild-type mice was higher than that of the db/db fat diabetic mice. As is clear from the results shown in Figure 13(b), it is suggested that the obtained results correspond to a difference in the content of the SIK2 protein. The liver homogenate had SIK2 activity. The activity in the liver homogenate in the db/db/ fat diabetic mice was slightly higher than that in the wild-type mice. The skeletal muscle homogenate did not exhibit SIK2 activity that was detectable. These results show that the amount of the SIK2 protein increased in the white adipose cell tissues of the diabetic mice, as in the case of the activity thereof.

### Example 17

As described in Example 8, it was found that serine existing at position 577 of rat SIK1 is phosphorylated. In addition, it was also found that serine at position 577 was phosphorylated to a considerable extent even in COS-7 cells (untreated with 8Br-cAMP) in a state where a cAMP-PKA system was not increased. Considering the phenomenon whereby SIK1 is transferred from the nucleus when serine at position 577 is phosphorylated, it is predicted that serine at position 577 is phosphorylated by phosphotransferase other than PKA even in untreated cells. For example, even in a Y1 mutant strain wherein almost no PKA activity was detected, it was observed that SIK was transferred from the nucleus in untreated cells. In addition, it has been reported that although a mutant wherein arginine at position 575 is substituted with alanine is not phosphorylated by PKA, SIK is increasingly transferred from the nucleus (Takemori H, et al., Journal of Biological Chemistry 277 (44) 42334-42343 (2002)). From the above description, it is found that serine at position 577 of SIK is phosphorylated by phosphotransferase other than PKA, and that the phosphorylated SIK is transferred from the nucleus.

Thus, it was studied whether or not SIK1 itself phosphorylates serine at position 577. Using the GST-rat SIK1 obtained in Example 4, the same experiment as in Example 4 was carried out. Wild-type rat SIK1, mutant (S577A) wherein serine at position 577 is substituted with alanine, mutant (R574A) wherein arginine at position 574 is substituted with alanine, mutant (R575A) wherein arginine at position 575 is substituted with alanine, and mutant (R574/575A) wherein arginines at positions 574 and 575 are substituted with alanines , were used as substrates.

Such mutants were produced by the method described in Takemori H, et al., Journal of Biological Chemistry 277(44) 42334-42343 (2002).

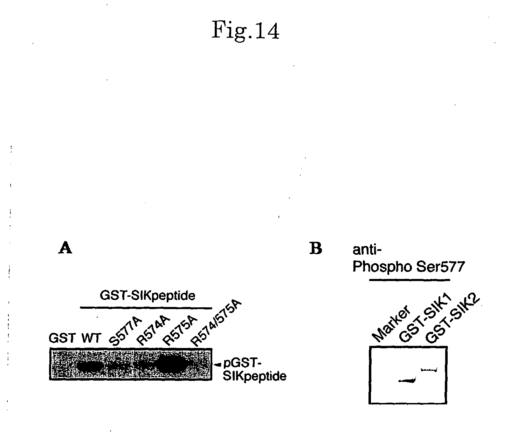

The results are shown in Figure 14A. As shown in Figure 14A, the wild-type SIK1 was phosphorylated, but the mutants S577A, R574A, and R574/575A were not phosphorylated. On the other hand, the mutant R575A was phosphorylated much more efficiently than the case of the wild-type SIK1. The reaction in the present example is an intermolecular reaction using an SIK peptide as a substrate. Since serine at position 577 of SIK1 exists in a molecule of SIK1, the concentration of the substrate is much higher than that in a test tube. Thus, it is predicted that serine at position 577 is efficiently phosphorylated by SIK1 domain 1.

### Example 18

Subsequently, using phosphorylated GST-SIK1 and phosphorylated GST-SIK2 as substrates, and using the antibody obtained in Example 8, the Western blot analysis was carried out. The results are shown in Figure 14B. As shown in Figure 14B, it was found that the antibody obtained in Example 8 recognizes the auto-phosphorylation states of serine at position 577 of SIK1 and serine at position 587 of SIK2. It is to be noted that the sequence adjacent to position 577 of SIK1 is conserved in a mouse, a rat, and a human.

### Example 19

As stated above, it was found that serine at position 577 of SIK1 is a phosphorylated portion. Meanwhile, it has been known that a portion of domain 1 or 2 of SIK1 has auto-phosphorylating activity (X. Lin et al., Molecular Endocrinology 15(8), 1264-1276 (2001)). That is to say, other than serine at position 577, there exist other auto-phosphorylated portions. Thus, in order to examine the type of amino acids that are phosphorylated, GST-rat SIK1 (1-343 amino acids) and GST-human SIK2 (3-348 amino acids) (GenBank EMBL/DDBJ Accession No. BAB91442) were allowed to react in the presence of ³²P-ATP, so that they became auto-phosphorylated. Subsequently, an SIK peptide was hydrolyzed with 6 N hydrochloric acid, and it was decomposed into individual amino acids. The amino acids were then separated by thin-layer chromatography. The detailed results are not shown herein, but the auto-phosphorylated amino acids were serines both in SIK1 and SIK2. There were 4 serines that were shared in the region of SIK1 and that of SIK2. In SIK1, these are serines at positions 135, 186, 209, and 248. These 4 serines were all substituted with alanines. Regarding the thus obtained mutants SIK1, the ratio between the protein-phosphorylating activity and the auto-phosphorylating activity was measured. The detailed results are not shown herein. When serine at position 186 was substituted with alanine, both the protein-phosphorylating activity and the auto-phosphorylating activity disappeared. From these results, it was found that serine at position 186 is an auto-phosphorylated portion.

Moreover, threonine at position 182 of SIK1 was substituted with alanine, and the same above activities were measured. As a result, it was found that the phosphorylating activity of SIK1 became extremely low. From these results, it was found that threonine at position 182 of SIK1 is phosphorylated in cells, and that it is necessary for the enzyme activity of SIK1.

Subsequently, an antibody recognizing the double phosphorylation of threonine at position 182 and serine at position 186 of SIK1 was produced as follows.

A synthetic peptide GEPLSpTWCGpSPPYAA (pT: phosphorylated threonine; pS: phosphorylated serine) was purchased from Sigma Genosys Co., Ltd. The synthetic peptide was allowed to bind to KLH, and the obtained product was used as an antigen. The method for producing an antibody was the same as that in Example 8.

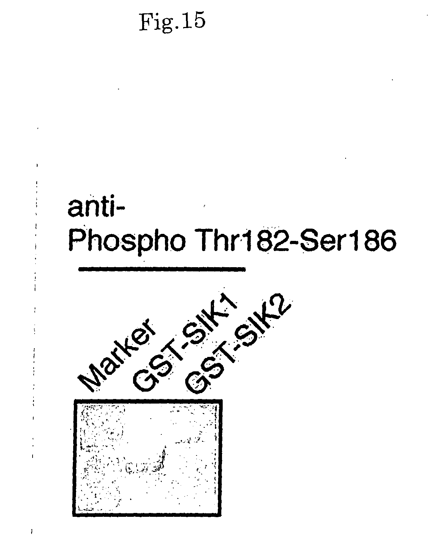

The obtained antibody and the GST-SIK1 and GST-SIK2 in Example 4 used as substrates were subjected to the Western blot analysis. The results are shown in Figure 15. As shown in Figure 15, the obtained antibody reacted well with SIK1, but it only slightly reacted with SIK2.

### Example 20

3T3-L1 cells were inoculated on 96-well plates, and the cells were cultured until they became confluent. The medium placed in such a plate was DMEM/10% FCS. The culture was carried out at 37°C in 5% CO₂. Thereafter, dexamethasone was added to each of the plates, thereby resulting in a concentration of dexamethasone of 1 µM, so that SIK was induced. After 1 hour had passed after the induction, an SIK inhibitor, staurosporine, was added to the plates, thereby resulting in concentrations of 10, 100, and 1,000 nM. Thereafter, the culture was further carried out for 1 hour. It is to be noted that 0 indicates that only a solvent was added. Subsequently, the cells were added to PBS containing 4% paraformaldehyde, and the mixture was then left at room temperature for 15 minutes, so that the cells were immobilized. Thereafter, the cells were washed with PBS 4 times, and they were then allowed to react with a blocking solution obtained by dissolving 1% skimmed milk in TBST (TBS containing 0.1% Tween 20). Subsequently, 100 µl of antiserum (500 times diluted) containing an antibody binding to SIK1 wherein serine at position 577 is phosphorylated, an antibody binding to SIK1 wherein threonine at position 182 and serine at position 186 are phosphorylated, an anti-SIK antibody, or an antibody binding to SIK1 wherein serine at position 577 is not phosphorylated, was added to the same above blocking solution. The obtained mixture was reacted overnight. After completion of the reaction overnight, an unreacted antibody portion was eliminated by washing with PBS 4 times. A solution obtained by 1,000 times diluting an HRP-labeled anti-rabbit IgG secondary antibody was added to a reaction solution obtained by dissolving 1% skimmed milk in TBS, and the mixture was then reacted at room temperature for 2 hours. Thereafter, the resultant product was washed with PBS 4 times. The antigen-antibody reaction was visualized using Konica Immuno Stain HRP reagent (manufactured by Konica).

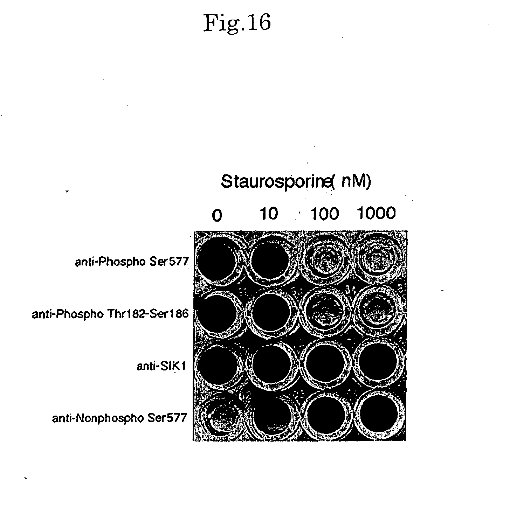

The results are shown in Figure 16. As shown in Figure 16, the reaction of an anti-phosphorylation Ser577 antibody with SIK and the reaction of an anti-phosphorylation Thr182-Ser186 antibody with SIK were significantly decreased with 10 nM staurosporine. In contrast, the reaction of SIK with an anti-SIK1 antibody was not significantly decreased by addition of staurosporine. In addition, in the case of using an antibody binding to SIK1 wherein serine at position 577 is not phosphorylated, the reactivity was increased depending on the concentration of staurosporine.

From the above results, it is found that a substance promoting or inhibiting the activity of SIK can be screened using the aforementioned system.

### Example 21

There was synthesized a peptide GELLApTWCGpSPPYAA (pT: phosphorylated threonine; pS: phosphorylated serine), wherein threonine at position 175 and serine at position 179 are phosphorylated in 15 amino acids ranging from glycine at position 170 to alanine at position 184 of human SIK2. Previously phosphorylated threonine and serine were used. A KLH-peptide complex obtained by binding the above peptide to KLH was used as an immunogen. 0.4 mg of the immunogen was subcutaneously injected into a female rabbit (Japanese white rabbit) for the initiation immunization. Thereafter, 0.2 mg of the immunogen was subcutaneously injected thereto 3 times every 2 weeks. 10 days after the final immunization, antiserum was collected. An affinity column produced by binding a peptide wherein threonine at position 175 and serine at position 179 were phosphorylated to a sepharose resin via covalent bond was equilibrated with PBS. Thereafter, the collected antiserum was passed through the column. The column was washed with PBS. Thereafter, a fraction eluted with a 0.1 M glycine-HCl buffer solution (pH 2.7) was immediately neutralized with a 1 M Tris-HCl buffer solution (pH 9.2), and then collected. The obtained fraction was dialyzed to PBS, so as to obtain a specific antibody. Approximately 0.5 mg of the specific antibody was obtained from a single rabbit.

### Example 22

For the purpose of detecting the CRE transcription-suppressing activity of SIK2, a gene encoding SIK2 was introduced into EcR293 cells.

Distilled water was added to 5 µl of pIND vector, 5 µl of 10 x H buffer (manufactured by Toyobo Co., Ltd.; consisting of 500 mM Tris-HCl (pH 7.5), 100 mM MgCl₂, 10 mM DTT, and 1,000 mM NaCl), and 2 µl of *Bam*HI, to a total amount of 50 µl. The mixture was blended and then treated with restriction enzymes at a temperature of 37°C for 1.5 hours.

Separately, the same above operations were carried out with the exception that the pEBG-SIK2 obtained in Example 3 was used instead of the aforementioned pIND vector.

After completion of the above restriction enzyme treatment, the reaction system was treated with phenol/chloroform,followed byethanolprecipitation. 2 µl of 10 x H buffer and 1 µl of *Not*I were added to the ethanol precipitate. Distilled water was further added thereto to a total amount of 20 µl. The mixture was blended and then treated with restriction enzymes at a temperature of 37°C for 1.5 hours. After completion of the reaction, DNA was subjected to electrophoresis (100 V, 30 minutes) on 1% LMP agarose gel. After completion of the electrophoresis, a band of interest was cut out using a knife, and the cut gel band was then purified using a kit for cutting and recovering a gel band.

2 µl of a solution containing the purified DNA fragment (pIND vector or pEGB-SIK2) and 1 µl of DNA ligase were placed in a test tube. Distilled water was then added thereto to a total amount of 20 µl. Then, a ligation reaction was carried out. The ligation reaction was carried out at a temperature of 16°C for 3 hours. After completion of the ligation reaction, 15 µl of JM109 competent cells were transformed and then applied on an LM-amp medium, followed by culture at 37°C for 16 hours. The obtained colonies were cultured in 3 ml of a liquid medium (LB-amp) at 37°C for 16 hours. Thereafter, a plasmid (pIND-SIK2) was recovered. The obtained plasmid was again treated with restriction enzymes, *Bam*HI and *Not*I, and the resultant product was then subjected to electrophoresis.

Ecr293 cells were transfected with the pVgRXR plasmid. The cells into which the plasmid had been introduced were selected with Zeocin. The surviving EcR293 cells were cultured at 4 types of concentrations in a 60-cm Petri dish (37°C, for 1 to 2 days). Thereafter, cells in a state where they became 50% confluent were selected, and the cells were washed with 2 ml of opti-MEM twice, followed by transfection.

0.5 µg of pIND-SIK2 and 1.5 µg of pTAL-CRE were added to 200 µl of a DMEM solution containing 10% FCS, and 6 µl of lipofectamin 2000 reagent was further added thereto, followed by mixing them. Thereafter, the mixture was incubated at room temperature for 15 minutes, so as to obtain DNA to be transfected. As a transfection reaction, the above treated DNA solution was added to the above treated cells, and the mixture was reacted at 37°C for 3 hours. After completion of the reaction, the medium was exchanged with 4 ml of a fresh DMEM solution containing 10% FCS, and the culture was then carried out at 37°C for 16 hours.

Thereafter, the medium for culturing the cells was exchanged with DMEM + 10%FCS + Geneticin (500 µg/µl), and the cells were cultured for about 1 week, while exchanging the medium with a fresh one every 2 days. When the cells died to just the light extent, using a chip, a single cell was transferred into each well of a 24-well plate. Thereafter, the culture was further carried out. When cells increased to just the light extent, reporter assay was carried out, and a stabilized cell line was collected. The collected cells were again selected by the above described method, and a stabilized cell line was collected. The second screening was carried out, and the collected cells were allowed to grow in high volume, so as to obtain cells used to detect the CRE transcription-suppressing activity of SIK2.

A DMEM medium containing 10% FCS (0.2 ml) was placed in each well of a 96-well plate. Thereafter, the cells obtained as described above were placed in each well, and they were cultured at 37°C in 5% CO₂, until the cells became confluent. Subsequently, ponasterone A (4.3 µM; manufactured by Invitrogen), forskolin (40 µM), and a test compound were placed in each well, followed by giving stimulation for 6 hours. After completion of the stimulation for 6 hours, the cells were recovered, and the luciferase activitywas measured using Dual Luciferase Assay kit (Promega), so as to examine the SIK2-inhibiting activity of the test compound.

The present invention provides SIK2 specifically expressing in fat tissues, an antibody specific for SIK2, and an antibody recognizing the phosphorylation state of SIK2, thereby proposing a reagent for identifying a compound promoting or regulating the physiological activity of SIK2, or a reagent for evaluating the CRE-suppressing activity thereof. In addition, the present invention also provides a composition suppressing or increasing the action of SIK2 by screening the agonist or antagonist of SIK2. Moreover, it is expected that such a composition will be used as an agent for preventing or improving a disease, with which the differentiation of fat cells or the disorder of glucose or lipid metabolism is associated, in particular, disease caused by metabolic disorder, such as diabetes, hyperlipidemia, hypertension or arteriosclerosis, or adiposis itself.

Furthermore, a compound promoting the protein-phosphorylating activity of the polypeptide of the present invention has a possibility of inhibiting the cell survival activity via CRE activation, thereby causing cell death. Thus, it is expected that such a compound will be used against cancers. It is also expected that an agent for inhibiting or promoting SIK functions will regulate CRE activity that is associated with cell survival, when it specifically acts due to SIK, thereby preventing brain damage. Thus, it is expected that such an agent will have effects of improving Alzheimer's disease and the like.

## Claims

1. A polypeptide comprising an amino acid sequence having homology of at least 90% to any one of the polypeptides described in the following (A) to (L), or a salt thereof:
(A) a polypeptide comprising an amino acid sequence identical to or substantially identical to the amino acid sequence represented by SEQ ID NO: 2, or a polypeptide comprising an amino acid sequence encoded by cDNA capable of hybridizing to the nucleotide sequence represented by SEQ ID NO: 1;
(B) a polypeptide comprising an amino acid sequence identical to or substantially identical to the amino acid sequence represented by SEQ ID NO: 2 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 1 and having activity of regulating the transcription of a gene that is under the control of a cAMP responsive element;
(C) a polypeptide comprising an amino acid sequence identical to or substantially identical to the amino acid sequence represented by SEQ ID NO: 4, or a polypeptide comprising an amino acid sequence encoded by cDNA capable of hybridizing to the nucleotide sequence represented by SEQ ID NO: 3;
(D) a polypeptide comprising an amino acid sequence identical to or substantially identical to the amino acid sequence represented by SEQ ID NO: 4 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 3 and having activity of regulating the transcription of a gene that is under the control of a cAMP responsive element;
(E) a polypeptide comprising an amino acid sequence identical to or substantially identical to the amino acid sequence represented by SEQ ID NO: 6, or a polypeptide comprising an amino acid sequence encoded by cDNA capable of hybridizing to the nucleotide sequence represented by SEQ ID NO: 5;
(F) a polypeptide comprising an amino acid sequence identical to or substantially identical to the amino acid sequence represented by SEQ ID NO: 6 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 5 and having activity of regulating the transcription of a gene that is under the control of a cAMP responsive element;
(G) a polypeptide comprising an amino acid sequence identical to or substantially identical to the amino acid sequence represented by SEQ ID NO: 8, or a polypeptide comprising an amino acid sequence encoded by cDNA capable of hybridizing to the nucleotide sequence represented by SEQ ID NO: 7;
(H) a polypeptide comprising an amino acid sequence identical to or substantially identical to the amino acid sequence represented by SEQ ID NO: 8 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 7 and having activity of regulating the transcription of a gene that is under the control of a cAMP responsive element;
(I) a polypeptide comprising an amino acid sequence identical to or substantially identical to the amino acid sequence represented by SEQ ID NO: 10, or a polypeptide comprising an amino acid sequence encoded by cDNA capable of hybridizing to the nucleotide sequence represented by SEQ ID NO: 9;
(J) a polypeptide comprising an amino acid sequence identical to or substantially identical to the amino acid sequence represented by SEQ ID NO: 10 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 9 and having activity of regulating the transcription of a gene that is under the control of a cAMP responsive element;
(K) a polypeptide comprising an amino acid sequence identical to or substantially identical to the amino acid sequence represented by SEQ ID NO: 12, or a polypeptide comprising an amino acid sequence encoded by cDNA capable of hybridizing to the nucleotide sequence represented by SEQ ID NO: 11; and
(L) a polypeptide comprising an amino acid sequence identical to or substantially identical to the amino acid sequence represented by SEQ ID NO: 12 and having activity of regulating the transcription of a gene that is under the control of a cAMP responsive element, or a polypeptide comprising an amino acid sequence encoded by cDNA capable of hybridizing to the nucleotide sequence represented by SEQ ID NO: 11 and having activity of regulating the transcription of a gene that is under the control of a cAMP responsive element.

2. The polypeptide according to claim 1, or a salt thereof, which is obtained by separating and purifying from warm-blooded animal cells.

3. The polypeptide according to claim 2, or a salt thereof, wherein the animal cells are derived from a mouse.

4. A polynucleotide comprising a nucleotide sequence having homology of at least 95% to any one of the polynucleotides described in the following (a) to (m):
(a) a polynucleotide comprising the nucleotide sequence represented by SEQ ID NO: 1, or a polynucleotide that is cDNA capable of hybridizing to the polynucleotide comprising the nucleotide sequence represented by SEQ ID NO: 1;
(b) a polynucleotide comprising the nucleotide sequence represented by SEQ ID NO: 3, or a polynucleotide that is cDNA capable of hybridizing to the polynucleotide comprising the nucleotide sequence represented by SEQ ID NO: 3;
(c) a polynucleotide comprising the nucleotide sequence represented by SEQ ID NO: 5, or a polynucleotide that is cDNA capable of hybridizing to the polynucleotide comprising the nucleotide sequence represented by SEQ ID NO: 5;
(d) a polynucleotide comprising the nucleotide sequence represented by SEQ ID NO: 7, or a polynucleotide that is cDNA capable of hybridizing to the polynucleotide comprising the nucleotide sequence represented by SEQ ID NO: 7;
(e) a polynucleotide comprising the nucleotide sequence represented by SEQ ID NO: 9, or a polynucleotide that is cDNA capable of hybridizing to the polynucleotide comprising the nucleotide sequence represented by SEQ ID NO: 9;
(f) a polynucleotide comprising the nucleotide sequence represented by SEQ ID NO: 11, or a polynucleotide that is cDNA capable of hybridizing to the polynucleotide comprising the nucleotide sequence represented by SEQ ID NO: 11;
(g) a polynucleotide comprising a nucleotide sequence encoding a polypeptide comprising an amino acid sequence identical to or substantially identical to the amino acid sequence represented by SEQ ID NO: 2, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 2;
(h) a polynucleotide comprising a nucleotide sequence encoding a polypeptide comprising an amino acid sequence identical to or substantially identical to the amino acid sequence represented by SEQ ID NO: 4, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 4;
(i) a polynucleotide comprising a nucleotide sequence encoding a polypeptide comprising an amino acid sequence identical to or substantially identical to the amino acid sequence represented by SEQ ID NO: 6, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 6;
(j) a polynucleotide comprising a nucleotide sequence encoding a polypeptide comprising an amino acid sequence identical to or substantially identical to the amino acid sequence represented by SEQ ID NO: 8, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 8;
(k) a polynucleotide comprising a nucleotide sequence encoding a polypeptide comprising an amino acid sequence identical to or substantially identical to the amino acid sequence represented by SEQ ID NO: 10, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 10;
(l) a polynucleotide comprising a nucleotide sequence encoding a polypeptide comprising an amino acid sequence identical to or substantially identical to the amino acid sequence represented by SEQ ID NO: 12, or a polynucleotide that is cDNA capable of hybridizing to the polynucleotide comprising the nucleotide sequence encoding the polypeptide comprising an amino acid sequence identical to or substantially identical to the amino acid sequence represented by SEQ ID NO: 12; and
(m) a polynucleotide capable of hybridizing to any one of the polynucleotides described in (a) to (l) above under stringent conditions.

5. A recombinant vector comprising a polynucleotide as recited in claim 4.

6. An expression vector comprising a the polynucleotide as recited in claim 4.

7. Host cells harboring an expression vector as recited in claim 6.

8. A method for producing a polypeptide or a salt thereof as recited in claim 1, which comprises culturing a host cell as recited in claim 7 under conditions that are suitable for the expression of the polypeptide, and recovering the polypeptide from the culture product obtained.

9. The method for producing a polypeptide or a salt thereof according to claim 8, wherein the culture of the host cell is carried out in the presence of a substance having an action to induce fat differentiation.

10. A method for producing a polypeptide or a salt thereof according to claim 1, which comprises culturing precursor fat cells in the presence of a substance having an action to induce fat differentiation, and recovering the polypeptide as recited in claim 1 or a salt thereof from the culture product obtained.

11. A polypeptide or a salt thereof produced by a method for producing a polypeptide or a salt thereof as recited in any one of claims 8 to 10.

12. A nucleic acid probe, which is useful for detecting a polynucleotide as recited in claim 4 or a polynucleotide encoding a polypeptide as recited in claim 1.

13. An antibody having an affinity for the polypeptide as recited in claim 1 or a fragment thereof.

14. A hybridoma capable of generating an antibody having an affinity for the polypeptide as recited in claim 1.

15. A pharmaceutical composition, which comprises the polypeptide as recited in claim 1 or the recombinant vector as recited in claim 5.

16. The pharmaceutical composition according to claim 15, which further comprises a substance having an action to induce fat differentiation.

17. The pharmaceutical composition according to claim 15 or 16, which is used as an agent for preventing or improving a disease with which the differentiation of fat cells or an increase in the function of glucose or lipid metabolism is associated.

18. The pharmaceutical composition according to claim 15 or 16, wherein the disease is diabetes, adiposis, hyperlipidemia, hypertension, arteriosclerosis, hyperuricemia, or a cardiovascular disease.

19. A composition for use in gene diagnosis, which comprises a polynucleotide as recited in claim 4.

20. The composition for use in gene diagnosis according to claim 19, which detects the expression of DNA or mRNA encoding salt-inducible kinase 2.

21. The composition for use in gene diagnosis according to claim 19, which is used for diagnosis of a disease with which the differentiation of fat cells or the disorder of function of glucose or lipid metabolism is associated.

22. The composition for use in gene diagnosis according to claim 21, wherein the disease is diabetes, adiposis, hyperlipidemia, hypertension, arteriosclerosis, hyperuricemia, or a cardiovascular disease.

23. A pharmaceutical composition, which comprises an antibody as recited in claim 13, a fragment thereof, or an antisense nucleotide complementarily binding to a polynucleotide as recited in claim 4.

24. The pharmaceutical composition according to claim 23, which is used as an agent for preventing or improving a disease with which the differentiation of fat cells or glucose or lipid metabolism is associated.

25. The pharmaceutical composition according to claim 23, wherein the disease is diabetes, adiposis, hyperlipidemia, hypertension, arteriosclerosis, hyperuricemia, or a cardiovascular disease.

26. The pharmaceutical composition according to claim 23, which is used for diagnosis of a disease with which the suppression of the differentiation of fat cells or the disorder of the metabolic function thereof is associated.

27. The pharmaceutical composition according to claim 23, wherein the disease is diabetes, adiposis, hyperlipidemia, hypertension, arteriosclerosis, hyperuricemia, or a cardiovascular disease.

28. A method for preventing or improving a disease or physiological condition which is developed by a decrease in the expression of salt-inducible kinase 2, **characterized in that** the method comprises administration of a pharmaceutical composition as recited in claim 15 or 16.

29. The method for preventing or improving a disease or physiological condition according to claim 28, wherein the disease or physiological condition involves the disorder of glucose metabolism, the disorder of lipid metabolism, or cranial nerve injury.

30. A method for preventing or improving a disease or physiological condition which is developed by an increase in the expression of salt-inducible kinase 2, **characterized in that** the method comprises administration of a pharmaceutical composition as recited in claim 23.

31. The method for preventing or improving a disease or physiological condition according to claim 30, wherein the disease or physiological condition involves the disorder of glucose metabolism, the disorder of lipid metabolism, or cranial nerve injury.

32. A method for screening a compound capable of promoting or inhibiting the activity of a polypeptide as recited in claim 1, comprising the steps of: allowing an analyte comprising a polypeptide as recited in claim 1 to come into contact with a test compound; and detecting an activity of promoting or inhibiting the activity of the polypeptide as recited in claim 1.

33. The screening method according to claim 32, which detects the activity of promoting or inhibiting the activity of the polypeptide as recited in claim 1, using the auto-phosphorylating activity of the polypeptide as recited in claim 1 and/or the activity thereof of phosphorylating other proteins as an indicator.

34. A method for screening a compound capable of promoting or inhibiting the activity of a polypeptide as recited in claim 1, the method comprising steps of: allowing an expression vector comprising a polynucleotide as recited in claim 4 and a reporter gene that is under the control of a cAMP responsive element to come into contact with a test compound; and detecting an activity of promoting or inhibiting the activity of the polypeptide as recited in claim 1.

35. A method for screening a compound specifically binding to a polypeptide as recited in claim 1, comprising steps of: allowing a polypeptide as recited in claim 1 to come into contact with a test compound; and detecting the binding of the test compound with the polypeptide, thereby identifying a compound specifically binding to the polypeptide.

36. A method for screening a compound having an ability to regulate the activity of a polypeptide as recited in claim 1, the method comprising steps of: allowing a polypeptide as recited in claim 1 to come into contact with a test compound under conditions where the polypeptide exhibits its activity; evaluating the activity of the polypeptide in the presence of the test compound; comparing the thus evaluated activity with the activity of the polypeptide in the absence of the test compound; and identifying the ability of the test compound to regulate the activity of the polypeptide based on the comparison results.

37. A method for screening a compound having effects on the expression of a polynucleotide as recited in claim 4, the method comprising steps of: allowing a target polynucleotide analyte comprising a polynucleotide as recited in claim 4 to come into contact with a test compound under conditions that are suitable for the expression of the target polynucleotide; detecting a change in the expression of the target nucleotide; and comparing the expression of the target polynucleotide in the absence of the test compound and in the presence of various amounts of the test compounds.

38. The method for screening a compound according to any one of claims 32 to 37, which is carried out in the presence of a substance having an action to induce fat differentiation.

39. A method for screening a compound capable of promoting or inhibiting the activity of a protein having an auto-phosphorylation ability, comprising:
a phosphorylating step of phosphorylating a protein having auto-phosphorylation ability in the presence of a test compound;
an antibody-binding step of allowing an antibody recognizing an auto-phosphorylated portion of the protein that is in a state where it has been phosphorylated or has not been phosphorylated, to react with the protein; and
a measuring step of measuring the reaction of the protein with the antibody.

40. A method for screening a compound capable of promoting or inhibiting the activity of salt-inducible kinase, comprising:
a phosphorylating step of phosphorylating salt-inducible kinase in the presence of a test compound;
an antibody-binding step of allowing an antibody recognizing an auto-phosphorylated portion of the salt-inducible kinase that is in a state where it has been phosphorylated or has not been phosphorylated, to react with the salt-inducible kinase; and
a measuring step of measuring the reaction of the salt-inducible kinase with the antibody.

41. A method for screening a compound capable of promoting or inhibiting the activity of the polypeptide according to claim 1, comprising:
a phosphorylating step of phosphorylating a peptide as recited in claim 1 in the presence of a test compound;
an antibody-binding step of allowing an antibody recognizing an auto-phosphorylated portion of the polypeptide that is in a state where it has been phosphorylated or has not been phosphorylated, to react with the polypeptide; and
a measuring step of measuring the reaction of the polypeptide with the antibody.

42. The screening method according to claim 39, wherein cells having an ability to generate a protein having an auto-phosphorylation ability or host cells transformed with an expression vector comprising such a protein having an auto-phosphorylation ability are cultured under conditions that are suitable for the expression of a protein, and a polypeptide obtained from the obtained culture product is used as a protein having an auto-phosphorylation ability.

43. The screening method according to claim 40, wherein cells having an ability to generate salt-inducible kinase or host cells transformed with the expression vector as recited in claim 6 are cultured under conditions that are suitable for the expression of a polypeptide, and a polypeptide obtained from the obtained culture product is used as salt-inducible kinase.

44. The screening method according to claim 41, wherein host cells transformed with an expressionvector as recited in claim 6 are cultured under conditions that are suitable for the expression of a polypeptide, and a polypeptide obtained from the obtained culture product is used as the polypeptide or a salt thereof.

45. The screening method according to any one of claims 39 to 44, which is carried out in the presence of a substance having an action to induce fat differentiation.

46. A method for screening a compound capable of promoting or inhibiting the activity of a protein having an auto-phosphorylation ability, comprising:
a step of culturing cells having an ability to generate a protein having an auto-phosphorylation ability in the presence of a test compound under conditions that are suitable for the expression of the protein;
an antibody-binding step of allowing the cells to come into contact with an antibody recognizing an auto-phosphorylated portion of the protein that is in a state where it has been phosphorylated or has not been phosphorylated, so as to allow the protein to react with the antibody; and
a measuring step of detecting the reaction of the protein with the antibody.

47. The screening method according to claim 46, wherein the culture of the cells is carried out in the presence of a substance having an action to induce fat differentiation.

48. A method for screening a compound capable of promoting or inhibiting the activity of salt-inducible kinase, comprising:
a step of culturing cells having an ability to generate salt-inducible kinase in the presence of a test compound under conditions that are suitable for the expression of salt-inducible kinase;
an antibody-binding step of allowing the cells to come into contact with an antibody recognizing an auto-phosphorylated portion of the salt-inducible kinase that is in a state where it has been phosphorylated or has not been phosphorylated, so as to allow the salt-inducible kinase to react with the antibody; and
a measuring step of detecting the reaction of the salt-inducible kinase with the antibody.

49. The screening method according to claim 48, wherein the culture of the cells is carried out in the presence of a substance having an action to induce fat differentiation.

50. A method for screening a compound capable of promoting or inhibiting the activity of a polypeptide comprising:
a step of culturing host cells harboring a recombinant vector comprising a polynucleotide as recited in claim 4 in the presence of a test compound under conditions that are suitable for the expression of a polypeptide;
an antibody-binding step of allowing the host cells to come into contact with an antibody recognizing an auto-phosphorylated portion of the polypeptide that is in a state where it has been phosphorylated or has not been phosphorylated, so as to allow the polypeptide to react with the antibody; and
a measuring step of detecting the reaction of the polypeptide with the antibody.

51. The screening method according to claim 50, wherein the culture of the cells is carried out in the presence of a substance having an action to induce fat differentiation.

52. A method for screening a compound capable of promoting or inhibiting the activity of salt-inducible kinase, comprising:
a phosphorylating step of allowing salt-inducible kinase to come into contact with a substrate that is to be phosphorylated with the salt-inducible kinase in the presence of a test compound, so as to phosphorylate the substrate;
an antibody-binding step of allowing an antibody recognizing a substrate that is in a state where it has been phosphorylated or has not been phosphorylated, to react with the substrate; and
a measuring step of detecting the reaction of the substrate with the antibody.

53. A pharmaceutical composition, which comprises the compound capable of promoting or inhibiting the activity of a polypeptide as recited in claim 1, which is identified by a screening method as recited in any one of claims 32 to 52.

54. The screening method according to any one of claims 32 to 52, which screens for a compound used for preventing or treating diabetes, adiposis, hyperlipidemia, hypertension, arteriosclerosis, hyperuricemia, or a cardiovascular disease.

55. A kit for screening a compound capable of promoting or inhibiting the activity of the polypeptide according to claim 1, comprising a polypeptide as recited in claim 1.

56. The screening kit according to claim 55, which comprises a polypeptide as recited in claim 1 or a phosphate group donor that phosphorylates other substrates.

57. The screening kit according to claim 55 or 56, which comprises a substrate polypeptide that is phosphorylated by a polypeptide as recited in claim 1.

58. A kit for screening a compound capable of promoting or inhibiting a polypeptide as recited in claim 1, comprising host cells transformed with an expression vector as recited in claim 6.

59. The screening kit according to claim 58, which comprises a medium used for the host cells and a reagent for activating a cAMP responsive element.

60. The screening kit according to any one of claims 55 to 59, which comprises a substance having an action to induce fat differentiation.

61. A kit for screening a compound capable of promoting or inhibiting the activity of a protein having an auto-phosphorylation ability, comprising a protein having an auto-phosphorylation ability and an antibody recognizing an auto-phosphorylated portion of the protein that is in a state where it has been phosphorylated or has not been phosphorylated.

62. A kit for screening a compound capable of promoting or inhibiting the activity of salt-inducible kinase, comprising salt-inducible kinase and an antibody recognizing an auto-phosphorylated portion of the salt-inducible kinase that is in a state where it has been phosphorylated or has not been phosphorylated.

63. A kit for screening a compound capable of promoting or inhibiting the activity of the polypeptide according to claim 1, comprising a polypeptide as recited in claim 1 and an antibody recognizing an auto-phosphorylated portion of the polypeptide that is in a state where it has been phosphorylated or has not been phosphorylated.

64. A kit for screening a compound capable of promoting or inhibiting the activity of a protein having an auto-phosphorylation ability, comprising cells having an ability to generate a protein having an auto-phosphorylation ability and an antibody recognizing an auto-phosphorylated portion of the protein that is in a state where it has been phosphorylated or has not been phosphorylated.

65. A kit for screening a compound capable of promoting or inhibiting the activity of a protein having an auto-phosphorylation ability, comprising cells having an ability to generate salt-inducible kinase and an antibody recognizing an auto-phosphorylated portion of the salt-inducible kinase that is in a state where it has been phosphorylated or has not been phosphorylated.

66. A kit for screening a compound capable of promoting or inhibiting the activity of a protein having an auto-phosphorylation ability, comprising host cells harboring a recombinant vector comprising the polynucleotide as recited in claim 4 and an antibody recognizing an auto-phosphorylated portion of a protein that is in a state where it has been phosphorylated or has not been phosphorylated.

67. A kit for screening a compound capable of promoting or inhibiting the activity of salt-inducible kinase, comprising host cells harboring a recombinant vector comprising a polynucleotide as recited in claim 4 and an antibody recognizing an auto-phosphorylated portion of salt-inducible kinase that is in a state where it has been phosphorylated or has not been phosphorylated.

68. A kit for screening a compound capable of promoting or inhibiting the activity of the polypeptide according to claim 1, comprising host cells harboring a recombinant vector comprising a polynucleotide as recited in claim 4 and an antibody recognizing a polypeptide as recited in claim 1 that is in a state where it has been phosphorylated or has not been phosphorylated.

69. The screening kit according to any one of claims 61 to 68, which comprises a substance having an action to induce fat differentiation.

70. A method for screening a compound capable of promoting or inhibiting the induction of a polypeptide as recited in claim 1, comprising a step of detecting the activity of promoting or inhibiting the induction of a polypeptide as recited in claim 1, using, as an indicator, mRNA encoding the polypeptide as recited in claim 1, the auto-phosphorylating activity of the polypeptide as recited in claim 1 and/or the activity thereof of phosphorylating other proteins, or the activity of regulating the transcription of a gene that is under the control of a cAMP responsive element.


SEQUENCE LISTING

<110> Protein Express Co., Ltd.
Takemori, Hiroshi
5 Okamoto, Mitsuhiro

<120> Salt-inducible kinase and its use

<130> PCTF161
10

<140>
<141>

<150> 2002-240092
15 <151> 2002-08-21

<150> 2003-23295
<151> 2003-01-31

20 <160> 17

<170> PatentIn Ver. 2.1

<210> 1
25 <211> 2986
<212> DNA
<213> Mus musculus

<400> 1

gaggcgcgcc gcccgacgcc ctcctgcccg ctccgtgccg cttcttgccg gccagttcgt 60

tcgcgttccc gtcgccgtgt cctgcagcgg gggccaagca tggtcatggc ggatggcccg 120

aggcacttgc agcgcgggcc agtccgggtg gggttctacg acatcgaggg cacgctgggc 180

aagggcaact ttgccgtggt gaagctgggg cggcaccgga ccaccaagac ggaggtggct 240

ataaaaataa tagacaagtc acagctggat gcagtaaacc ttgagaaaat ctaccgagaa 300

gtacagataa tgaaaatgct cgaccatcct cacatcatta aactgtatca ggtaatggag 360

accaaaagta tgttgtacct tgtgacagaa tatgccaaaa atggagaaat ttttgattat 420

cttgctaatc atggccgatt aaatgaatct gaagccagga gaaaattctg gcaaattctg 480

tctgctgttg attattgcca tggccggaag gtagtgcaca gagacctgaa ggctgaaaat 540

ctcctgctgg ataacaacat gaatatcaaa atagcagatt tcggctttgg aaatttcttt 600

aaaactggtg aactgctggc aacatggtgt ggcagccccc cttatgcagc cccagaagtc 660

tttgaagggc agcagtatga aggaccacag ctggatatat ggagcatggg agttgttctt 720

tatgtccttg tctgtggagc tctacctttt gatggaccaa ctctccctat tttgaggcag 780

agggttttag aaggaagatt ccggattcct tatttcatgt cagaagattg tgaacacctc 840

attagaagga tgttggtcct agatccttcc aaacggctaa gcatagctca aatcaaggag 900

cacaagtgga tgctcataga ggttcctgta cagaggccta ttctctaccc acaagaacag 960

gaaaacgagc cgtccattgg agaatttaat gagcaggttc ttcgactgat gcacagcctt 1020

gggattgatc agcagaagac tgttgagtct ttgcaaaaca agagctataa tcactttgct 1080

gccatttatt tcttgttggt ggaacgtcta aaatcacata ggagcagttt tcctgtggag 1140

cagagacttg atggccgcca gcgtcggcct agcaccattg ctgaacaaac agttgccaag 1200

gcacaaactg tggggctgcc agtgaccttg catccaccga acgtgagact gatgcgatct 1260

accctcctcc cacaggcatc caatgtggag gccttttcat ttccaacatc cagctgtcaa 1320

gcagaagctg cctttatgga ggaagagtgt gtcgacactc caaaggtgaa tggctgcctg 1380

cttgaccctg tgccccctgt cctggtgagg aagggatgcc agtcactgcc cagcagtatg 1440

atggagacct ccattgatga aggcttggag acagaaggag aggctgagga agaccccagt 1500

caggcctttg aagctttcca ggccacacgc agtgggcagc gacggcacac tctgtcagaa 1560

gtgactaacc aattggttgt gatgcccggg gcagggaaaa tgttctccat gagtgataac 1620

ccctcccttg agagtgtgga ctctgagtat gatatggggt ctgcccagag ggacctgaac 1680
tttctggaag acagcccttc cttgaaggac atcatgttag ccaatcagcc gtcaccccgc 1740
atgacatctc ctttcataag cctcagacct gccaacccag ccatgcaggc tctgagctca 1800
cagaagcgtg aggcccacaa ccggtccccc gtgagcttcc gagaaggccg cagagcatca 1860
gatacgtccc ttacacaagg aattgtggca tttagacaac atcttcagaa tcttgctaga 1920
accaaaggaa ttctggagtt gaacaaagta caattgctgt atgaacaaat gggatcaaac 1980
gcagacccta ccttaacatc aactgctcct cagctccaag acctttcgag cagttgccct 2040
caggaggaaa tctcccagca gcaggaaagt gtctccagcc tgtctgccag catgcaccct 2100
cagctctcac cacagcaaag cttggaaacc cagtacctac agcatcgact ccagaagccc 2160
aatcttctgc caaaggccca gagtccttgt ccagtgtatt gtaaagagcc acctcggagc 2220
ctggaacagc agctacagga gcataggctc caacagaagc gactcttcct ccagaagcag 2280
tctcagctgc aagcatattt taatcagatg cagatagcag agagctccta ccctggacca 2340
agtcagcagc tggctcttcc ccaccaggag actccactga catcccagca gcccccatca 2400
ttcagcctga cccaggccct gagtcctgtc ctcgagccct cttctgagca gatgcaattt 2460
agctctttcc tcagccaata cccagagatg cagttgcagc cactgccctc tactcccggc 2520
ccccgggctc cacctccctt accctcacaa ttgcagcaac atcagcagcc accaccccca 2580
ccaccacccc ctccaccaca gcagccagga gctgctccaa cctccttaca gttctcctat 2640
cagacttgtg agctgccaag caccacttct tctgtaccaa actatcctgc ttcctgtcac 2700
tatcctgtgg atggagccca gcagagcaac ctcacagggg cggactgtcc caggagctca 2760
ggacttcagg acaccgcatc tagctatgac ccactggccc tctctgagct ccctggactc 2820
tttgattgtg aaatggtaga agctgtggat ccacaacaca atggggttgt gagctgctta 2880
gcccgggaga cctagcagct atggcaggac agttaggcaa cagatgaggg gctgcggaga 2940
ggctgtcaga agtttaaagg ctgcagagag aagctgggaa tgctca 2986

<210> 2

<211> 931

<212> PRT

<213> Mus musculus

<400> 2

Met Val Met Ala Asp Gly Pro Arg His Leu Gln Arg Gly Pro Val Arg
1               5                   10                  15

Val Gly Phe Tyr Asp Ile Glu Gly Thr Leu Gly Lys Gly Asn Phe Ala
            20                  25                  30

Val Val Lys Leu Gly Arg His Arg Thr Thr Lys Thr Glu Val Ala Ile
        35                  40                  45

Lys Ile Ile Asp Lys Ser Gln Leu Asp Ala Val Asn Leu Glu Lys Ile
    50                  55                  60

Tyr Arg Glu Val Gln Ile Met Lys Met Leu Asp His Pro His Ile Ile
65                  70                  75                  80

Lys Leu Tyr Gln Val Met Glu Thr Lys Ser Met Leu Tyr Leu Val Thr
                85                  90                  95

Glu Tyr Ala Lys Asn Gly Glu Ile Phe Asp Tyr Leu Ala Asn His Gly
            100                 105                 110

Arg Leu Asn Glu Ser Glu Ala Arg Arg Lys Phe Trp Gln Ile Leu Ser
        115                 120                 125

Ala Val Asp Tyr Cys His Gly Arg Lys Val Val His Arg Asp Leu Lys

    130                 135                 140

Ala Glu Asn Leu Leu Leu Asp Asn Asn Met Asn Ile Lys Ile Ala Asp
145                 150                 155                 160

Phe Gly Phe Gly Asn Phe Phe Lys Thr Gly Glu Leu Leu Ala Thr Trp
                165                 170                 175

Cys Gly Ser Pro Pro Tyr Ala Ala Pro Glu Val Phe Glu Gly Gln Gln
            180                 185                 190

Tyr Glu Gly Pro Gln Leu Asp Ile Trp Ser Met Gly Val Val Leu Tyr
        195                 200                 205

Val Leu Val Cys Gly Ala Leu Pro Phe Asp Gly Pro Thr Leu Pro Ile
    210                 215                 220

Leu Arg Gln Arg Val Leu Glu Gly Arg Phe Arg Ile Pro Tyr Phe Met
225                 230                 235                 240

Ser Glu Asp Cys Glu His Leu Ile Arg Arg Met Leu Val Leu Asp Pro
                245                 250                 255

Ser Lys Arg Leu Ser Ile Ala Gln Ile Lys Glu His Lys Trp Met Leu
            260                 265                 270

Ile Glu Val Pro Val Gln Arg Pro Ile Leu Tyr Pro Gln Glu Gln Glu
        275                 280                 285

Asn Glu Pro Ser Ile Gly Glu Phe Asn Glu Gln Val Leu Arg Leu Met
    290                 295                 300

His Ser Leu Gly Ile Asp Gln Gln Lys Thr Val Glu Ser Leu Gln Asn
305                 310                 315                 320

Lys Ser Tyr Asn His Phe Ala Ala Ile Tyr Phe Leu Leu Val Glu Arg
                325                 330                 335

Leu Lys Ser His Arg Ser Ser Phe Pro Val Glu Gln Arg Leu Asp Gly
            340                 345                 350

Arg Gln Arg Arg Pro Ser Thr Ile Ala Glu Gln Thr Val Ala Lys Ala
        355                 360                 365

Gln Thr Val Gly Leu Pro Val Thr Leu His Pro Pro Asn Val Arg Leu
    370                 375                 380

Met Arg Ser Thr Leu Leu Pro Gln Ala Ser Asn Val Glu Ala Phe Ser
385                 390                 395                 400

Phe Pro Thr Ser Ser Cys Gln Ala Glu Ala Ala Phe Met Glu Glu Glu
                405                 410                 415

Cys Val Asp Thr Pro Lys Val Asn Gly Cys Leu Leu Asp Pro Val Pro
            420                 425                 430

Pro Val Leu Val Arg Lys Gly Cys Gln Ser Leu Pro Ser Ser Met Met
        435                 440                 445

Glu Thr Ser Ile Asp Glu Gly Leu Glu Thr Glu Gly Glu Ala Glu Glu
    450                 455                 460

Asp Pro Ser Gln Ala Phe Glu Ala Phe Gln Ala Thr Arg Ser Gly Gln
465                 470                 475                 480

Arg Arg His Thr Leu Ser Glu Val Thr Asn Gln Leu Val Val Met Pro
                485                 490                 495

Gly Ala Gly Lys Met Phe Ser Met Ser Asp Asn Pro Ser Leu Glu Ser
            500                 505                 510

Val Asp Ser Glu Tyr Asp Met Gly Ser Ala Gln Arg Asp Leu Asn Phe
        515                 520                 525

Leu Glu Asp Ser Pro Ser Leu Lys Asp Ile Met Leu Ala Asn Gln Pro
    530                 535                 540

Ser Pro Arg Met Thr Ser Pro Phe Ile Ser Leu Arg Pro Ala Asn Pro
545                 550                 555                 560

Ala Met Gln Ala Leu Ser Ser Gln Lys Arg Glu Ala His Asn Arg Ser
                565                 570                 575

Pro Val Ser Phe Arg Glu Gly Arg Arg Ala Ser Asp Thr Ser Leu Thr

            580                 585                 590

Gln Gly Ile Val Ala Phe Arg Gln His Leu Gln Asn Leu Ala Arg Thr

        595                 600                 605

Lys Gly Ile Leu Glu Leu Asn Lys Val Gln Leu Leu Tyr Glu Gln Met

    610                 615                 620

Gly Ser Asn Ala Asp Pro Thr Leu Thr Ser Thr Ala Pro Gln Leu Gln

625                 630                 635                 640

Asp Leu Ser Ser Ser Cys Pro Gln Glu Glu Ile Ser Gln Gln Gln Glu

                645                 650                 655

Ser Val Ser Ser Leu Ser Ala Ser Met His Pro Gln Leu Ser Pro Gln

            660                 665                 670

Gln Ser Leu Glu Thr Gln Tyr Leu Gln His Arg Leu Gln Lys Pro Asn

        675                 680                 685

Leu Leu Pro Lys Ala Gln Ser Pro Cys Pro Val Tyr Cys Lys Glu Pro

    690                 695                 700

Pro Arg Ser Leu Glu Gln Gln Leu Gln Glu His Arg Leu Gln Gln Lys

705                 710                 715                 720

Arg Leu Phe Leu Gln Lys Gln Ser Gln Leu Gln Ala Tyr Phe Asn Gln

                725                 730                 735

Met Gln Ile Ala Glu Ser Ser Tyr Pro Gly Pro Ser Gln Gln Leu Ala
            740                 745                 750

Leu Pro His Gln Glu Thr Pro Leu Thr Ser Gln Gln Pro Pro Ser Phe
        755                 760                 765

Ser Leu Thr Gln Ala Leu Ser Pro Val Leu Glu Pro Ser Ser Glu Gln
    770                 775                 780

Met Gln Phe Ser Ser Phe Leu Ser Gln Tyr Pro Glu Met Gln Leu Gln
785                 790                 795                 800

Pro Leu Pro Ser Thr Pro Gly Pro Arg Ala Pro Pro Pro Leu Pro Ser
                805                 810                 815

Gln Leu Gln Gln His Gln Gln Pro Pro Pro Pro Pro Pro Pro Pro Pro
            820                 825                 830

Pro Gln Gln Pro Gly Ala Ala Pro Thr Ser Leu Gln Phe Ser Tyr Gln
        835                 840                 845

Thr Cys Glu Leu Pro Ser Thr Thr Ser Ser Val Pro Asn Tyr Pro Ala
    850                 855                 860

Ser Cys His Tyr Pro Val Asp Gly Ala Gln Gln Ser Asn Leu Thr Gly
865                 870                 875                 880

Ala Asp Cys Pro Arg Ser Ser Gly Leu Gln Asp Thr Ala Ser Ser Tyr
                885                 890                 895

Asp Pro Leu Ala Leu Ser Glu Leu Pro Gly Leu Phe Asp Cys Glu Met
            900                 905                 910

Val Glu Ala Val Asp Pro Gln His Asn Gly Val Val Ser Cys Leu Ala
        915                 920                 925

Arg Glu Thr
    930

<210> 3
<211> 141
<212> DNA
<213> Mus musculus

<400> 3
cccgtgagct tccgagaagg ccgcagagca tcagatacgt cccttacaca aggaattgtg 60
gcatttagac aacatcttca gaatcttgct agaaccaaag gaattctgga gttgaacaaa 120
gtacaattgc tgtatgaaca a 141

<210> 4
<211> 47
<212> PRT
<213> Mus musculus

<400> 4

Pro Val Ser Phe Arg Glu Gly Arg Arg Ala Ser Asp Thr Ser Leu Thr

1               5                   10                  15

Gln Gly Ile Val Ala Phe Arg Gln His Leu Gln Asn Leu Ala Arg Thr

            20                  25                  30

Lys Gly Ile Leu Glu Leu Asn Lys Val Gln Leu Leu Tyr Glu Gln

        35                  40                  45

<210> 5

<211> 756

<212> DNA

<213> Mus musculus

<400> 5

tacgacatcg agggcacgct gggcaagggc aactttgccg tggtgaagct ggggcggcac 60

cggaccacca agacggaggt ggctataaaa ataatagaca agtcacagct ggatgcagta 120

aaccttgaga aaatctaccg agaagtacag ataatgaaaa tgctcgacca tcctcacatc 180

attaaactgt atcaggtaat ggagaccaaa agtatgttgt accttgtgac agaatatgcc 240

aaaaatggag aaatttttga ttatcttgct aatcatggcc gattaaatga atctgaagcc 300

aggagaaaat tctggcaaat tctgtctgct gttgattatt gccatggccg gaaggtagtg 360

cacagagacc tgaaggctga aaatctcctg ctggataaca acatgaatat caaaatagca 420

gatttcggct ttggaaattt ctttaaaact ggtgaactgc tggcaacatg gtgtggcagc 480

cccccttatg cagccccaga agtctttgaa gggcagcagt atgaaggacc acagctggat 540

atatggagca tgggagttgt tctttatgtc cttgtctgtg gagctctacc ttttgatgga 600

ccaactctcc ctattttgag gcagagggtt ttagaaggaa gattccggat tccttatttc 660

atgtcagaag attgtgaaca cctcattaga aggatgttgg tcctagatcc ttccaaacgg 720

ctaagcatag ctcaaatcaa ggagcacaag tggatg 756

<210> 6

<211> 252

<212> PRT

<213> Mus musculus

<400> 6

Tyr Asp Ile Glu Gly Thr Leu Gly Lys Gly Asn Phe Ala Val Val Lys

1               5                   10                  15

Leu Gly Arg His Arg Thr Thr Lys Thr Glu Val Ala Ile Lys Ile Ile

            20                  25                  30

Asp Lys Ser Gln Leu Asp Ala Val Asn Leu Glu Lys Ile Tyr Arg Glu

        35                  40                  45

Val Gln Ile Met Lys Met Leu Asp His Pro His Ile Ile Lys Leu Tyr

    50                  55                  60

Gln Val Met Glu Thr Lys Ser Met Leu Tyr Leu Val Thr Glu Tyr Ala

65                  70                  75                  80

Lys Asn Gly Glu Ile Phe Asp Tyr Leu Ala Asn His Gly Arg Leu Asn

                85                  90                  95

Glu Ser Glu Ala Arg Arg Lys Phe Trp Gln Ile Leu Ser Ala Val Asp
            100                 105                 110

Tyr Cys His Gly Arg Lys Val Val His Arg Asp Leu Lys Ala Glu Asn
        115                 120                 125

Leu Leu Leu Asp Asn Asn Met Asn Ile Lys Ile Ala Asp Phe Gly Phe
    130                 135                 140

Gly Asn Phe Phe Lys Thr Gly Glu Leu Leu Ala Thr Trp Cys Gly Ser
145                 150                 155                 160

Pro Pro Tyr Ala Ala Pro Glu Val Phe Glu Gly Gln Gln Tyr Glu Gly
                165                 170                 175

Pro Gln Leu Asp Ile Trp Ser Met Gly Val Val Leu Tyr Val Leu Val
            180                 185                 190

Cys Gly Ala Leu Pro Phe Asp Gly Pro Thr Leu Pro Ile Leu Arg Gln
        195                 200                 205

Arg Val Leu Glu Gly Arg Phe Arg Ile Pro Tyr Phe Met Ser Glu Asp
    210                 215                 220

Cys Glu His Leu Ile Arg Arg Met Leu Val Leu Asp Pro Ser Lys Arg
225                 230                 235                 240

Leu Ser Ile Ala Gln Ile Lys Glu His Lys Trp Met

                245                 250

<210> 7

<211> 159

<212> DNA

<213> Mus musculus

<400> 7

attggagaat ttaatgagca ggttcttcga ctgatgcaca gccttgggat tgatcagcag 60

aagactgttg agtctttgca aaacaagagc tataatcact ttgctgccat ttatttcttg 120

ttggtggaac gtctaaaatc acataggagc agttttcct 159

<210> 8

<211> 53

<212> PRT

<213> Mus musculus

<400> 8

Ile Gly Glu Phe Asn Glu Gln Val Leu Arg Leu Met His Ser Leu Gly

1               5                   10                  15

Ile Asp Gln Gln Lys Thr Val Glu Ser Leu Gln Asn Lys Ser Tyr Asn

            20                  25                  30

His Phe Ala Ala Ile Tyr Phe Leu Leu Val Glu Arg Leu Lys Ser His

        35                  40                  45

Arg Ser Ser Phe Pro

    50

<210> 9

<211> 54

<212> DNA

<213> Mus musculus

<400> 9

cccgtgagct tccgagaagg ccgcagagca tcagatacgt cccttacaca agga 54

<210> 10

<211> 18

<212> PRT

<213> Mus musculus

<400> 10

Pro Val Ser Phe Arg Glu Gly Arg Arg Ala Ser Asp Thr Ser Leu Thr

1               5                   10                  15

Gln Gly

<210> 11

<211> 87

<212> DNA

<213> Mus musculus

<400> 11

attgtggcat ttagacaaca tcttcagaat cttgctagaa ccaaaggaat tctggagttg 60

aacaaagtac aattgctgta tgaacaa 87

<210> 12

<211> 29

<212> PRT

<213> Mus musculus

<400> 12

Ile Val Ala Phe Arg Gln His Leu Gln Asn Leu Ala Arg Thr Lys Gly

1               5                   10                  15

Ile Leu Glu Leu Asn Lys Val Gln Leu Leu Tyr Glu Gln

            20                  25

<210> 13

<211> 34

<212> DNA

<213> Artificial Sequence

<220>

<223> Description of Artificial Sequence:Primer

<400> 13

ttggatccat ggtcatggcg gatggcccga ggca 34

<210> 14

<211> 54

<212> DNA

<213> Artificial Sequence

<220>

<223> Description of Artificial Sequence:Primer

<400> 14

ctaggtctcc cgggctaagc agctcacaac cccattgtgt tgtgggtcca cagc 54

<210> 15

<211> 36

<212> DNA

<213> Artificial Sequence

<220>

<223> Description of Artificial Sequence:Primer

<400> 15

cggaggtggc tatcatgata atagacaagt cacagc 36

<210> 16

<211> 32

<212> DNA

<213> Artificial Sequence

<220>

<223> Description of Artificial Sequence:Primer

<400> 16

gaaggccgca gagctgcaga tacgtccctt ac 32

<210> 17

<211> 44

<212> DNA

<213> Artificial Sequence

<220>

<223> Description of Artificial Sequence:Primer

<400> 17

cagcacctcc gcctttccac tgctagcggt cgccttctct atgc 44